# EUROPEAN PATENT SPECIFICATION

(11) **EP 2 841 589 B1**
(45) Date of publication and mention of the grant of the patent: **12.09.2018**
(21) Application number: 13719473.4
(22) Date of filing: 23.04.2013
(51) Int. Cl.: C12P 21/02, C12N 15/67, C07K 14/00

(54) **POLYPEPTIDE EXPRESSION METHOD**
POLYPEPTIDEXPRESSIONSVERFAHREN
PROCÉDÉ D'EXPRESSION DE POLYPEPTIDES

(30) Priority: 23.04.2012 US 201261637093 P
(43) Date of publication of application: 04.03.2015
(73) Proprietor: DSM IP Assets B.V., 6411 TE Heerlen (NL)
(72) Inventor: ROUBOS, Johannes, Andries, 6100 AC Echt (NL); VAN DER LAAN, Jan Metske, 6100 AC Echt (NL); VAN DEN BERG, Bastiaan, 2628 CD Delft (NL); DE RIDDER, Dick, 2628 CD Delft (NL)
(74) Representative: DSM Intellectual Property
(86) International application number: PCT/EP2013/058412
(87) International publication number: WO 2013/160316

(56) References cited:
- EP-A1- 2 031 064
- WO-A2-2005/085457
- WO-A2-2009/155464
- JP-A- 2008 280 259
- US-A1- 2004 142 429
- US-A1- 2008 096 819
- US-A1- 2010 254 943
- KIM ET AL: "Matrix protein of VSV New Jersey serotype containing methionine to arginine substitutions at positions 48 and 51 allows near-normal host cell gene expression", VIROLOGY, ELSEVIER, AMSTERDAM, NL, vol. 357, no. 1, 29 November 2006 (2006-11-29), pages 41-53, XP005727567, ISSN: 0042-6822, DOI: 10.1016/J.VIROL.2006.07.022
- JOSHI B H ET AL: "Optimization of expression and purification of two biologically active chimeric fusion proteins that consist of human interleukin-13 and Pseudomonas exotoxin in Escherichia coli", PROTEIN EXPRESSION AND PURIFICATION, ACADEMIC PRESS, SAN DIEGO, CA, vol. 39, no. 2, 1 February 2005 (2005-02-01), pages 189-198, XP004707183, ISSN: 1046-5928, DOI: 10.1016/J.PEP.2004.10.012
- A. DERBISE ET AL: "Role of the C-Terminal Lysine Residues of Streptococcal Surface Enolase in Glu- and Lys-Plasminogen-Binding Activities of Group A Streptococci", INFECTION AND IMMUNITY, vol. 72, no. 1, 1 January 2004 (2004-01-01), pages 94-105, XP055075142, ISSN: 0019-9567, DOI: 10.1128/IAI.72.1.94-105.2004
- FU X ET AL: "SITE-DIRECTED MUTAGENESIS OF THE AVIAN RETROVIRUS NUCLEOCAPSID PROTEIN PP12 MUTATION WHICH AFFECTS RNA BINDING IN-VITRO BLOCKS VIRAL REPLICATION", JOURNAL OF BIOLOGICAL CHEMISTRY, vol. 263, no. 5, 1988, pages 2140-2145, XP002711316, ISSN: 0021-9258
- S. H. IRAM ET AL: "Mutation of Glu521 or Glu535 in Cytoplasmic Loop 5 Causes Differential Misfolding in Multiple Domains of Multidrug and Organic Anion Transporter MRP1 (ABCC1)", JOURNAL OF BIOLOGICAL CHEMISTRY, vol. 287, no. 10, 2 March 2012 (2012-03-02), pages 7543-7555, XP055075138, ISSN: 0021-9258, DOI: 10.1074/jbc.M111.310409
- TANAKA ET AL: "The role of protein kinase activity expressed by the UL13 gene of herpes simplex virus 1: The activity is not essential for optimal expression of UL41 and ICP0", VIROLOGY, ELSEVIER, AMSTERDAM, NL, vol. 341, no. 2, 8 October 2005 (2005-10-08), pages 301-312, XP005742345, ISSN: 0042-6822, DOI: 10.1016/J.VIROL.2005.07.010
- VAN DEN BERG BASTIAAN A ET AL: "Exploring Sequence Characteristics Related to High-Level Production of Secreted Proteins in Aspergillus niger", PLOS ONE, vol. 7, no. 10, October 2012 (2012-10), XP002711317,

### SEQUENCE LISTING

<110> DSM IP ASSETS B.V.
<120> POLYPEPTIDE EXPRESSION METHOD
<130> 28914-WO-PCT
<150> US 61/637093
   <151> 2012-04-23
<160> 99
<170> PatentIn version 3.5
<210> 1
   <211> 18
   <212> PRT
   <213> Aspergillus niger
<400> 1
   Met Leu Arg Arg Ala Leu Leu Leu Ser Ser Ser Ala Ile Leu Ala Val
   1 5 10 15
   Lys Ala
<210> 2
   <211> 20
   <212> PRT
   <213> Aspergillus niger
<400> 2
   Met Val Ala Trp Trp Ser Leu Phe Leu Tyr Gly Leu Gln Val Ala Ala
   1 5 10 15
   Pro Ala Leu Ala
   20
<210> 3
   <211> 17
   <212> PRT
   <213> Aspergillus niger
<400> 3
   Met Val Lys Ser Ile Leu Ala Ser Val Phe Phe Ala Ala Thr Ala Leu
   1 5 10 15
   Ala
<210> 4
   <211> 51
   <212> DNA
   <213> Artificial sequence
<220>
   <223> Codon-pair optimized PmeA signal sequence
<400> 4
   atggtcaagt ccatcctggc ctccgtcttc ttcgctgcca ctgctcttgc t 51
<210> 5
   <211> 455
   <212> PRT
   <213> Aspergillus niger
<400> 5
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Lys Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Lys Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Lys Met Gly Ala Ala Met Gln Gln Gly Met Val Leu
       370                 375                 380
   Val Met Ser Leu Trp Asp Asp Tyr Ala Ala Gln Met Leu Trp Leu Asp
   385                 390                 395                 400
   Ser Asp Tyr Pro Thr Asn Ala Ser Ala Thr Thr Pro Gly Val Ala Arg
                   405                 410                 415
   Gly Thr Cys Pro Thr Asp Ser Gly Val Pro Ser Gln Val Glu Ser Gln
               420                 425                 430
   Ser Pro Asn Ser Tyr Val Thr Tyr Ser Asn Ile Lys Phe Gly Pro Ile
           435                 440                 445
   Asn Ser Thr Phe Thr Ala Ser
   450 455
<210> 6
   <211> 498
   <212> PRT
   <213> Aspergillus niger
<400> 6
   Met Val Ala Trp Trp Ser Leu Phe Leu Tyr Gly Leu Gln Val Ala Ala
   1 5 10 15
   Pro Ala Leu Ala Ala Thr Pro Ala Asp Trp Arg Ser Gln Ser Ile Tyr
   20 25 30
   Phe Leu Leu Thr Asp Arg Phe Ala Arg Thr Asp Gly Ser Thr Thr Ala
   35 40 45
   Thr Cys Asn Thr Ala Asp Gln Lys Tyr Cys Gly Gly Thr Trp Gln Gly
   50 55 60
   Ile Ile Asp Lys Leu Asp Tyr Ile Gln Gly Met Gly Phe Thr Ala Ile
   65 70 75 80
   Trp Ile Thr Pro Val Thr Ala Gln Leu Pro Gln Thr Thr Ala Tyr Gly
   85 90 95
   Asp Ala Tyr His Gly Tyr Trp Gln Gln Asp Ile Tyr Ser Leu Asn Glu
   100 105 110
   Asn Tyr Gly Thr Ala Asp Asp Leu Lys Ala Leu Ser Ser Ala Leu His
   115 120 125
   Glu Arg Gly Met Tyr Leu Met Val Asp Val Val Ala Asn His Met Gly
   130 135 140
   Tyr Asp Gly Ala Gly Ser Ser Val Asp Tyr Ser Val Phe Lys Pro Phe
   145 150 155 160
   Ser Ser Gln Asp Tyr Phe His Pro Phe Cys Phe Ile Gln Asn Tyr Glu
   165 170 175
   Asp Gln Thr Gln Val Glu Asp Cys Trp Leu Gly Asp Asn Thr Val Ser
   180 185 190
   Leu Pro Asp Leu Asp Thr Thr Lys Asp Val Val Lys Asn Glu Trp Tyr
   195 200 205
   Asp Trp Val Gly Ser Leu Val Ser Asn Tyr Ser Ile Asp Gly Leu Arg
       210                 215                 220
   Ile Asp Thr Val Lys His Val Gln Lys Asp Phe Trp Pro Gly Tyr Asn
   225                 230                 235                 240
   Lys Ala Ala Gly Val Tyr Cys Ile Gly Glu Val Leu Asp Gly Asp Pro
                   245                 250                 255
   Ala Tyr Thr Cys Pro Tyr Gln Asn Val Met Asp Gly Val Leu Asn Tyr
               260                 265                 270
   Pro Ile Tyr Tyr Pro Leu Leu Asn Ala Phe Lys Ser Thr Ser Gly Ser
           275                 280                 285
   Met Asp Asp Leu Tyr Asn Met Ile Asn Thr Val Lys Ser Asp Cys Pro
       290                 295                 300
   Asp Ser Thr Leu Leu Gly Thr Phe Val Glu Asn His Asp Asn Pro Arg
   305                 310                 315                 320
   Phe Ala Ser Tyr Thr Asn Asp Ile Ala Leu Ala Lys Asn Val Ala Ala
                   325                 330                 335
   Phe Ile Ile Leu Asn Asp Gly Ile Pro Ile Ile Tyr Ala Gly Gln Glu
               340                 345                 350
   Gln His Tyr Ala Gly Gly Asn Asp Pro Ala Asn Arg Glu Ala Thr Trp
           355                 360                 365
   Leu Ser Gly Tyr Pro Thr Asp Ser Glu Leu Tyr Lys Leu Ile Ala Ser
       370                 375                 380
   Ala Asn Ala Ile Arg Asn Tyr Ala Ile Ser Lys Asp Thr Gly Phe Val
   385                 390                 395                 400
   Thr Tyr Lys Asn Trp Pro Ile Tyr Lys Asp Asp Thr Thr Ile Ala Met
                   405                 410                 415
   Arg Lys Gly Thr Asp Gly Ser Gln Ile Val Thr Ile Leu Ser Asn Lys
               420                 425                 430
   Gly Ala Ser Gly Asp Ser Tyr Thr Leu Ser Leu Ser Gly Ala Gly Tyr
           435                 440                 445
   Thr Ala Gly Gln Gln Leu Thr Glu Val Ile Gly Cys Thr Thr Val Thr
   450 455 460
   Val Gly Ser Asp Gly Asn Val Pro Val Pro Met Ala Gly Gly Leu Pro
   465 470 475 480
   Arg Val Leu Tyr Pro Thr Glu Lys Leu Ala Gly Ser Lys Ile Cys Ser
   485 490 495
   Ser Ser
<210> 7
   <211> 495
   <212> PRT
   <213> Artificial sequence
<220>
   <223> FUA (wt with signal sequence replaced by SS_PmeA)
<400> 7
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Lys Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Ile Tyr
               260                 265                 270
   Tyr Pro Leu Leu Asn Ala Phe Lys Ser Thr Ser Gly Ser Met Asp Asp
           275                 280                 285
   Leu Tyr Asn Met Ile Asn Thr Val Lys Ser Asp Cys Pro Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Lys Leu Ile Ala Ser Ala Asn Ala
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Met Arg Lys Gly
   405 410 415
   Thr Asp Gly Ser Gln Ile Val Thr Ile Leu Ser Asn Lys Gly Ala Ser
   420 425 430
   Gly Asp Ser Tyr Thr Leu Ser Leu Ser Gly Ala Gly Tyr Thr Ala Gly
   435 440 445
   Gln Gln Leu Thr Glu Val Ile Gly Cys Thr Thr Val Thr Val Gly Ser
   450 455 460
   Asp Gly Asn Val Pro Val Pro Met Ala Gly Gly Leu Pro Arg Val Leu
   465 470 475 480
   Tyr Pro Thr Glu Lys Leu Ala Gly Ser Lys Ile Cys Ser Ser Ser
   485 490 495
<210> 8
   <211> 455
   <212> PRT
   <213> Artificial sequence
<220>
   <223> Cellobiohydrolase variant
<400> 8
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Lys Leu Leu Asn Arg Glu Phe Thr Phe
   130 135 140
   Asp Val Asp Val Ser Asn Leu Pro Cys Gly Leu Asn Gly Ala Leu Tyr
   145 150 155 160
   Phe Val Ala Met Asp Ala Asp Gly Gly Val Ser Lys Tyr Pro Asn Asn
   165 170 175
   Lys Ala Gly Ala Lys Tyr Gly Thr Gly Tyr Cys Asp Ser Gln Cys Pro
   180 185 190
   Arg Asp Leu Lys Phe Ile Asp Gly Glu Gly Asn Val Glu Gly Trp Gln
   195 200 205
   Pro Ser Ser Asn Asn Ala Asn Thr Gly Ile Gly Asp His Gly Ser Cys
   210 215 220
   Cys Ala Glu Met Asp Val Trp Glu Ala Asn Ser Ile Ser Asn Ala Val
   225 230 235 240
   Thr Pro His Pro Cys Asp Thr Pro Gly Gln Thr Arg Cys Asp Gly Asp
   245 250 255
   Asp Cys Gly Gly Thr Tyr Ser Thr Asn Arg Tyr Ala Gly Glu Cys Asp
   260 265 270
   Pro Asp Gly Cys Asp Phe Asn Pro Tyr Arg Leu Gly Asn Thr Ser Phe
   275 280 285
   Tyr Gly Pro Gly Lys Ile Ile Asp Thr Thr Gln Pro Phe Thr Val Val
   290 295 300
   Thr Gln Phe Leu Thr Asp Asp Gly Thr Asp Thr Gly Thr Leu Ser Glu
   305 310 315 320
   Ile Lys Arg Phe Tyr Ile Gln Asn Gly Lys Val Ile Pro Gln Pro Asn
   325 330 335
   Ser Asp Ile Ser Gly Val Thr Gly Asn Ser Ile Thr Thr Glu Phe Cys
   340 345 350
   Thr Ala Gln Lys Gln Ala Phe Gly Asp Thr Asp Asp Phe Ser Gln His
   355 360 365
   Gly Gly Leu Ala Lys Met Gly Ala Ala Met Gln Gln Gly Met Val Leu
   370 375 380
   Val Leu Ser Leu Trp Asp Asp Tyr Ala Ala Gln Met Leu Trp Leu Asp
   385 390 395 400
   Ser Asp Tyr Pro Thr Asn Ala Ser Ala Thr Thr Pro Gly Val Ala Arg
   405 410 415
   Gly Thr Cys Pro Thr Asp Ser Gly Val Pro Ser Gln Val Glu Ser Gln
   420 425 430
   Ser Pro Asn Ser Tyr Val Thr Tyr Ser Asn Ile Lys Phe Gly Pro Ile
   435 440 445
   Asn Ser Thr Phe Thr Ala Ser
   450 455
<210> 9
   <211> 455
   <212> PRT
   <213> Artificial sequence
<220>
   <223> Cellobiohydrolase 1 variant
<400> 9
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Lys Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Arg Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Lys Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Lys Ile Gly Ala Ala Leu Gln Gln Gly Met Val Leu
   370 375 380
   Val Leu Ser Leu Trp Asp Asp Tyr Ala Ala Gln Met Leu Trp Leu Asp
   385 390 395 400
   Ser Asp Tyr Pro Thr Asn Ala Ser Ala Thr Thr Pro Gly Val Ala Arg
   405 410 415
   Gly Thr Cys Pro Thr Asp Ser Gly Val Pro Ser Gln Val Glu Ser Gln
   420 425 430
   Ser Pro Asn Ser Tyr Val Thr Tyr Ser Asn Ile Lys Phe Gly Pro Ile
   435 440 445
   Asn Ser Thr Phe Thr Ala Ser
   450 455
<210> 10
   <211> 455
   <212> PRT
   <213> Artificial sequence
<220>
   <223> Cellobiohydrolase 1 variant
<400> 10
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Lys Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Arg Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Leu Gly Asn Thr Ser Phe
           275                 280                 285
   Tyr Gly Pro Gly Lys Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Lys Ile Gly Ala Ala Leu Gln Gln Gly Met Val Leu
   370 375 380
   Val Met Ser Leu Trp Asp Asp Tyr Ala Ala Gln Met Leu Trp Leu Asp
   385 390 395 400
   Ser Asp Tyr Pro Thr Asn Ala Ser Ala Thr Thr Pro Gly Val Ala Arg
   405 410 415
   Gly Thr Cys Pro Thr Asp Ser Gly Val Pro Ser Gln Val Glu Ser Gln
   420 425 430
   Ser Pro Asn Ser Tyr Val Thr Tyr Ser Asn Ile Lys Phe Gly Pro Ile
   435 440 445
   Asn Ser Thr Phe Thr Ala Ser
   450 455
<210> 11
   <211> 455
   <212> PRT
   <213> Artificial sequence
<220>
   <223> Cellobiohydrolase 1 variant
<400> 11
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Lys Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Arg Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Leu Gly Asn Thr Ser Phe
           275                 280                 285
   Tyr Gly Pro Gly Lys Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Lys Ile Gly Ala Ala Leu Gln Gln Gly Met Val Leu
       370                 375                 380
   Val Leu Ser Leu Trp Asp Asp Tyr Ala Ala Gln Met Leu Trp Leu Asp
   385                 390                 395                 400
   Ser Asp Tyr Pro Thr Asn Ala Ser Ala Thr Thr Pro Gly Val Ala Arg
                   405                 410                 415
   Gly Thr Cys Pro Thr Asp Ser Gly Val Pro Ser Gln Val Glu Ser Gln
               420                 425                 430
   Ser Pro Asn Ser Tyr Val Thr Tyr Ser Asn Ile Lys Phe Gly Pro Ile
           435                 440                 445
   Asn Ser Thr Phe Thr Ala Ser
       450                 455
<210> 12
   <211> 455
   <212> PRT
   <213> Artificial sequence
<220>
   <223> Cellobiohydrolase 1 variant
<400> 12
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Lys Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Leu Asp Val Trp Glu Ala Asn Ser Ile Ser Asn Ala Val
   225                 230                 235                 240
   Thr Pro His Pro Cys Asp Thr Pro Gly Gln Thr Arg Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Leu Gly Asn Thr Ser Phe
           275                 280                 285
   Tyr Gly Pro Gly Lys Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Lys Val Gly Ala Ala Leu Gln Gln Gly Met Val Leu
       370                 375                 380
   Val Leu Ser Leu Trp Asp Asp Tyr Ala Ala Gln Met Leu Trp Leu Asp
   385                 390                 395                 400
   Ser Asp Tyr Pro Thr Asn Ala Ser Ala Thr Thr Pro Gly Val Ala Arg
                   405                 410                 415
   Gly Thr Cys Pro Thr Asp Ser Gly Val Pro Ser Gln Val Glu Ser Gln
               420                 425                 430
   Ser Pro Asn Ser Tyr Val Thr Tyr Ser Asn Ile Lys Phe Gly Pro Ile
           435                 440                 445
   Asn Ser Thr Phe Thr Ala Ser
       450                 455
<210> 13
   <211> 455
   <212> PRT
   <213> Artificial sequence
<220>
   <223> Cellobiohydrolase 1 variant
<400> 13
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Met Gln Asp
           115                 120                 125
   Asp Glu Thr Tyr Gln Met Phe Lys Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Met Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Lys Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Met Ala Lys Met Gly Ala Ala Met Gln Gln Gly Met Val Leu
   370 375 380
   Val Met Ser Leu Trp Asp Asp Tyr Ala Ala Gln Met Leu Trp Leu Asp
   385 390 395 400
   Ser Asp Tyr Pro Thr Asn Ala Ser Ala Thr Thr Pro Gly Val Ala Arg
   405 410 415
   Gly Thr Cys Pro Thr Asp Ser Gly Val Pro Ser Gln Val Glu Ser Gln
   420 425 430
   Ser Pro Asn Ser Tyr Val Thr Tyr Ser Asn Ile Lys Phe Gly Pro Ile
   435 440 445
   Asn Ser Thr Phe Thr Ala Ser
   450 455
<210> 14
   <211> 455
   <212> PRT
   <213> Artificial sequence
<220>
   <223> Cellobiohydrolase 1 variant
<400> 14
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Met
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Met Tyr Leu Met Gln Asp
           115                 120                 125
   Asp Glu Thr Tyr Gln Met Phe Lys Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Met Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Lys Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Lys Met Gly Ala Ala Met Gln Gln Gly Met Val Leu
   370 375 380
   Val Met Ser Leu Trp Asp Asp Tyr Ala Ala Gln Met Leu Trp Leu Asp
   385 390 395 400
   Ser Asp Tyr Pro Thr Asn Ala Ser Ala Thr Thr Pro Gly Val Ala Arg
   405 410 415
   Gly Thr Cys Pro Thr Asp Ser Gly Val Pro Ser Gln Val Glu Ser Gln
   420 425 430
   Ser Pro Asn Ser Tyr Val Thr Tyr Ser Asn Ile Lys Phe Gly Pro Ile
   435 440 445
   Asn Ser Thr Phe Thr Ala Ser
   450 455
<210> 15
   <211> 455
   <212> PRT
   <213> Artificial sequence
<220>
   <223> Cellobiohydrolase 1 variant
<400> 15
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Met
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Met Tyr Leu Met Gln Asp
           115                 120                 125
   Asp Glu Thr Tyr Gln Met Phe Lys Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Met Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Met Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Met Ala Lys Met Gly Ala Ala Met Gln Gln Gly Met Val Leu
   370 375 380
   Val Met Ser Leu Trp Asp Asp Tyr Ala Ala Gln Met Leu Trp Leu Asp
   385 390 395 400
   Ser Asp Tyr Pro Thr Asn Ala Ser Ala Thr Thr Pro Gly Val Ala Arg
   405 410 415
   Gly Thr Cys Pro Thr Asp Ser Gly Val Pro Ser Gln Val Glu Ser Gln
   420 425 430
   Ser Pro Asn Ser Tyr Val Thr Tyr Ser Asn Ile Lys Phe Gly Pro Ile
   435 440 445
   Asn Ser Thr Phe Thr Ala Ser
   450 455
<210> 16
   <211> 455
   <212> PRT
   <213> Artificial sequence
<220>
   <223> Cellobiohydrolase 1 variant
<400> 16
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Asn Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Lys Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Arg Arg Phe Tyr Ile Gln Asn Gly Asn Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Gly Met Gly Ala Ala Met Gln Gln Gly Met Val Leu
   370 375 380
   Val Met Ser Leu Trp Asp Asp Tyr Ala Ala Gln Met Leu Trp Leu Asp
   385 390 395 400
   Ser Asp Tyr Pro Thr Asn Ala Ser Ala Thr Thr Pro Gly Val Ala Arg
   405 410 415
   Gly Thr Cys Pro Thr Asp Ser Gly Val Pro Ser Gln Val Glu Ser Gln
   420 425 430
   Ser Pro Asn Ser Tyr Val Thr Tyr Ser Asn Ile Lys Phe Gly Pro Ile
   435 440 445
   Asn Ser Thr Phe Thr Ala Ser
   450 455
<210> 17
   <211> 455
   <212> PRT
   <213> Artificial sequence
<220>
   <223> Cellobiohydrolase 1 variant
<400> 17
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Asn Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Arg Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Lys Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Arg Arg Phe Tyr Ile Gln Asn Gly Asn Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Gly Met Gly Ala Ala Met Gln Gln Gly Met Val Leu
   370 375 380
   Val Met Ser Leu Trp Asp Asp Tyr Ala Ala Gln Met Leu Trp Leu Asp
   385 390 395 400
   Ser Asp Tyr Pro Thr Asn Ala Ser Ala Thr Thr Pro Gly Val Ala Arg
   405 410 415
   Gly Thr Cys Pro Thr Asp Ser Gly Val Pro Ser Gln Val Glu Ser Gln
   420 425 430
   Ser Pro Asn Ser Tyr Val Thr Tyr Ser Asn Ile Arg Phe Gly Pro Ile
   435 440 445
   Asn Ser Thr Phe Thr Ala Ser
   450 455
<210> 18
   <211> 455
   <212> PRT
   <213> Artificial sequence
<220>
   <223> Cellobiohydrolase 1 variant
<400> 18
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Asn Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Leu Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Arg Arg Phe Tyr Ile Gln Asn Gly Asn Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Gly Met Gly Ala Ala Met Gln Gln Gly Met Val Leu
   370 375 380
   Val Met Ser Leu Trp Asp Asp Tyr Ala Ala Gln Met Leu Trp Leu Asp
   385 390 395 400
   Ser Asp Tyr Pro Thr Asn Ala Ser Ala Thr Thr Pro Gly Val Ala Arg
   405 410 415
   Gly Thr Cys Pro Thr Asp Ser Gly Val Pro Ser Gln Val Glu Ser Gln
   420 425 430
   Ser Pro Asn Ser Tyr Val Thr Tyr Ser Asn Ile Lys Phe Gly Pro Ile
   435 440 445
   Asn Ser Thr Phe Thr Ala Ser
   450 455
<210> 19
   <211> 455
   <212> PRT
   <213> Artificial sequence
<220>
   <223> Cellobiohydrolase 1 variant
<400> 19
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Asn Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Arg Tyr Pro Asn Asn
                   165                 170                 175
   Thr Ala Gly Ala Gln Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Lys Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Arg Arg Phe Tyr Ile Gln Asn Gly Asn Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Gly Met Gly Ala Ala Met Gln Gln Gly Met Val Leu
           370                 375                 380
   Val Met Ser Leu Trp Asp Asp Tyr Ala Ala Gln Met Leu Trp Leu Asp
   385                 390                 395                 400
   Ser Asp Tyr Pro Thr Asn Ala Ser Ala Thr Thr Pro Gly Val Ala Arg
                   405                 410                 415
   Gly Thr Cys Pro Thr Asp Ser Gly Val Pro Ser Gln Val Glu Ser Gln
               420                 425                 430
   Ser Pro Asn Ser Tyr Val Thr Tyr Ser Asn Ile Arg Phe Gly Pro Ile
           435                 440                 445
   Asn Ser Thr Phe Thr Ala Ser
       450                 455
<210> 20
   <211> 455
   <212> PRT
   <213> Artificial sequence
<220>
   <223> Cellobiohydrolase 1 variant
<400> 20
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Asn Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Arg Tyr Pro Asn Asn
                   165                 170                 175
   Thr Ala Gly Ala Gln Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Leu Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Arg Arg Phe Tyr Ile Gln Asn Gly Asn Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Gly Met Gly Ala Ala Met Gln Gln Gly Met Val Leu
           370                 375                 380
   Val Met Ser Leu Trp Asp Asp Tyr Ala Ala Gln Met Leu Trp Leu Asp
   385                 390                 395                 400
   Ser Asp Tyr Pro Thr Asn Ala Ser Ala Thr Thr Pro Gly Val Ala Arg
                   405                 410                 415
   Gly Thr Cys Pro Thr Asp Ser Gly Val Pro Ser Gln Val Glu Ser Gln
               420                 425                 430
   Ser Pro Asn Ser Tyr Val Thr Tyr Ser Asn Ile Arg Phe Gly Pro Ile
           435                 440                 445
   Asn Ser Thr Phe Thr Ala Ser
       450                 455
<210> 21
   <211> 455
   <212> PRT
   <213> Artificial sequence
<220>
   <223> Cellobiohydrolase 1 variant
<400> 21
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Lys Cys Thr Ala Pro Gly Ser Cys Thr Thr Gln Asn Gly
           35                  40                  45
   Lys Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Lys Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Lys Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Lys Ile Ile Asp Thr Thr Lys Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Lys Met Gly Ala Ala Met Gln Gln Gly Met Val Leu
           370                 375                 380
   Val Met Ser Leu Trp Asp Asp Tyr Ala Ala Gln Met Leu Trp Leu Asp
   385                 390                 395                 400
   Ser Asp Tyr Pro Thr Asn Ala Ser Ala Thr Thr Pro Gly Val Ala Arg
                   405                 410                 415
   Gly Thr Cys Pro Thr Asp Ser Gly Val Pro Ser Gln Val Glu Ser Gln
               420                 425                 430
   Ser Pro Asn Ser Tyr Val Thr Tyr Ser Asn Ile Lys Phe Gly Pro Ile
           435                 440                 445
   Asn Ser Thr Phe Thr Ala Ser
       450                 455
<210> 22
   <211> 455
   <212> PRT
   <213> Artificial sequence
<220>
   <223> Cellobiohydrolase 1 variant
<400> 22
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Lys Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Lys Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Lys Leu Leu Asn Lys Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Lys Ile Ile Asp Thr Thr Lys Pro Phe Thr Val Val
       290                 295                 300
   Thr Gln Phe Leu Thr Asp Asp Gly Thr Asp Thr Gly Thr Leu Lys Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln Lys
           355                 360                 365
   Gly Gly Leu Ala Lys Met Gly Ala Ala Met Gln Gln Gly Met Val Leu
   370 375 380
   Val Met Ser Leu Trp Asp Asp Tyr Ala Ala Gln Met Leu Trp Leu Asp
   385 390 395 400
   Ser Asp Tyr Pro Thr Asn Ala Ser Ala Thr Thr Pro Gly Val Ala Arg
   405 410 415
   Gly Thr Cys Pro Thr Asp Ser Gly Val Pro Ser Gln Val Glu Ser Gln
   420 425 430
   Ser Pro Asn Ser Tyr Val Thr Tyr Ser Asn Ile Lys Phe Gly Pro Ile
   435 440 445
   Asn Ser Thr Phe Thr Ala Ser
   450 455
<210> 23
   <211> 455
   <212> PRT
   <213> Artificial sequence
<220>
   <223> Cellobiohydrolase 1 variant
<400> 23
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Lys Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Lys Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Lys Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Lys
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Lys Ser Phe
           275                 280                 285
   Tyr Gly Pro Gly Lys Ile Ile Asp Thr Thr Lys Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Lys Met Gly Ala Ala Met Gln Gln Gly Met Val Leu
       370                 375                 380
   Val Met Ser Leu Trp Asp Asp Tyr Ala Ala Gln Met Leu Trp Leu Asp
   385                 390                 395                 400
   Ser Asp Tyr Pro Thr Asn Ala Ser Ala Thr Thr Pro Gly Val Ala Arg
                   405                 410                 415
   Gly Thr Cys Pro Thr Asp Ser Gly Val Pro Ser Gln Val Glu Ser Gln
               420                 425                 430
   Ser Pro Asn Ser Tyr Val Thr Tyr Ser Asn Ile Lys Phe Gly Pro Ile
           435                 440                 445
   Asn Ser Thr Phe Thr Ala Ser
       450                 455
<210> 24
   <211> 455
   <212> PRT
   <213> Artificial sequence
<220>
   <223> Cellobiohydrolase 1 variant
<400> 24
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Lys Cys Thr Ala Pro Gly Ser Cys Thr Thr Gln Asn Gly
           35                  40                  45
   Lys Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Lys Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Lys Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Lys Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Thr Ser Phe
           275                 280                 285
   Tyr Gly Pro Gly Lys Ile Ile Asp Thr Thr Lys Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Lys Ala Phe Gly Asp Thr Asp Asp Phe Ser Gln His
           355                 360                 365
   Gly Gly Leu Ala Lys Met Gly Lys Ala Met Gln Gln Gly Met Val Leu
   370 375 380
   Val Met Ser Leu Trp Asp Asp Tyr Ala Ala Gln Met Leu Trp Leu Asp
   385 390 395 400
   Ser Asp Tyr Pro Thr Asn Ala Ser Ala Thr Thr Pro Gly Val Ala Arg
   405 410 415
   Gly Thr Cys Pro Thr Asp Ser Gly Val Pro Ser Gln Val Glu Ser Gln
   420 425 430
   Ser Pro Asn Ser Tyr Val Thr Tyr Ser Asn Ile Lys Phe Gly Pro Ile
   435 440 445
   Asn Ser Thr Phe Thr Ala Ser
   450 455
<210> 25
   <211> 455
   <212> PRT
   <213> Artificial sequence
<220>
   <223> Cellobiohydrolase 1 variant
<400> 25
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Lys Cys Thr Ala Pro Gly Ser Cys Thr Thr Gln Asn Gly
           35                  40                  45
   Lys Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Lys Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Lys Leu Leu Asn Lys Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Lys
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Met Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Met Gly Asn Lys Ser Phe
           275                 280                 285
   Tyr Gly Pro Gly Lys Ile Ile Asp Thr Thr Lys Pro Phe Thr Val Val
       290                 295                 300
   Thr Gln Phe Leu Thr Asp Asp Gly Thr Asp Thr Gly Thr Leu Lys Glu
   305                 310                 315                 320
   Ile Lys Arg Phe Tyr Ile Gln Asn Gly Lys Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln Lys
           355                 360                 365
   Gly Gly Leu Ala Lys Met Gly Ala Ala Met Gln Gln Gly Met Val Leu
       370                 375                 380
   Val Met Ser Leu Trp Asp Asp Tyr Ala Ala Gln Met Leu Trp Leu Asp
   385                 390                 395                 400
   Ser Asp Tyr Pro Thr Asn Ala Ser Ala Thr Thr Pro Gly Val Ala Arg
                   405                 410                 415
   Gly Thr Cys Pro Thr Asp Ser Gly Val Pro Ser Gln Val Glu Ser Gln
               420                 425                 430
   Ser Pro Asn Ser Tyr Val Thr Tyr Ser Asn Ile Lys Phe Gly Pro Ile
           435                 440                 445
   Asn Ser Thr Phe Thr Ala Ser
       450                 455
<210> 26
   <211> 455
   <212> PRT
   <213> Artificial sequence
<220>
   <223> Cellobiohydrolase 1 variant
<400> 26
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Asn Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Lys Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Arg Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Leu Gly Asn Thr Ser Phe
           275                 280                 285
   Tyr Gly Pro Gly Lys Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Arg Arg Phe Tyr Ile Gln Asn Gly Asn Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Gly Met Gly Ala Ala Met Gln Gln Gly Met Val Leu
           370                 375                 380
   Val Leu Ser Leu Trp Asp Asp Tyr Ala Ala Gln Met Leu Trp Leu Asp
   385                 390                 395                 400
   Ser Asp Tyr Pro Thr Asn Ala Ser Ala Thr Thr Pro Gly Val Ala Arg
                   405                 410                 415
   Gly Thr Cys Pro Thr Asp Ser Gly Val Pro Ser Gln Val Glu Ser Gln
               420                 425                 430
   Ser Pro Asn Ser Tyr Val Thr Tyr Ser Asn Ile Lys Phe Gly Pro Ile
           435                 440                 445
   Asn Ser Thr Phe Thr Ala Ser
       450                 455
<210> 27
   <211> 455
   <212> PRT
   <213> Artificial sequence
<220>
   <223> Cellobiohydrolase 1 variant
<400> 27
   Met Leu Arg Arg Ala Leu Leu Leu Ser Ser Ser Ala Ile Leu Ala Val
   1               5                   10                  15
   Lys Ala Gln Gln Ala Gly Thr Ala Thr Ala Glu Asn His Pro Pro Leu
               20                  25                  30
   Thr Trp Gln Glu Cys Thr Ala Pro Gly Ser Cys Thr Thr Gln Asn Gly
           35                  40                  45
   Ala Val Val Leu Asp Ser Asn Trp Arg Trp Val His Asn Val Gly Gly
       50                  55                  60
   Tyr Thr Asn Cys Tyr Thr Gly Asn Thr Trp Asn Pro Thr Tyr Cys Pro
   65                  70                  75                  80
   Asp Asp Val Thr Cys Ala Glu Asn Cys Ala Leu Asp Gly Ala Asp Tyr
                   85                  90                  95
   Glu Gly Thr Tyr Gly Val Thr Ser Ser Gly Ser Glu Leu Arg Leu Asn
               100                 105                 110
   Phe Val Thr Gly Ser Asn Val Gly Ser Arg Leu Tyr Leu Leu Gln Asp
           115                 120                 125
   Asp Glu Thr Tyr Gln Ile Phe Asn Leu Leu Asn Arg Glu Phe Thr Phe
       130                 135                 140
   Asp Val Asp Val Ser Asn Leu Pro Cys Gly Leu Asn Gly Ala Leu Tyr
   145                 150                 155                 160
   Phe Val Ala Met Asp Ala Asp Gly Gly Val Ser Arg Tyr Pro Asn Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Glu Gly Asn Val Glu Gly Trp Gln
           195                 200                 205
   Pro Ser Ser Asn Asn Ala Asn Thr Gly Ile Gly Asp His Gly Ser Cys
       210                 215                 220
   Cys Ala Glu Met Asp Val Trp Glu Ala Asn Ser Ile Ser Asn Ala Val
   225                 230                 235                 240
   Thr Pro His Pro Cys Asp Thr Pro Gly Gln Thr Arg Cys Asp Gly Asp
                   245                 250                 255
   Asp Cys Gly Gly Thr Tyr Ser Thr Asn Arg Tyr Ala Gly Glu Cys Asp
               260                 265                 270
   Pro Asp Gly Cys Asp Phe Asn Pro Tyr Arg Leu Gly Asn Thr Ser Phe
           275                 280                 285
   Tyr Gly Pro Gly Leu Ile Ile Asp Thr Thr Gln Pro Phe Thr Val Val
       290                 295                 300
   Thr Gln Phe Leu Thr Asp Asp Gly Thr Asp Thr Gly Thr Leu Ser Glu
   305                 310                 315                 320
   Ile Arg Arg Phe Tyr Ile Gln Asn Gly Asn Val Ile Pro Gln Pro Asn
                   325                 330                 335
   Ser Asp Ile Ser Gly Val Thr Gly Asn Ser Ile Thr Thr Glu Phe Cys
               340                 345                 350
   Thr Ala Gln Lys Gln Ala Phe Gly Asp Thr Asp Asp Phe Ser Gln His
           355                 360                 365
   Gly Gly Leu Ala Gly Val Gly Ala Ala Leu Gln Gln Gly Met Val Leu
       370                 375                 380
   Val Leu Ser Leu Trp Asp Asp Tyr Ala Ala Gln Met Leu Trp Leu Asp
   385                 390                 395                 400
   Ser Asp Tyr Pro Thr Asn Ala Ser Ala Thr Thr Pro Gly Val Ala Arg
                   405                 410                 415
   Gly Thr Cys Pro Thr Asp Ser Gly Val Pro Ser Gln Val Glu Ser Gln
               420                 425                 430
   Ser Pro Asn Ser Tyr Val Thr Tyr Ser Asn Ile Arg Phe Gly Pro Ile
           435                 440                 445
   Asn Ser Thr Phe Thr Ala Ser
       450                 455
<210> 28
   <211> 495
   <212> PRT
   <213> Artificial sequence
<220>
   <223> Fungal amylase variant
<400> 28
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Lys Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Leu Asp Gly Val Leu Asn Tyr Pro Ile Tyr
               260                 265                 270
   Tyr Pro Leu Leu Asn Ala Phe Lys Ser Thr Ser Gly Ser Met Asp Asp
           275                 280                 285
   Leu Tyr Asn Met Ile Asn Thr Val Lys Ser Asp Cys Pro Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Lys Leu Ile Ala Ser Ala Asn Ala
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Leu Arg Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Gln Leu Thr Glu Val Ile Gly Cys Thr Thr Val Thr Val Gly Ser
       450                 455                 460
   Asp Gly Asn Val Pro Val Pro Leu Ala Gly Gly Leu Pro Arg Val Leu
   465                 470                 475                 480
   Tyr Pro Thr Glu Lys Leu Ala Gly Ser Lys Ile Cys Ser Ser Ser
                   485                 490                 495
<210> 29
   <211> 495
   <212> PRT
   <213> Artificial sequence
<220>
   <223> Fungal amylase variant
<400> 29
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Lys Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Leu Asp Gly Val Leu Asn Tyr Pro Ile Tyr
               260                 265                 270
   Tyr Pro Leu Leu Asn Ala Phe Lys Ser Thr Ser Gly Ser Ile Asp Asp
           275                 280                 285
   Leu Tyr Asn Met Ile Asn Thr Val Lys Ser Asp Cys Pro Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
   340 345 350
   Ala Gly Gly Asn Asp Pro Ala Asn Arg Glu Ala Thr Trp Leu Ser Gly
   355 360 365
   Tyr Pro Thr Asp Ser Glu Leu Tyr Lys Leu Ile Ala Ser Ala Asn Ala
   370 375 380
   Ile Arg Asn Tyr Ala Ile Ser Lys Asp Thr Gly Phe Val Thr Tyr Lys
   385 390 395 400
   Asn Trp Pro Ile Tyr Lys Asp Asp Thr Thr Ile Ala Met Arg Lys Gly
   405 410 415
   Thr Asp Gly Ser Gln Ile Val Thr Ile Leu Ser Asn Lys Gly Ala Ser
   420 425 430
   Gly Asp Ser Tyr Thr Leu Ser Leu Ser Gly Ala Gly Tyr Thr Ala Gly
   435 440 445
   Gln Gln Leu Thr Glu Val Ile Gly Cys Thr Thr Val Thr Val Gly Ser
   450 455 460
   Asp Gly Asn Val Pro Val Pro Leu Ala Gly Gly Leu Pro Arg Val Leu
   465 470 475 480
   Tyr Pro Thr Glu Lys Leu Ala Gly Ser Lys Ile Cys Ser Ser Ser
   485 490 495
<210> 30
   <211> 495
   <212> PRT
   <213> Artificial sequence
<220>
   <223> Fungal amylase variant
<400> 30
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Met Gly Phe Thr Ala Ile Trp Ile Thr
   65 70 75 80
   Pro Val Thr Ala Gln Leu Pro Gln Thr Thr Ala Tyr Gly Asp Ala Tyr
   85 90 95
   His Gly Tyr Trp Gln Gln Asp Ile Tyr Ser Leu Asn Glu Asn Tyr Gly
   100 105 110
   Thr Ala Asp Asp Leu Lys Ala Leu Ser Ser Ala Leu His Glu Arg Gly
   115 120 125
   Met Tyr Leu Met Val Asp Val Val Ala Asn His Met Gly Tyr Asp Gly
   130 135 140
   Ala Gly Ser Ser Val Asp Tyr Ser Val Phe Lys Pro Phe Ser Ser Gln
   145 150 155 160
   Asp Tyr Phe His Pro Phe Cys Phe Ile Gln Asn Tyr Glu Asp Gln Thr
   165 170 175
   Gln Val Glu Asp Cys Trp Leu Gly Asp Asn Thr Val Ser Leu Pro Asp
   180 185 190
   Leu Asp Thr Thr Lys Asp Val Val Lys Asn Glu Trp Tyr Asp Trp Val
   195 200 205
   Gly Ser Leu Val Ser Asn Tyr Ser Ile Asp Gly Leu Arg Ile Asp Thr
   210 215 220
   Val Lys His Val Gln Lys Asp Phe Trp Pro Gly Tyr Asn Lys Ala Ala
   225 230 235 240
   Gly Val Tyr Cys Ile Gly Glu Val Leu Asp Gly Asp Pro Ala Tyr Thr
   245 250 255
   Cys Pro Tyr Gln Asn Val Leu Asp Gly Val Leu Asn Tyr Pro Ile Tyr
   260 265 270
   Tyr Pro Leu Leu Asn Ala Phe Lys Ser Thr Ser Gly Ser Met Asp Asp
   275 280 285
   Leu Tyr Asn Glu Ile Asn Thr Val Lys Ser Asp Cys Pro Asp Ser Thr
   290 295 300
   Leu Leu Gly Thr Phe Val Glu Asn His Asp Asn Pro Arg Phe Ala Ser
   305 310 315 320
   Tyr Thr Asn Asp Ile Ala Leu Ala Lys Asn Val Ala Ala Phe Ile Ile
   325 330 335
   Leu Asn Asp Gly Ile Pro Ile Ile Tyr Ala Gly Gln Glu Gln His Tyr
   340 345 350
   Ala Gly Gly Asn Asp Pro Ala Asn Arg Glu Ala Thr Trp Leu Ser Gly
   355 360 365
   Tyr Pro Thr Asp Ser Glu Leu Tyr Lys Leu Ile Ala Ser Ala Asn Ala
   370 375 380
   Ile Arg Asn Tyr Ala Ile Ser Lys Asp Thr Gly Phe Val Thr Tyr Lys
   385 390 395 400
   Asn Trp Pro Ile Tyr Lys Asp Asp Thr Thr Ile Ala Leu Arg Lys Gly
   405 410 415
   Thr Asp Gly Ser Gln Ile Val Thr Ile Leu Ser Asn Lys Gly Ala Ser
   420 425 430
   Gly Asp Ser Tyr Thr Leu Ser Leu Ser Gly Ala Gly Tyr Thr Ala Gly
   435 440 445
   Gln Gln Leu Thr Glu Val Ile Gly Cys Thr Thr Val Thr Val Gly Ser
   450 455 460
   Asp Gly Asn Val Pro Val Pro Met Ala Gly Gly Leu Pro Arg Val Leu
   465 470 475 480
   Tyr Pro Thr Glu Lys Leu Ala Gly Ser Lys Ile Cys Ser Ser Ser
   485 490 495
<210> 31
   <211> 495
   <212> PRT
   <213> Artificial sequence
<220>
   <223> Fungal amylase variant
<400> 31
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
   35 40 45
   Thr Ala Asp Gln Lys Tyr Cys Gly Gly Thr Trp Gln Gly Ile Ile Asp
   50 55 60
   Lys Leu Asp Tyr Ile Gln Gly Met Gly Phe Thr Ala Ile Trp Ile Thr
   65 70 75 80
   Pro Val Thr Ala Gln Leu Pro Gln Thr Thr Ala Tyr Gly Asp Ala Tyr
   85 90 95
   His Gly Tyr Trp Gln Gln Asp Ile Tyr Ser Leu Asn Glu Asn Tyr Gly
   100 105 110
   Thr Ala Asp Asp Leu Lys Ala Leu Ser Ser Ala Leu His Glu Arg Gly
   115 120 125
   Met Tyr Leu Met Val Asp Val Val Ala Asn His Met Gly Tyr Asp Gly
   130 135 140
   Ala Gly Ser Ser Val Asp Tyr Ser Val Phe Lys Pro Phe Ser Ser Gln
   145 150 155 160
   Asp Tyr Phe His Pro Phe Cys Phe Ile Gln Asn Tyr Glu Asp Gln Thr
   165 170 175
   Gln Val Glu Asp Cys Trp Leu Gly Asp Asn Thr Val Ser Leu Pro Asp
   180 185 190
   Leu Asp Thr Thr Lys Asp Val Val Lys Asn Glu Trp Tyr Asp Trp Val
   195 200 205
   Gly Ser Leu Val Ser Asn Tyr Ser Ile Asp Gly Leu Arg Ile Asp Thr
   210 215 220
   Val Lys His Val Gln Lys Asp Phe Trp Pro Gly Tyr Asn Lys Ala Ala
   225 230 235 240
   Gly Val Tyr Cys Ile Gly Glu Val Leu Asp Gly Asp Pro Ala Tyr Thr
   245 250 255
   Cys Pro Tyr Gln Asn Val Leu Asp Gly Val Leu Asn Tyr Pro Ile Tyr
   260 265 270
   Tyr Pro Leu Leu Asn Ala Phe Lys Ser Thr Ser Gly Ser Ile Asp Asp
               275                 280                 285
   Leu Tyr Asn Glu Ile Asn Thr Val Lys Ser Asp Cys Pro Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Lys Leu Ile Ala Ser Ala Asn Ala
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Leu Arg Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Gln Leu Thr Glu Val Ile Gly Cys Thr Thr Val Thr Val Gly Ser
       450                 455                 460
   Asp Gly Asn Val Pro Val Pro Leu Ala Gly Gly Leu Pro Arg Val Leu
   465                 470                 475                 480
   Tyr Pro Thr Glu Lys Leu Ala Gly Ser Lys Ile Cys Ser Ser Ser
                   485                 490                 495
<210> 32
   <211> 495
   <212> PRT
   <213> Artificial sequence
<220>
   <223> Fungal amylase variant
<400> 32
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Met Tyr Leu Met Val Asp Val Val Ala Asn His Phe Gly Tyr Asp Gly
       130                 135                 140
   Ala Gly Ser Ser Val Asp Tyr Ser Val Phe Lys Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Cys Ile Gly Glu Val Leu Asp Gly Asp Pro Ala Tyr Thr
   245 250 255
   Cys Pro Tyr Gln Asn Val Met Asp Gly Val Leu Asn Tyr Pro Ile Tyr
   260 265 270
   Tyr Pro Leu Leu Asn Ala Phe Lys Ser Thr Ser Gly Ser Ile Asp Asp
   275 280 285
   Leu Tyr Asn Glu Ile Asn Thr Val Lys Ser Asp Cys Pro Asp Ser Thr
   290 295 300
   Leu Leu Gly Thr Phe Val Glu Asn His Asp Asn Pro Arg Phe Ala Ser
   305 310 315 320
   Tyr Thr Asn Asp Ile Ala Leu Ala Lys Asn Val Ala Ala Phe Ile Ile
   325 330 335
   Leu Asn Asp Gly Ile Pro Ile Ile Tyr Ala Gly Gln Glu Gln His Tyr
   340 345 350
   Ala Gly Gly Asn Asp Pro Ala Asn Arg Glu Ala Thr Trp Leu Ser Gly
   355 360 365
   Tyr Pro Thr Asp Ser Glu Leu Tyr Lys Leu Ile Ala Ser Ala Asn Ala
   370 375 380
   Ile Arg Asn Tyr Ala Ile Ser Lys Asp Thr Gly Phe Val Thr Tyr Lys
   385 390 395 400
   Asn Trp Pro Ile Tyr Lys Asp Asp Thr Thr Ile Ala Leu Arg Lys Gly
   405 410 415
   Thr Asp Gly Ser Gln Ile Val Thr Ile Leu Ser Asn Lys Gly Ala Ser
   420 425 430
   Gly Asp Ser Tyr Thr Leu Ser Leu Ser Gly Ala Gly Tyr Thr Ala Gly
   435 440 445
   Gln Gln Leu Thr Glu Val Ile Gly Cys Thr Thr Val Thr Val Gly Ser
   450 455 460
   Asp Gly Asn Val Pro Val Pro Leu Ala Gly Gly Leu Pro Arg Val Leu
   465 470 475 480
   Tyr Pro Thr Glu Lys Leu Ala Gly Ser Lys Ile Cys Ser Ser Ser
   485 490 495
<210> 33
   <211> 495
   <212> PRT
   <213> Artificial sequence
<220>
   <223> Fungal amylase variant
<400> 33
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Leu Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Met Tyr Leu Met Val Asp Val Val Ala Asn His Phe Gly Tyr Asp Gly
       130                 135                 140
   Ala Gly Ser Ser Val Asp Tyr Ser Val Phe Lys Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Leu Asp Gly Val Leu Asn Tyr Pro Ile Tyr
               260                 265                 270
   Tyr Pro Leu Leu Asn Ala Phe Lys Ser Thr Ser Gly Ser Met Asp Asp
           275                 280                 285
   Leu Tyr Asn Met Ile Asn Thr Val Lys Ser Asp Cys Pro Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Lys Leu Ile Ala Ser Ala Asn Ala
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Leu Arg Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Gln Leu Thr Glu Val Ile Gly Cys Thr Thr Val Thr Val Gly Ser
       450                 455                 460
   Asp Gly Asn Val Pro Val Pro Leu Ala Gly Gly Leu Pro Arg Val Leu
   465                 470                 475                 480
   Tyr Pro Thr Glu Lys Leu Ala Gly Ser Lys Ile Cys Ser Ser Ser
                   485                 490                 495
<210> 34
   <211> 495
   <212> PRT
   <213> Artificial sequence
<220>
   <223> Fungal amylase variant
<400> 34
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Leu Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Met Tyr Leu Met Val Asp Val Val Ala Asn His Phe Gly Tyr Asp Gly
       130                 135                 140
   Ala Gly Ser Ser Val Asp Tyr Ser Val Phe Lys Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Leu Asp Gly Val Leu Asn Tyr Pro Ile Tyr
               260                 265                 270
   Tyr Pro Leu Leu Asn Ala Phe Lys Ser Thr Ser Gly Ser Ile Asp Asp
           275                 280                 285
   Leu Tyr Asn Glu Ile Asn Thr Val Lys Ser Asp Cys Pro Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Lys Leu Ile Ala Ser Ala Asn Ala
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Met Arg Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Gln Leu Thr Glu Val Ile Gly Cys Thr Thr Val Thr Val Gly Ser
       450                 455                 460
   Asp Gly Asn Val Pro Val Pro Met Ala Gly Gly Leu Pro Arg Val Leu
   465                 470                 475                 480
   Tyr Pro Thr Glu Lys Leu Ala Gly Ser Lys Ile Cys Ser Ser Ser
                   485                 490                 495
<210> 35
   <211> 495
   <212> PRT
   <213> Artificial sequence
<220>
   <223> Fungal amylase variant
<400> 35
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Leu Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Ile Tyr Leu Leu Val Asp Val Val Ala Asn His Phe Gly Tyr Asp Gly
       130                 135                 140
   Ala Gly Ser Ser Val Asp Tyr Ser Val Phe Lys Pro Phe Ser Ser Gln
   145 150 155 160
   Asp Tyr Phe His Pro Phe Cys Phe Ile Gln Asn Tyr Glu Asp Gln Thr
   165 170 175
   Gln Val Glu Asp Cys Trp Leu Gly Asp Asn Thr Val Ser Leu Pro Asp
   180 185 190
   Leu Asp Thr Thr Lys Asp Val Val Lys Asn Glu Trp Tyr Asp Trp Val
   195 200 205
   Gly Ser Leu Val Ser Asn Tyr Ser Ile Asp Gly Leu Arg Ile Asp Thr
   210 215 220
   Val Lys His Val Gln Lys Asp Phe Trp Pro Gly Tyr Asn Lys Ala Ala
   225 230 235 240
   Gly Val Tyr Cys Ile Gly Glu Val Leu Asp Gly Asp Pro Ala Tyr Thr
   245 250 255
   Cys Pro Tyr Gln Asn Val Leu Asp Gly Val Leu Asn Tyr Pro Ile Tyr
   260 265 270
   Tyr Pro Leu Leu Asn Ala Phe Lys Ser Thr Ser Gly Ser Ile Asp Asp
   275 280 285
   Leu Tyr Asn Glu Ile Asn Thr Val Lys Ser Asp Cys Pro Asp Ser Thr
   290 295 300
   Leu Leu Gly Thr Phe Val Glu Asn His Asp Asn Pro Arg Phe Ala Ser
   305 310 315 320
   Tyr Thr Asn Asp Ile Ala Leu Ala Lys Asn Val Ala Ala Phe Ile Ile
   325 330 335
   Leu Asn Asp Gly Ile Pro Ile Ile Tyr Ala Gly Gln Glu Gln His Tyr
   340 345 350
   Ala Gly Gly Asn Asp Pro Ala Asn Arg Glu Ala Thr Trp Leu Ser Gly
   355 360 365
   Tyr Pro Thr Asp Ser Glu Leu Tyr Lys Leu Ile Ala Ser Ala Asn Ala
   370 375 380
   Ile Arg Asn Tyr Ala Ile Ser Lys Asp Thr Gly Phe Val Thr Tyr Lys
   385 390 395 400
   Asn Trp Pro Ile Tyr Lys Asp Asp Thr Thr Ile Ala Leu Arg Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Gln Leu Thr Glu Val Ile Gly Cys Thr Thr Val Thr Val Gly Ser
       450                 455                 460
   Asp Gly Asn Val Pro Val Pro Leu Ala Gly Gly Leu Pro Arg Val Leu
   465                 470                 475                 480
   Tyr Pro Thr Glu Lys Leu Ala Gly Ser Lys Ile Cys Ser Ser Ser
                   485                 490                 495
<210> 36
   <211> 495
   <212> PRT
   <213> Artificial sequence
<220>
   <223> Fungal amylase variant
<400> 36
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
   115 120 125
   Met Tyr Leu Met Val Asp Val Val Ala Asn His Met Gly Tyr Asp Gly
   130 135 140
   Ala Gly Ser Ser Val Asp Tyr Ser Val Phe Lys Pro Phe Ser Ser Gln
   145 150 155 160
   Asp Tyr Phe His Pro Phe Cys Phe Ile Gln Asn Tyr Glu Asp Gln Thr
   165 170 175
   Gln Val Glu Asp Cys Trp Leu Gly Asp Asn Thr Val Ser Leu Pro Asp
   180 185 190
   Leu Asp Thr Thr Lys Asp Val Val Lys Asn Glu Trp Tyr Asp Trp Val
   195 200 205
   Gly Ser Leu Val Ser Asn Tyr Ser Ile Asp Gly Leu Arg Ile Asp Thr
   210 215 220
   Val Lys His Val Gln Lys Asp Phe Trp Pro Gly Tyr Asn Lys Ala Ala
   225 230 235 240
   Gly Val Tyr Cys Ile Gly Glu Val Leu Asp Gly Asp Pro Ala Tyr Thr
   245 250 255
   Cys Pro Tyr Gln Asn Val Met Asp Gly Val Leu Asn Tyr Pro Met Tyr
   260 265 270
   Tyr Pro Leu Leu Asn Ala Phe Lys Ser Thr Ser Gly Ser Met Asp Asp
   275 280 285
   Leu Tyr Asn Met Ile Asn Thr Met Lys Ser Asp Cys Pro Asp Ser Thr
   290 295 300
   Leu Leu Gly Thr Phe Val Glu Asn His Asp Asn Pro Arg Phe Ala Ser
   305 310 315 320
   Tyr Thr Asn Asp Met Ala Leu Ala Lys Asn Val Ala Ala Phe Ile Ile
   325 330 335
   Leu Asn Asp Gly Ile Pro Ile Ile Tyr Ala Gly Gln Glu Gln His Tyr
   340 345 350
   Ala Gly Gly Asn Asp Pro Ala Asn Arg Glu Ala Thr Trp Leu Ser Gly
   355 360 365
   Tyr Pro Thr Asp Ser Glu Leu Tyr Lys Leu Ile Ala Ser Ala Asn Ala
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Met Arg Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Gln Leu Thr Glu Val Ile Gly Cys Thr Thr Val Thr Val Gly Ser
       450                 455                 460
   Asp Gly Asn Val Pro Val Pro Met Ala Gly Gly Leu Pro Arg Val Leu
   465                 470                 475                 480
   Tyr Pro Thr Glu Lys Leu Ala Gly Ser Lys Ile Cys Ser Ser Ser
                   485                 490                 495
<210> 37
   <211> 495
   <212> PRT
   <213> Artificial sequence
<220>
   <223> Fungal amylase variant
<400> 37
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Lys Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Met Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Ile Tyr
               260                 265                 270
   Tyr Pro Leu Leu Asn Ala Phe Lys Ser Thr Ser Gly Ser Met Asp Asp
           275                 280                 285
   Leu Tyr Asn Met Ile Asn Thr Val Lys Ser Asp Cys Pro Asp Ser Thr
       290                 295                 300
   Leu Leu Gly Thr Phe Met Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Lys Leu Ile Ala Ser Ala Asn Ala
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Met Arg Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Gln Leu Met Glu Val Ile Gly Cys Thr Thr Val Thr Val Gly Ser
       450                 455                 460
   Asp Gly Asn Val Pro Val Pro Met Ala Gly Gly Leu Pro Arg Val Leu
   465                 470                 475                 480
   Tyr Pro Thr Glu Lys Leu Ala Gly Ser Lys Ile Cys Ser Ser Ser
                   485                 490                 495
<210> 38
   <211> 495
   <212> PRT
   <213> Artificial sequence
<220>
   <223> Fungal amylase variant
<400> 38
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Lys Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Met Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Met Tyr
               260                 265                 270
   Tyr Pro Leu Leu Asn Ala Phe Lys Ser Thr Ser Gly Ser Met Asp Asp
           275                 280                 285
   Leu Tyr Asn Met Ile Asn Thr Met Lys Ser Asp Cys Pro Asp Ser Thr
       290                 295                 300
   Leu Leu Gly Thr Phe Met Glu Asn His Asp Asn Pro Arg Phe Ala Ser
   305                 310                 315                 320
   Tyr Thr Asn Asp Met Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Lys Leu Ile Ala Ser Ala Asn Ala
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Met Arg Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Gln Leu Met Glu Val Ile Gly Cys Thr Thr Val Thr Val Gly Ser
       450                 455                 460
   Asp Gly Asn Val Pro Val Pro Met Ala Gly Gly Leu Pro Arg Val Leu
   465                 470                 475                 480
   Tyr Pro Thr Glu Lys Leu Ala Gly Ser Lys Ile Cys Ser Ser Ser
                   485                 490                 495
<210> 39
   <211> 495
   <212> PRT
   <213> Artificial sequence
<220>
   <223> Fungal amylase variant
<400> 39
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Lys Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Met Val Glu Asp Cys Trp Leu Gly Asp Asn Thr Val Ser Leu Pro Asp
               180                 185                 190
   Leu Asp Thr Thr Lys Asp Val Val Lys Asn Met Trp Tyr Asp Trp Val
           195                 200                 205
   Gly Ser Met Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Met Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Met Tyr
               260                 265                 270
   Tyr Pro Leu Leu Asn Ala Phe Lys Ser Thr Ser Gly Ser Met Asp Asp
           275                 280                 285
   Leu Tyr Asn Met Ile Asn Thr Met Lys Ser Asp Cys Pro Asp Ser Thr
       290                 295                 300
   Leu Leu Gly Thr Phe Met Glu Asn His Asp Asn Pro Arg Phe Ala Ser
   305                 310                 315                 320
   Tyr Thr Asn Asp Met Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Lys Leu Ile Ala Ser Ala Asn Ala
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Met Arg Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Gln Leu Met Glu Val Ile Gly Cys Thr Thr Val Thr Val Gly Ser
       450                 455                 460
   Asp Gly Asn Val Pro Val Pro Met Ala Gly Gly Leu Pro Arg Val Leu
   465                 470                 475                 480
   Tyr Pro Thr Glu Lys Leu Ala Gly Ser Lys Ile Cys Ser Ser Ser
                   485                 490                 495
<210> 40
   <211> 495
   <212> PRT
   <213> Artificial sequence
<220>
   <223> Fungal amylase variant
<400> 40
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Met Gly Phe Thr Ala Ile Trp Ile Thr
   65                  70                  75                  80
   Pro Val Thr Lys Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Lys Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Glu Trp Tyr Asp Trp Val
           195                 200                 205
   Lys Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Ile Tyr
               260                 265                 270
   Tyr Pro Leu Leu Asn Ala Phe Lys Ser Thr Ser Gly Ser Met Asp Asp
           275                 280                 285
   Leu Tyr Asn Met Ile Asn Thr Val Lys Ser Asp Cys Lys Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Lys Leu Ile Ala Ser Ala Asn Lys
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Met Arg Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Lys Leu Thr Glu Val Ile Gly Cys Thr Thr Val Thr Val Gly Ser
       450                 455                 460
   Asp Gly Asn Val Pro Val Pro Met Ala Gly Gly Leu Pro Arg Val Leu
   465                 470                 475                 480
   Tyr Pro Thr Glu Lys Leu Ala Gly Ser Lys Ile Cys Ser Ser Ser
                   485                 490                 495
<210> 41
   <211> 495
   <212> PRT
   <213> Artificial sequence
<220>
   <223> Fungal amylase variant
<400> 41
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Met Gly Phe Thr Ala Ile Trp Ile Thr
   65                  70                  75                  80
   Pro Val Thr Lys Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Lys Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Glu Trp Tyr Asp Trp Val
           195                 200                 205
   Lys Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Ile Tyr
               260                 265                 270
   Tyr Pro Leu Leu Asn Ala Phe Lys Ser Thr Ser Gly Ser Met Asp Asp
           275                 280                 285
   Leu Tyr Asn Met Ile Asn Thr Val Lys Ser Asp Cys Lys Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Lys Leu Ile Ala Ser Ala Asn Lys
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Met Lys Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Lys Leu Thr Glu Val Ile Gly Cys Thr Thr Val Thr Val Gly Ser
       450                 455                 460
   Asp Gly Asn Val Pro Val Pro Met Ala Gly Gly Leu Pro Lys Val Leu
   465 470 475 480
   Tyr Pro Thr Glu Lys Leu Ala Gly Ser Lys Ile Cys Ser Ser Ser
   485 490 495
<210> 42
   <211> 495
   <212> PRT
   <213> Artificial sequence
<220>
   <223> Fungal amylase variant
<400> 42
   Met Val Lys Ser Ile Leu Ala Ser Val Phe Phe Ala Ala Thr Ala Leu
   1 5 10 15
   Ala Ala Thr Pro Ala Asp Trp Lys Ser Gln Ser Ile Tyr Phe Leu Leu
   20 25 30
   Thr Asp Arg Phe Ala Arg Thr Asp Gly Ser Thr Thr Ala Thr Cys Asn
   35 40 45
   Thr Ala Asp Gln Lys Tyr Cys Gly Gly Thr Trp Lys Gly Ile Ile Asp
   50 55 60
   Lys Leu Asp Tyr Ile Gln Gly Met Gly Phe Thr Ala Ile Trp Ile Thr
   65 70 75 80
   Pro Val Thr Lys Gln Leu Pro Gln Thr Thr Ala Tyr Gly Asp Ala Tyr
   85 90 95
   His Gly Tyr Trp Gln Gln Asp Ile Tyr Ser Leu Asn Glu Asn Tyr Gly
   100 105 110
   Thr Ala Asp Asp Leu Lys Ala Leu Ser Ser Ala Leu His Glu Arg Gly
   115 120 125
   Met Tyr Leu Met Val Asp Val Val Ala Asn His Met Gly Tyr Asp Gly
   130 135 140
   Ala Gly Ser Ser Val Asp Tyr Ser Val Phe Lys Pro Phe Ser Ser Gln
   145 150 155 160
   Asp Tyr Phe His Pro Phe Cys Phe Ile Gln Asn Tyr Glu Asp Gln Thr
   165 170 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Glu Trp Tyr Asp Trp Val
           195                 200                 205
   Lys Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Ile Tyr
               260                 265                 270
   Tyr Pro Leu Leu Asn Ala Phe Lys Ser Thr Ser Gly Ser Met Asp Asp
           275                 280                 285
   Leu Tyr Asn Met Ile Asn Thr Val Lys Ser Asp Cys Lys Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Lys Leu Ile Ala Lys Ala Asn Lys
       370                 375                 380
   Ile Arg Lys Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Met Lys Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
   435 440 445
   Gln Lys Leu Thr Glu Val Ile Gly Cys Thr Thr Val Thr Val Gly Ser
   450 455 460
   Asp Gly Lys Val Pro Val Pro Met Ala Gly Gly Leu Pro Lys Val Leu
   465 470 475 480
   Tyr Pro Thr Glu Lys Leu Ala Gly Ser Lys Ile Cys Ser Ser Ser
   485 490 495
<210> 43
   <211> 495
   <212> PRT
   <213> Artificial sequence
<220>
   <223> Fungal amylase variant
<400> 43
   Met Val Lys Ser Ile Leu Ala Ser Val Phe Phe Ala Ala Thr Ala Leu
   1 5 10 15
   Ala Ala Thr Pro Ala Asp Trp Arg Ser Gln Ser Ile Tyr Phe Leu Leu
   20 25 30
   Thr Asp Arg Phe Ala Arg Thr Asp Gly Ser Thr Thr Ala Thr Cys Asn
   35 40 45
   Thr Ala Asp Gln Lys Tyr Cys Gly Gly Thr Trp Gln Gly Ile Ile Asp
   50 55 60
   Lys Leu Asp Tyr Ile Gln Gly Met Gly Phe Thr Ala Ile Trp Ile Thr
   65 70 75 80
   Pro Val Thr Ala Gln Leu Pro Gln Thr Thr Ala Tyr Gly Asp Ala Tyr
   85 90 95
   His Gly Tyr Trp Gln Gln Asp Ile Tyr Ser Leu Asn Glu Asn Tyr Gly
   100 105 110
   Thr Ala Asp Asp Leu Lys Ala Leu Ser Ser Ala Leu His Glu Arg Gly
   115 120 125
   Met Tyr Leu Met Val Asp Val Val Ala Asn His Met Gly Tyr Asp Gly
   130 135 140
   Ala Gly Ser Ser Val Asp Tyr Ser Val Phe Asn Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Arg Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Asp Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Ile Tyr
               260                 265                 270
   Tyr Pro Leu Leu Asn Ala Phe Gln Ser Thr Ser Gly Ser Met Asp Asp
           275                 280                 285
   Leu Tyr Asn Met Ile Asn Thr Val Lys Ser Asp Cys Pro Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Lys Leu Ile Ala Ser Ala Asn Ala
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Gln Asp Asp Thr Thr Ile Ala Met Arg Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Gln Leu Thr Glu Val Ile Gly Cys Thr Thr Val Thr Val Gly Ser
       450                 455                 460
   Asp Gly Asn Val Pro Val Pro Met Ala Gly Gly Leu Pro Arg Val Leu
   465                 470                 475                 480
   Tyr Pro Thr Glu Lys Leu Ala Gly Ser Gly Ile Cys Ser Ser Ser
                   485                 490                 495
<210> 44
   <211> 495
   <212> PRT
   <213> Artificial sequence
<220>
   <223> Fungal amylase variant
<400> 44
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   His Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
   115 120 125
   Met Tyr Leu Met Val Asp Val Val Ala Asn His Met Gly Tyr Asp Gly
   130 135 140
   Ala Gly Ser Ser Val Asp Tyr Ser Val Phe Asn Pro Phe Ser Ser Gln
   145 150 155 160
   Asp Tyr Phe His Pro Phe Cys Phe Ile Gln Asn Tyr Glu Asp Gln Thr
   165 170 175
   Gln Val Glu Asp Cys Trp Leu Gly Asp Asn Thr Val Ser Leu Pro Asp
   180 185 190
   Leu Asp Thr Thr Lys Asp Val Val Lys Asn Glu Trp Tyr Asp Trp Val
   195 200 205
   Gly Ser Leu Val Ser Asn Tyr Ser Ile Asp Gly Leu Arg Ile Asp Thr
   210 215 220
   Val Lys His Val Gln Lys Asp Phe Trp Pro Gly Tyr Asn Ser Ala Ala
   225 230 235 240
   Gly Val Tyr Cys Ile Gly Glu Val Leu Asp Gly Asp Pro Ala Tyr Thr
   245 250 255
   Cys Pro Tyr Gln Asn Val Met Asp Gly Val Leu Asn Tyr Pro Ile Tyr
   260 265 270
   Tyr Pro Leu Leu Asn Ala Phe Lys Ser Thr Ser Gly Ser Met Asp Asp
   275 280 285
   Leu Tyr Asn Met Ile Asn Thr Val Ala Ser Asp Cys Pro Asp Ser Thr
   290 295 300
   Leu Leu Gly Thr Phe Val Glu Asn His Asp Asn Pro Arg Phe Ala Ser
   305 310 315 320
   Tyr Thr Asn Asp Ile Ala Leu Ala Lys Asn Val Ala Ala Phe Ile Ile
   325 330 335
   Leu Asn Asp Gly Ile Pro Ile Ile Tyr Ala Gly Gln Glu Gln His Tyr
   340 345 350
   Ala Gly Gly Asn Asp Pro Ala Asn Arg Glu Ala Thr Trp Leu Ser Gly
   355 360 365
   Tyr Pro Thr Asp Ser Glu Leu Tyr Gln Leu Ile Ala Ser Ala Asn Ala
   370 375 380
   Ile Arg Asn Tyr Ala Ile Ser Lys Asp Thr Gly Phe Val Thr Tyr Lys
   385 390 395 400
   Asn Trp Pro Ile Tyr Lys Asp Asp Thr Thr Ile Ala Met Arg Lys Gly
   405 410 415
   Thr Asp Gly Ser Gln Ile Val Thr Ile Leu Ser Asn Lys Gly Ala Ser
   420 425 430
   Gly Asp Ser Tyr Thr Leu Ser Leu Ser Gly Ala Gly Tyr Thr Ala Gly
   435 440 445
   Gln Gln Leu Thr Glu Val Ile Gly Cys Thr Thr Val Thr Val Gly Ser
   450 455 460
   Asp Gly Asn Val Pro Val Pro Met Ala Gly Gly Leu Pro Arg Val Leu
   465 470 475 480
   Tyr Pro Thr Glu Lys Leu Ala Gly Ser Gly Ile Cys Ser Ser Ser
   485 490 495
<210> 45
   <211> 495
   <212> PRT
   <213> Artificial sequence
<220>
   <223> Fungal amylase variant
<400> 45
   Met Val Lys Ser Ile Leu Ala Ser Val Phe Phe Ala Ala Thr Ala Leu
   1 5 10 15
   Ala Ala Thr Pro Ala Asp Trp Arg Ser Gln Ser Ile Tyr Phe Leu Leu
   20 25 30
   Thr Asp Arg Phe Ala Arg Thr Asp Gly Ser Thr Thr Ala Thr Cys Asn
   35 40 45
   Thr Ala Asp Gln Lys Tyr Cys Gly Gly Thr Trp Gln Gly Ile Ile Asp
   50 55 60
   Lys Leu Asp Tyr Ile Gln Gly Met Gly Phe Thr Ala Ile Trp Ile Thr
   65 70 75 80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Arg Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Lys Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Lys Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Ile Tyr
               260                 265                 270
   Tyr Pro Leu Leu Asn Ala Phe Gln Ser Thr Ser Gly Ser Met Asp Asp
           275                 280                 285
   Leu Tyr Asn Met Ile Asn Thr Val Lys Ser Asp Cys Pro Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
   340 345 350
   Ala Gly Gly Asn Asp Pro Ala Asn Arg Glu Ala Thr Trp Leu Ser Gly
   355 360 365
   Tyr Pro Thr Asp Ser Glu Leu Tyr Lys Leu Ile Ala Ser Ala Asn Ala
   370 375 380
   Ile Arg Asn Tyr Ala Ile Ser Gln Asp Thr Gly Phe Val Thr Tyr Gln
   385 390 395 400
   Asn Trp Pro Ile Tyr Gln Asp Asp Thr Thr Ile Ala Met Arg Lys Gly
   405 410 415
   Thr Asp Gly Ser Gln Ile Val Thr Ile Leu Ser Asn Leu Gly Ala Ser
   420 425 430
   Gly Asp Ser Tyr Thr Leu Ser Leu Ser Gly Ala Gly Tyr Thr Ala Gly
   435 440 445
   Gln Gln Leu Thr Glu Val Ile Gly Cys Thr Thr Val Thr Val Gly Ser
   450 455 460
   Asp Gly Asn Val Pro Val Pro Met Ala Gly Gly Leu Pro Arg Val Leu
   465 470 475 480
   Tyr Pro Thr Glu Lys Leu Ala Gly Ser Lys Ile Cys Ser Ser Ser
   485 490 495
<210> 46
   <211> 495
   <212> PRT
   <213> Artificial sequence
<220>
   <223> Fungal amylase variant
<400> 46
   Met Val Lys Ser Ile Leu Ala Ser Val Phe Phe Ala Ala Thr Ala Leu
   1 5 10 15
   Ala Ala Thr Pro Ala Asp Trp Arg Ser Gln Ser Ile Tyr Phe Leu Leu
   20 25 30
   Thr Asp Arg Phe Ala Arg Thr Asp Gly Ser Thr Thr Ala Thr Cys Asn
   35 40 45
   Thr Ala Asp Gln Lys Tyr Cys Gly Gly Thr Trp Gln Gly Ile Ile Asp
       50                  55                  60
   His Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Lys Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Asn Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Arg Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Asp Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Ile Tyr
               260                 265                 270
   Tyr Pro Leu Leu Asn Ala Phe Gln Ser Thr Ser Gly Ser Met Asp Asp
           275                 280                 285
   Leu Tyr Asn Met Ile Asn Thr Val Lys Ser Asp Cys Pro Asp Ser Thr
       290                 295                 300
   Leu Leu Gly Thr Phe Val Glu Asn His Asp Asn Pro Arg Phe Ala Ser
   305 310 315 320
   Tyr Thr Asn Asp Ile Ala Leu Ala Lys Asn Val Ala Ala Phe Ile Ile
   325 330 335
   Leu Asn Asp Gly Ile Pro Ile Ile Tyr Ala Gly Gln Glu Gln His Tyr
   340 345 350
   Ala Gly Gly Asn Asp Pro Ala Asn Arg Glu Ala Thr Trp Leu Ser Gly
   355 360 365
   Tyr Pro Thr Asp Ser Glu Leu Tyr Gln Leu Ile Ala Ser Ala Asn Ala
   370 375 380
   Ile Arg Asn Tyr Ala Ile Ser Ala Asp Thr Gly Phe Val Thr Tyr Lys
   385 390 395 400
   Asn Trp Pro Ile Tyr Gln Asp Asp Thr Thr Ile Ala Met Arg Lys Gly
   405 410 415
   Thr Asp Gly Ser Gln Ile Val Thr Ile Leu Ser Asn Leu Gly Ala Ser
   420 425 430
   Gly Asp Ser Tyr Thr Leu Ser Leu Ser Gly Ala Gly Tyr Thr Ala Gly
   435 440 445
   Gln Gln Leu Thr Glu Val Ile Gly Cys Thr Thr Val Thr Val Gly Ser
   450 455 460
   Asp Gly Asn Val Pro Val Pro Met Ala Gly Gly Leu Pro Arg Val Leu
   465 470 475 480
   Tyr Pro Thr Glu Lys Leu Ala Gly Ser Gly Ile Cys Ser Ser Ser
   485 490 495
<210> 47
   <211> 495
   <212> PRT
   <213> Artificial sequence
<220>
   <223> Fungal amylase variant
<400> 47
   Met Val Lys Ser Ile Leu Ala Ser Val Phe Phe Ala Ala Thr Ala Leu
   1 5 10 15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   Lys Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Arg Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Asn Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Lys Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Ser Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Ile Tyr
               260                 265                 270
   Tyr Pro Leu Leu Asn Ala Phe Gln Ser Thr Ser Gly Ser Met Asp Asp
   275 280 285
   Leu Tyr Asn Met Ile Asn Thr Val Ala Ser Asp Cys Pro Asp Ser Thr
   290 295 300
   Leu Leu Gly Thr Phe Val Glu Asn His Asp Asn Pro Arg Phe Ala Ser
   305 310 315 320
   Tyr Thr Asn Asp Ile Ala Leu Ala Lys Asn Val Ala Ala Phe Ile Ile
   325 330 335
   Leu Asn Asp Gly Ile Pro Ile Ile Tyr Ala Gly Gln Glu Gln His Tyr
   340 345 350
   Ala Gly Gly Asn Asp Pro Ala Asn Arg Glu Ala Thr Trp Leu Ser Gly
   355 360 365
   Tyr Pro Thr Asp Ser Glu Leu Tyr Gln Leu Ile Ala Ser Ala Asn Ala
   370 375 380
   Ile Arg Asn Tyr Ala Ile Ser Gln Asp Thr Gly Phe Val Thr Tyr Gln
   385 390 395 400
   Asn Trp Pro Ile Tyr Gln Asp Asp Thr Thr Ile Ala Met Arg Lys Gly
   405 410 415
   Thr Asp Gly Ser Gln Ile Val Thr Ile Leu Ser Asn Lys Gly Ala Ser
   420 425 430
   Gly Asp Ser Tyr Thr Leu Ser Leu Ser Gly Ala Gly Tyr Thr Ala Gly
   435 440 445
   Gln Gln Leu Thr Glu Val Ile Gly Cys Thr Thr Val Thr Val Gly Ser
   450 455 460
   Asp Gly Asn Val Pro Val Pro Met Ala Gly Gly Leu Pro Arg Val Leu
   465 470 475 480
   Tyr Pro Thr Glu Lys Leu Ala Gly Ser Gly Ile Cys Ser Ser Ser
   485 490 495
<210> 48
   <211> 495
   <212> PRT
   <213> Artificial sequence
<220>
   <223> Fungal amylase variant
<400> 48
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   His Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Arg Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Asn Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Arg Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Lys Asp Phe Trp Pro Gly Tyr Asn Ser Ala Ala
   225                 230                 235                 240
   Gly Val Tyr Cys Ile Gly Glu Val Leu Asp Gly Asp Pro Ala Tyr Thr
   245 250 255
   Cys Pro Tyr Gln Asn Val Met Asp Gly Val Leu Asn Tyr Pro Ile Tyr
   260 265 270
   Tyr Pro Leu Leu Asn Ala Phe Gln Ser Thr Ser Gly Ser Met Asp Asp
   275 280 285
   Leu Tyr Asn Met Ile Asn Thr Val Ala Ser Asp Cys Pro Asp Ser Thr
   290 295 300
   Leu Leu Gly Thr Phe Val Glu Asn His Asp Asn Pro Arg Phe Ala Ser
   305 310 315 320
   Tyr Thr Asn Asp Ile Ala Leu Ala Lys Asn Val Ala Ala Phe Ile Ile
   325 330 335
   Leu Asn Asp Gly Ile Pro Ile Ile Tyr Ala Gly Gln Glu Gln His Tyr
   340 345 350
   Ala Gly Gly Asn Asp Pro Ala Asn Arg Glu Ala Thr Trp Leu Ser Gly
   355 360 365
   Tyr Pro Thr Asp Ser Glu Leu Tyr Gln Leu Ile Ala Ser Ala Asn Ala
   370 375 380
   Ile Arg Asn Tyr Ala Ile Ser Gln Asp Thr Gly Phe Val Thr Tyr Gln
   385 390 395 400
   Asn Trp Pro Ile Tyr Gln Asp Asp Thr Thr Ile Ala Met Arg Lys Gly
   405 410 415
   Thr Asp Gly Ser Gln Ile Val Thr Ile Leu Ser Asn Leu Gly Ala Ser
   420 425 430
   Gly Asp Ser Tyr Thr Leu Ser Leu Ser Gly Ala Gly Tyr Thr Ala Gly
   435 440 445
   Gln Gln Leu Thr Glu Val Ile Gly Cys Thr Thr Val Thr Val Gly Ser
   450 455 460
   Asp Gly Asn Val Pro Val Pro Met Ala Gly Gly Leu Pro Arg Val Leu
   465 470 475 480
   Tyr Pro Thr Glu Lys Leu Ala Gly Ser Gly Ile Cys Ser Ser Ser
   485 490 495
<210> 49
   <211> 495
   <212> PRT
   <213> Artificial sequence
<220>
   <223> Fungal amylase variant
<400> 49
   Met Val Lys Ser Ile Leu Ala Ser Val Phe Phe Ala Ala Thr Ala Leu
   1               5                   10                  15
   Ala Ala Thr Pro Ala Asp Trp Arg Ser Gln Ser Ile Tyr Phe Leu Leu
               20                  25                  30
   Thr Asp Arg Phe Ala Arg Thr Asp Gly Ser Thr Thr Ala Thr Cys Asn
           35                  40                  45
   Thr Ala Asp Gln Lys Tyr Cys Gly Gly Thr Trp Gln Gly Ile Ile Asp
       50                  55                  60
   His Leu Asp Tyr Ile Gln Gly Met Gly Phe Thr Ala Ile Trp Ile Thr
   65                  70                  75                  80
   Pro Val Thr Ala Gln Leu Pro Gln Thr Thr Ala Tyr Gly Asp Ala Tyr
                   85                  90                  95
   His Gly Tyr Trp Gln Gln Asp Ile Tyr Ser Leu Asn Glu Asn Tyr Gly
               100                 105                 110
   Thr Ala Asp Asp Leu Arg Ala Leu Ser Ser Ala Leu His Glu Arg Gly
           115                 120                 125
   Met Tyr Leu Met Val Asp Val Val Ala Asn His Met Gly Tyr Asp Gly
       130                 135                 140
   Ala Gly Ser Ser Val Asp Tyr Ser Val Phe Asn Pro Phe Ser Ser Gln
   145                 150                 155                 160
   Asp Tyr Phe His Pro Phe Cys Phe Ile Gln Asn Tyr Glu Asp Gln Thr
                   165                 170                 175
   Gln Val Glu Asp Cys Trp Leu Gly Asp Asn Thr Val Ser Leu Pro Asp
               180                 185                 190
   Leu Asp Thr Thr Lys Asp Val Val Arg Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Lys His Val Gln Pro Asp Phe Trp Pro Gly Tyr Asn Ser Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Ile Tyr
               260                 265                 270
   Tyr Pro Leu Leu Asn Ala Phe Gln Ser Thr Ser Gly Ser Met Asp Asp
           275                 280                 285
   Leu Tyr Asn Met Ile Asn Thr Val Ala Ser Asp Cys Pro Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Lys Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Gln Leu Ile Ala Ser Ala Asn Ala
       370                 375                 380
   Ile Arg Asn Tyr Ala Ile Ser Gln Asp Thr Gly Phe Val Thr Tyr Gln
   385                 390                 395                 400
   Asn Trp Pro Ile Tyr Gln Asp Asp Thr Thr Ile Ala Met Arg Lys Gly
                   405                 410                 415
   Thr Asp Gly Ser Gln Ile Val Thr Ile Leu Ser Asn Leu Gly Ala Ser
               420                 425                 430
   Gly Asp Ser Tyr Thr Leu Ser Leu Ser Gly Ala Gly Tyr Thr Ala Gly
           435                 440                 445
   Gln Gln Leu Thr Glu Val Ile Gly Cys Thr Thr Val Thr Val Gly Ser
       450                 455                 460
   Asp Gly Asn Val Pro Val Pro Met Ala Gly Gly Leu Pro Arg Val Leu
   465 470 475 480
   Tyr Pro Thr Glu Arg Leu Ala Gly Ser Gly Ile Cys Ser Ser Ser
   485 490 495
<210> 50
   <211> 495
   <212> PRT
   <213> Artificial sequence
<220>
   <223> Fungal amylase variant
<400> 50
   Met Val Lys Ser Ile Leu Ala Ser Val Phe Phe Ala Ala Thr Ala Leu
   1 5 10 15
   Ala Ala Thr Pro Ala Asp Trp Arg Ser Gln Ser Ile Tyr Phe Leu Leu
   20 25 30
   Thr Asp Arg Phe Ala Arg Thr Asp Gly Ser Thr Thr Ala Thr Cys Asn
   35 40 45
   Thr Ala Asp Gln Ile Tyr Cys Gly Gly Thr Trp Gln Gly Ile Ile Asp
   50 55 60
   His Leu Asp Tyr Ile Gln Gly Met Gly Phe Thr Ala Ile Trp Ile Thr
   65 70 75 80
   Pro Val Thr Ala Gln Leu Pro Gln Thr Thr Ala Tyr Gly Asp Ala Tyr
   85 90 95
   His Gly Tyr Trp Gln Gln Asp Ile Tyr Ser Leu Asn Glu Asn Tyr Gly
   100 105 110
   Thr Ala Asp Asp Leu Arg Ala Leu Ser Ser Ala Leu His Glu Arg Gly
   115 120 125
   Met Tyr Leu Met Val Asp Val Val Ala Asn His Met Gly Tyr Asp Gly
   130 135 140
   Ala Gly Ser Ser Val Asp Tyr Ser Val Phe Asn Pro Phe Ser Ser Gln
   145 150 155 160
   Asp Tyr Phe His Pro Phe Cys Phe Ile Gln Asn Tyr Glu Asp Gln Thr
   165 170 175
   Gln Val Glu Asp Cys Trp Leu Gly Asp Asn Thr Val Ser Leu Pro Asp
               180                 185                 190
   Leu Asp Thr Thr Ser Asp Val Val Arg Asn Glu Trp Tyr Asp Trp Val
           195                 200                 205
   Gly Ser Leu Val Ser Asn Tyr Ser Ile Asp Gly Leu Arg Ile Asp Thr
       210                 215                 220
   Val Arg His Val Gln Pro Asp Phe Trp Pro Gly Tyr Asn Ser Ala Ala
   225                 230                 235                 240
   Gly Val Tyr Cys Ile Gly Glu Val Leu Asp Gly Asp Pro Ala Tyr Thr
                   245                 250                 255
   Cys Pro Tyr Gln Asn Val Met Asp Gly Val Leu Asn Tyr Pro Ile Tyr
               260                 265                 270
   Tyr Pro Leu Leu Asn Ala Phe Gln Ser Thr Ser Gly Ser Met Asp Asp
           275                 280                 285
   Leu Tyr Asn Met Ile Asn Thr Val Ala Ser Asp Cys Pro Asp Ser Thr
       290                 295                 300
   Leu Leu Gly Thr Phe Val Glu Asn His Asp Asn Pro Arg Phe Ala Ser
   305                 310                 315                 320
   Tyr Thr Asn Asp Ile Ala Leu Ala Gln Asn Val Ala Ala Phe Ile Ile
                   325                 330                 335
   Leu Asn Asp Gly Ile Pro Ile Ile Tyr Ala Gly Gln Glu Gln His Tyr
               340                 345                 350
   Ala Gly Gly Asn Asp Pro Ala Asn Arg Glu Ala Thr Trp Leu Ser Gly
           355                 360                 365
   Tyr Pro Thr Asp Ser Glu Leu Tyr Gln Leu Ile Ala Ser Ala Asn Ala
       370                 375                 380
   Ile Arg Asn Tyr Ala Ile Ser Gln Asp Thr Gly Phe Val Thr Tyr Gln
   385                 390                 395                 400
   Asn Trp Pro Ile Tyr Gln Asp Asp Thr Thr Ile Ala Met Arg Arg Gly
                   405                 410                 415
   Thr Asp Gly Ser Gln Ile Val Thr Ile Leu Ser Asn Leu Gly Ala Ser
               420                 425                 430
   Gly Asp Ser Tyr Thr Leu Ser Leu Ser Gly Ala Gly Tyr Thr Ala Gly
   435 440 445
   Gln Gln Leu Thr Glu Val Ile Gly Cys Thr Thr Val Thr Val Gly Ser
   450 455 460
   Asp Gly Asn Val Pro Val Pro Met Ala Gly Gly Leu Pro Arg Val Leu
   465 470 475 480
   Tyr Pro Thr Glu Arg Leu Ala Gly Ser Gly Ile Cys Ser Ser Ser
   485 490 495
<210> 51
   <211> 495
   <212> PRT
   <213> Artificial sequence
<220>
   <223> Fungal amylase variant
<400> 51
   Met Val Lys Ser Ile Leu Ala Ser Val Phe Phe Ala Ala Thr Ala Leu
   1 5 10 15
   Ala Ala Thr Pro Ala Asp Trp Arg Ser Gln Ser Ile Tyr Phe Leu Leu
   20 25 30
   Thr Asp Arg Phe Ala Arg Thr Asp Gly Ser Thr Thr Ala Thr Cys Asn
   35 40 45
   Thr Ala Asp Gln Lys Tyr Cys Gly Gly Thr Trp Gln Gly Ile Ile Asp
   50 55 60
   Lys Leu Asp Tyr Ile Gln Gly Met Gly Phe Thr Ala Ile Trp Ile Thr
   65 70 75 80
   Pro Val Thr Ala Gln Leu Pro Gln Thr Thr Ala Tyr Gly Asp Ala Tyr
   85 90 95
   His Gly Tyr Trp Gln Gln Asp Ile Tyr Ser Leu Asn Glu Asn Tyr Gly
   100 105 110
   Thr Ala Asp Asp Leu Lys Ala Leu Ser Ser Ala Leu His Glu Arg Gly
   115 120 125
   Met Tyr Leu Met Val Asp Val Val Ala Asn His Met Gly Tyr Asp Gly
   130 135 140
   Ala Gly Ser Ser Val Asp Tyr Ser Val Phe Asn Pro Phe Ser Ser Gln
   145 150 155 160
   Asp Tyr Phe His Pro Phe Cys Phe Ile Gln Asn Tyr Glu Asp Gln Thr
   165 170 175
   Gln Val Glu Asp Cys Trp Leu Gly Asp Asn Thr Val Ser Leu Pro Asp
   180 185 190
   Leu Asp Thr Thr Lys Asp Val Val Arg Asn Glu Trp Tyr Asp Trp Val
   195 200 205
   Gly Ser Leu Val Ser Asn Tyr Ser Ile Asp Gly Leu Arg Ile Asp Thr
   210 215 220
   Val Lys His Val Gln Lys Asp Phe Trp Pro Gly Tyr Asn Asp Ala Ala
   225 230 235 240
   Gly Val Tyr Cys Ile Gly Glu Val Leu Asp Gly Asp Pro Ala Tyr Thr
   245 250 255
   Cys Pro Tyr Gln Asn Val Leu Asp Gly Val Leu Asn Tyr Pro Ile Tyr
   260 265 270
   Tyr Pro Leu Leu Asn Ala Phe Gln Ser Thr Ser Gly Ser Met Asp Asp
   275 280 285
   Leu Tyr Asn Met Ile Asn Thr Val Lys Ser Asp Cys Pro Asp Ser Thr
   290 295 300
   Leu Leu Gly Thr Phe Val Glu Asn His Asp Asn Pro Arg Phe Ala Ser
   305 310 315 320
   Tyr Thr Asn Asp Ile Ala Leu Ala Lys Asn Val Ala Ala Phe Ile Ile
   325 330 335
   Leu Asn Asp Gly Ile Pro Ile Ile Tyr Ala Gly Gln Glu Gln His Tyr
   340 345 350
   Ala Gly Gly Asn Asp Pro Ala Asn Arg Glu Ala Thr Trp Leu Ser Gly
   355 360 365
   Tyr Pro Thr Asp Ser Glu Leu Tyr Lys Leu Ile Ala Ser Ala Asn Ala
   370 375 380
   Ile Arg Asn Tyr Ala Ile Ser Lys Asp Thr Gly Phe Val Thr Tyr Lys
   385 390 395 400
   Asn Trp Pro Ile Tyr Gln Asp Asp Thr Thr Ile Ala Leu Arg Lys Gly
   405 410 415
   Thr Asp Gly Ser Gln Ile Val Thr Ile Leu Ser Asn Lys Gly Ala Ser
   420 425 430
   Gly Asp Ser Tyr Thr Leu Ser Leu Ser Gly Ala Gly Tyr Thr Ala Gly
   435 440 445
   Gln Gln Leu Thr Glu Val Ile Gly Cys Thr Thr Val Thr Val Gly Ser
   450 455 460
   Asp Gly Asn Val Pro Val Pro Leu Ala Gly Gly Leu Pro Arg Val Leu
   465 470 475 480
   Tyr Pro Thr Glu Lys Leu Ala Gly Ser Gly Ile Cys Ser Ser Ser
   485 490 495
<210> 52
   <211> 495
   <212> PRT
   <213> Artificial sequence
<220>
   <223> Fungal amylase variant
<400> 52
   Met Val Lys Ser Ile Leu Ala Ser Val Phe Phe Ala Ala Thr Ala Leu
   1 5 10 15
   Ala Ala Thr Pro Ala Asp Trp Arg Ser Gln Ser Ile Tyr Phe Leu Leu
   20 25 30
   Thr Asp Arg Phe Ala Arg Thr Asp Gly Ser Thr Thr Ala Thr Cys Asn
   35 40 45
   Thr Ala Asp Gln Lys Tyr Cys Gly Gly Thr Trp Gln Gly Ile Ile Asp
   50 55 60
   His Leu Asp Tyr Ile Gln Gly Met Gly Phe Thr Ala Ile Trp Ile Thr
   65 70 75 80
   Pro Val Thr Ala Gln Leu Pro Gln Thr Thr Ala Tyr Gly Asp Ala Tyr
   85 90 95
   His Gly Tyr Trp Gln Gln Asp Ile Tyr Ser Leu Asn Glu Asn Tyr Gly
   100 105 110
   Thr Ala Asp Asp Leu Arg Ala Leu Ser Ser Ala Leu His Glu Arg Gly
   115 120 125
   Met Tyr Leu Met Val Asp Val Val Ala Asn His Met Gly Tyr Asp Gly
   130 135 140
   Ala Gly Ser Ser Val Asp Tyr Ser Val Phe Asn Pro Phe Ser Ser Gln
   145 150 155 160
   Asp Tyr Phe His Pro Phe Cys Phe Ile Gln Asn Tyr Glu Asp Gln Thr
   165 170 175
   Gln Val Glu Asp Cys Trp Leu Gly Asp Asn Thr Val Ser Leu Pro Asp
   180 185 190
   Leu Asp Thr Thr Lys Asp Val Val Arg Asn Glu Trp Tyr Asp Trp Val
   195 200 205
   Gly Ser Leu Val Ser Asn Tyr Ser Ile Asp Gly Leu Arg Ile Asp Thr
   210 215 220
   Val Lys His Val Gln Lys Asp Phe Trp Pro Gly Tyr Asn Ser Ala Ala
   225 230 235 240
   Gly Val Tyr Cys Ile Gly Glu Val Leu Asp Gly Asp Pro Ala Tyr Thr
   245 250 255
   Cys Pro Tyr Gln Asn Val Leu Asp Gly Val Leu Asn Tyr Pro Ile Tyr
   260 265 270
   Tyr Pro Leu Leu Asn Ala Phe Gln Ser Thr Ser Gly Ser Ile Asp Asp
   275 280 285
   Leu Tyr Asn Glu Ile Asn Thr Val Lys Ser Asp Cys Pro Asp Ser Thr
   290 295 300
   Leu Leu Gly Thr Phe Val Glu Asn His Asp Asn Pro Arg Phe Ala Ser
   305 310 315 320
   Tyr Thr Asn Asp Ile Ala Leu Ala Lys Asn Val Ala Ala Phe Ile Ile
   325 330 335
   Leu Asn Asp Gly Ile Pro Ile Ile Tyr Ala Gly Gln Glu Gln His Tyr
   340 345 350
   Ala Gly Gly Asn Asp Pro Ala Asn Arg Glu Ala Thr Trp Leu Ser Gly
   355 360 365
   Tyr Pro Thr Asp Ser Glu Leu Tyr Lys Leu Ile Ala Ser Ala Asn Ala
       370                 375                 380
   Ile Arg Asn Tyr Ala Ile Ser Lys Asp Thr Gly Phe Val Thr Tyr Lys
   385                 390                 395                 400
   Asn Trp Pro Ile Tyr Lys Asp Asp Thr Thr Ile Ala Met Arg Lys Gly
                   405                 410                 415
   Thr Asp Gly Ser Gln Ile Val Thr Ile Leu Ser Asn Lys Gly Ala Ser
               420                 425                 430
   Gly Asp Ser Tyr Thr Leu Ser Leu Ser Gly Ala Gly Tyr Thr Ala Gly
           435                 440                 445
   Gln Gln Leu Thr Glu Val Ile Gly Cys Thr Thr Val Thr Val Gly Ser
       450                 455                 460
   Asp Gly Asn Val Pro Val Pro Met Ala Gly Gly Leu Pro Arg Val Leu
   465                 470                 475                 480
   Tyr Pro Thr Glu Lys Leu Ala Gly Ser Lys Ile Cys Ser Ser Ser
                   485                 490                 495
<210> 53
   <211> 1368
   <212> DNA
   <213> Aspergillus niger
<400> 53
   atgctccgcc gtgccttgct tctgtcttct tctgccatcc tggccgtcaa ggcccagcag      60
   gctggtactg ccactgctga gaaccaccct cctctgacct ggcaggagtg cactgctcct     120
   ggctcttgca ccacccagaa tggtgctgtt gttctcgact cgaactggag atgggttcac     180
   aacgttggtg gatacaccaa ctgctacact ggcaacacct ggaaccccac ctactgcccc     240
   gacgatgtca cctgcgcgga gaactgcgct cttgacggtg ccgactacga gggcacctac     300
   ggtgtcacct cctccggcag cgaactccgt ctgaacttcg tcaccggaag caacgtcggc     360
   tctcgtctgt accttctcca ggatgacgaa acctaccaga tcttcaagct gctcaaccgc     420
   gagttcacct tcgatgtcga tgtctccaac cttccttgcg gtctgaacgg tgccctctac     480
   ttcgtcgcca tggatgcgga cggtggtgtc agcaagtacc ccaacaacaa ggccggtgcc     540
   aagtacggca ctggatactg cgactctcag tgcccccgtg acctcaagtt cattgatggc     600
   gagggcaacg tggaaggctg gcagccctcc tccaacaacg ccaacactgg tatcggtgac     660
   cacggatcct gctgcgctga gatggatgtc tgggaggcca actcgatctc caacgccgtc     720
   actcctcacc cttgcgacac ccccggccag accatgtgcg atggagatga ctgcggtgga     780
   acctacagca ccaaccgcta cgctggtgag tgcgaccccg acggctgcga cttcaacccc     840
   taccgcatgg gtaacacctc gttctacggc cccggcaaga tcattgacac cactcagcct     900
   ttcactgttg tcacccagtt cctgaccgat gatggcaccg acaccggcac cctcagcgaa     960
   atcaagcgct tctacatcca gaacggcaag gtcattcctc agcccaactc cgacatctcc    1020
   ggtgtgaccg gcaactccat caccactgag ttctgcaccg ctcagaagca ggctttcggt    1080
   gacaccgatg acttctccca gcacggtggt cttgccaaga tgggtgctgc catgcagcag    1140
   ggtatggtcc tcgtcatgtc cctctgggat gactacgctg ctcagatgct ctggttggac    1200
   tccgactacc ccaccaacgc ctctgccacc actcccggtg ttgctcgtgg aacttgcccc    1260
   accgacagcg gtgttccctc ccaggttgag agccagtctc ccaacagcta cgtgacctac    1320
   tccaacatca agttcggtcc catcaactcg accttcactg cctcctaa                 1368
<210> 54
   <211> 1488
   <212> DNA
   <213> Aspergillus niger
<400> 54
   atggtcaagt ccatcctggc ctccgtcttc ttcgctgcca ctgctcttgc tgccaccccc 60
   gccgactggc gctcccagtc catctacttc ctgctcaccg accgtttcgc tcgcaccgac 120
   ggctccacta ctgccacctg caacactgcg gaccagaagt actgcggtgg cacctggcag 180
   ggtatcattg acaagctcga ctacatccag ggtatgggtt tcactgccat ctggatcacc 240
   cccgtcactg ctcagctccc ccagaccact gcctacggtg atgcctacca cggatactgg 300
   cagcaggata tctactctct gaacgagaac tacggtactg ccgatgacct caaggccctt 360
   tcttctgctc tgcacgagcg tggcatgtac ctgatggttg atgttgttgc caaccacatg 420
   ggatacgatg gagcgggcag cagcgttgac tactccgtct tcaagccctt ctcctcccag 480
   gactacttcc accccttctg cttcatccag aattacgagg accagaccca ggttgaggac 540
   tgctggttgg gtgacaacac tgtctctctg cccgatctcg acaccaccaa ggatgttgtc 600
   aagaacgaat ggtacgactg ggtgggctct cttgtctcca actactccat cgatggcctc 660
   cgtatcgaca ctgtcaagca cgtccagaag gacttctggc ctggatacaa caaggctgct 720
   ggtgtctact gcattggtga ggtcctcgat ggtgaccctg cctacacctg cccctaccag 780
   aacgtcatgg atggtgtcct caactaccct atctactacc ctctgctcaa cgccttcaag 840
   tccacctccg gcagcatgga tgacctgtac aacatgatca acaccgtcaa gtccgactgc 900
   cccgactcga ccctccttgg caccttcgtc gagaaccacg acaaccctcg cttcgcctcc 960
   tacaccaacg acattgccct tgccaagaac gtcgctgctt tcatcatcct caacgacggt 1020
   atccccatca tctatgctgg ccaggagcag cactacgctg gtggcaacga ccccgccaac 1080
   cgtgaggcca cctggctctc cggctaccct accgacagcg aactctacaa gttgattgcc 1140
   tccgccaacg ccatccgcaa ctacgccatc tccaaggaca ccggtttcgt cacctacaag 1200
   aactggccca tctacaagga tgataccacc attgccatgc gcaagggcac cgatggctcc 1260
   cagatcgtca ccattttgtc gaacaagggt gcttctggtg acagctacac tctgtctctt 1320
   tctggtgctg gctacactgc cggtcagcag ctgaccgaag tcattggatg caccaccgtc 1380
   accgttggct ccgatggcaa cgtccccgtg cccatggccg gtggtcttcc tcgtgtcctc 1440
   taccccactg agaagctggc tggaagcaag atctgctcga gcagctaa 1488
<210> 55
   <211> 1368
   <212> DNA
   <213> Artificial sequence
<220>
   <223> Cellbiohydrolase 1 variant
<400> 55
   atgctccgcc gtgccttgct tctgtcttct tctgccatcc tggccgtcaa ggcccagcag      60
   gctggtactg ccactgctga gaaccaccct cctctgacct ggcaggagtg cactgctcct     120
   ggctcttgca ccacccagaa tggtgctgtt gttctcgact cgaactggag atgggttcac     180
   aacgttggtg gatacaccaa ctgctacact ggcaacacct ggaaccccac ctactgcccc     240
   gacgatgtca cctgcgcgga gaactgcgct cttgacggtg ccgactacga gggcacctac     300
   ggtgtcacct cctccggcag cgaactccgt ctgaacttcg tcaccggaag caacgtcggc     360
   tctcgtctgt accttctcca ggatgacgaa acctaccaga tcttcaagct gctcaaccgc     420
   gagttcacct tcgatgtcga tgtctccaac cttccttgcg gtctgaacgg tgccctctac     480
   ttcgtcgcca tggatgcgga cggtggtgtc agcaagtacc ccaacaacaa ggccggtgcc     540
   aagtacggca ctggatactg cgactctcag tgcccccgtg acctcaagtt cattgatggc     600
   gagggcaacg tggaaggctg gcagccctcc tccaacaacg ccaacactgg tatcggtgac     660
   cacggatcct gctgcgctga gatggatgtc tgggaggcca actcgatctc caacgccgtc     720
   actcctcacc cttgcgacac ccccggccag acccgctgcg atggagatga ctgcggtgga     780
   acctacagca ccaaccgcta cgctggtgag tgcgaccccg acggctgcga cttcaacccc     840
   taccgcctcg gtaacacctc gttctacggc cccggcaaga tcattgacac cactcagcct     900
   ttcactgttg tcacccagtt cctgaccgat gatggcaccg acaccggcac cctcagcgaa     960
   atcaagcgct tctacatcca gaacggcaag gtcattcctc agcccaactc cgacatctcc    1020
   ggtgtgaccg gcaactccat caccactgag ttctgcaccg ctcagaagca ggctttcggt    1080
   gacaccgatg acttctccca gcacggtggt cttgccaaga tgggtgctgc catgcagcag    1140
   ggtatggtcc tcgtcctctc cctctgggat gactacgctg ctcagatgct ctggttggac    1200
   tccgactacc ccaccaacgc ctctgccacc actcccggtg ttgctcgtgg aacttgcccc    1260
   accgacagcg gtgttccctc ccaggttgag agccagtctc ccaacagcta cgtgacctac    1320
   tccaacatca agttcggtcc catcaactcg accttcactg cctcctaa                 1368
<210> 56
   <211> 1368
   <212> DNA
   <213> Artificial sequence
<220>
   <223> Cellbiohydrolase 1 variant
<400> 56
   atgctccgcc gtgccttgct tctgtcttct tctgccatcc tggccgtcaa ggcccagcag 60
   gctggtactg ccactgctga gaaccaccct cctctgacct ggcaggagtg cactgctcct 120
   ggctcttgca ccacccagaa tggtgctgtt gttctcgact cgaactggag atgggttcac 180
   aacgttggtg gatacaccaa ctgctacact ggcaacacct ggaaccccac ctactgcccc 240
   gacgatgtca cctgcgcgga gaactgcgct cttgacggtg ccgactacga gggcacctac 300
   ggtgtcacct cctccggcag cgaactccgt ctgaacttcg tcaccggaag caacgtcggc 360
   tctcgtctgt accttctcca ggatgacgaa acctaccaga tcttcaagct gctcaaccgc 420
   gagttcacct tcgatgtcga tgtctccaac cttccttgcg gtctgaacgg tgccctctac 480
   ttcgtcgcca tggatgcgga cggtggtgtc agcaagtacc ccaacaacaa ggccggtgcc 540
   aagtacggca ctggatactg cgactctcag tgcccccgtg acctcaagtt cattgatggc 600
   gagggcaacg tggaaggctg gcagccctcc tccaacaacg ccaacactgg tatcggtgac 660
   cacggatcct gctgcgctga gatggatgtc tgggaggcca actcgatctc caacgccgtc 720
   actcctcacc cttgcgacac ccccggccag acccgctgcg atggagatga ctgcggtgga 780
   acctacagca ccaaccgcta cgctggtgag tgcgaccccg acggctgcga cttcaacccc 840
   taccgcatgg gtaacacctc gttctacggc cccggcaaga tcattgacac cactcagcct 900
   ttcactgttg tcacccagtt cctgaccgat gatggcaccg acaccggcac cctcagcgaa 960
   atcaagcgct tctacatcca gaacggcaag gtcattcctc agcccaactc cgacatctcc 1020
   ggtgtgaccg gcaactccat caccactgag ttctgcaccg ctcagaagca ggctttcggt 1080
   gacaccgatg acttctccca gcacggtggt cttgccaaga tcggtgctgc cctccagcag 1140
   ggtatggtcc tcgtcctctc cctctgggat gactacgctg ctcagatgct ctggttggac 1200
   tccgactacc ccaccaacgc ctctgccacc actcccggtg ttgctcgtgg aacttgcccc 1260
   accgacagcg gtgttccctc ccaggttgag agccagtctc ccaacagcta cgtgacctac 1320
   tccaacatca agttcggtcc catcaactcg accttcactg cctcctaa 1368
<210> 57
   <211> 1368
   <212> DNA
   <213> Artificial sequence
<220>
   <223> Cellobiohydrolase 1 variant
<400> 57
   atgctccgcc gtgccttgct tctgtcttct tctgccatcc tggccgtcaa ggcccagcag 60
   gctggtactg ccactgctga gaaccaccct cctctgacct ggcaggagtg cactgctcct 120
   ggctcttgca ccacccagaa tggtgctgtt gttctcgact cgaactggag atgggttcac 180
   aacgttggtg gatacaccaa ctgctacact ggcaacacct ggaaccccac ctactgcccc 240
   gacgatgtca cctgcgcgga gaactgcgct cttgacggtg ccgactacga gggcacctac 300
   ggtgtcacct cctccggcag cgaactccgt ctgaacttcg tcaccggaag caacgtcggc 360
   tctcgtctgt accttctcca ggatgacgaa acctaccaga tcttcaagct gctcaaccgc 420
   gagttcacct tcgatgtcga tgtctccaac cttccttgcg gtctgaacgg tgccctctac 480
   ttcgtcgcca tggatgcgga cggtggtgtc agcaagtacc ccaacaacaa ggccggtgcc 540
   aagtacggca ctggatactg cgactctcag tgcccccgtg acctcaagtt cattgatggc 600
   gagggcaacg tggaaggctg gcagccctcc tccaacaacg ccaacactgg tatcggtgac 660
   cacggatcct gctgcgctga gatggatgtc tgggaggcca actcgatctc caacgccgtc 720
   actcctcacc cttgcgacac ccccggccag acccgctgcg atggagatga ctgcggtgga 780
   acctacagca ccaaccgcta cgctggtgag tgcgaccccg acggctgcga cttcaacccc 840
   taccgcctcg gtaacacctc gttctacggc cccggcaaga tcattgacac cactcagcct 900
   ttcactgttg tcacccagtt cctgaccgat gatggcaccg acaccggcac cctcagcgaa 960
   atcaagcgct tctacatcca gaacggcaag gtcattcctc agcccaactc cgacatctcc 1020
   ggtgtgaccg gcaactccat caccactgag ttctgcaccg ctcagaagca ggctttcggt 1080
   gacaccgatg acttctccca gcacggtggt cttgccaaga tcggtgctgc cctccagcag 1140
   ggtatggtcc tcgtcatgtc cctctgggat gactacgctg ctcagatgct ctggttggac 1200
   tccgactacc ccaccaacgc ctctgccacc actcccggtg ttgctcgtgg aacttgcccc 1260
   accgacagcg gtgttccctc ccaggttgag agccagtctc ccaacagcta cgtgacctac 1320
   tccaacatca agttcggtcc catcaactcg accttcactg cctcctaa 1368
<210> 58
   <211> 1368
   <212> DNA
   <213> Artificial sequence
<220>
   <223> Cellobiohydrolase 1 variant
<400> 58
   atgctccgcc gtgccttgct tctgtcttct tctgccatcc tggccgtcaa ggcccagcag 60
   gctggtactg ccactgctga gaaccaccct cctctgacct ggcaggagtg cactgctcct 120
   ggctcttgca ccacccagaa tggtgctgtt gttctcgact cgaactggag atgggttcac 180
   aacgttggtg gatacaccaa ctgctacact ggcaacacct ggaaccccac ctactgcccc 240
   gacgatgtca cctgcgcgga gaactgcgct cttgacggtg ccgactacga gggcacctac 300
   ggtgtcacct cctccggcag cgaactccgt ctgaacttcg tcaccggaag caacgtcggc 360
   tctcgtctgt accttctcca ggatgacgaa acctaccaga tcttcaagct gctcaaccgc 420
   gagttcacct tcgatgtcga tgtctccaac cttccttgcg gtctgaacgg tgccctctac 480
   ttcgtcgcca tggatgcgga cggtggtgtc agcaagtacc ccaacaacaa ggccggtgcc 540
   aagtacggca ctggatactg cgactctcag tgcccccgtg acctcaagtt cattgatggc 600
   gagggcaacg tggaaggctg gcagccctcc tccaacaacg ccaacactgg tatcggtgac 660
   cacggatcct gctgcgctga gatggatgtc tgggaggcca actcgatctc caacgccgtc 720
   actcctcacc cttgcgacac ccccggccag acccgctgcg atggagatga ctgcggtgga 780
   acctacagca ccaaccgcta cgctggtgag tgcgaccccg acggctgcga cttcaacccc 840
   taccgcctcg gtaacacctc gttctacggc cccggcaaga tcattgacac cactcagcct 900
   ttcactgttg tcacccagtt cctgaccgat gatggcaccg acaccggcac cctcagcgaa 960
   atcaagcgct tctacatcca gaacggcaag gtcattcctc agcccaactc cgacatctcc 1020
   ggtgtgaccg gcaactccat caccactgag ttctgcaccg ctcagaagca ggctttcggt 1080
   gacaccgatg acttctccca gcacggtggt cttgccaaga tcggtgctgc cctccagcag 1140
   ggtatggtcc tcgtcctctc cctctgggat gactacgctg ctcagatgct ctggttggac 1200
   tccgactacc ccaccaacgc ctctgccacc actcccggtg ttgctcgtgg aacttgcccc 1260
   accgacagcg gtgttccctc ccaggttgag agccagtctc ccaacagcta cgtgacctac 1320
   tccaacatca agttcggtcc catcaactcg accttcactg cctcctaa 1368
<210> 59
   <211> 1368
   <212> DNA
   <213> Artificial sequence
<220>
   <223> Cellobiohydrolase 1 variant
<400> 59
   atgctccgcc gtgccttgct tctgtcttct tctgccatcc tggccgtcaa ggcccagcag 60
   gctggtactg ccactgctga gaaccaccct cctctgacct ggcaggagtg cactgctcct     120
   ggctcttgca ccacccagaa tggtgctgtt gttctcgact cgaactggag atgggttcac     180
   aacgttggtg gatacaccaa ctgctacact ggcaacacct ggaaccccac ctactgcccc     240
   gacgatgtca cctgcgcgga gaactgcgct cttgacggtg ccgactacga gggcacctac     300
   ggtgtcacct cctccggcag cgaactccgt ctgaacttcg tcaccggaag caacgtcggc     360
   tctcgtctgt accttctcca ggatgacgaa acctaccaga tcttcaagct gctcaaccgc     420
   gagttcacct tcgatgtcga tgtctccaac cttccttgcg gtctgaacgg tgccctctac     480
   ttcgtcgcca tggatgcgga cggtggtgtc agcaagtacc ccaacaacaa ggccggtgcc     540
   aagtacggca ctggatactg cgactctcag tgcccccgtg acctcaagtt cattgatggc     600
   gagggcaacg tggaaggctg gcagccctcc tccaacaacg ccaacactgg tatcggtgac     660
   cacggatcct gctgcgctga gcttgatgtc tgggaggcca actcgatctc caacgccgtc     720
   actcctcacc cttgcgacac ccccggccag acccgctgcg atggagatga ctgcggtgga     780
   acctacagca ccaaccgcta cgctggtgag tgcgaccccg acggctgcga cttcaacccc     840
   taccgcctcg gtaacacctc gttctacggc cccggcaaga tcattgacac cactcagcct     900
   ttcactgttg tcacccagtt cctgaccgat gatggcaccg acaccggcac cctcagcgaa     960
   atcaagcgct tctacatcca gaacggcaag gtcattcctc agcccaactc cgacatctcc    1020
   ggtgtgaccg gcaactccat caccactgag ttctgcaccg ctcagaagca ggctttcggt    1080
   gacaccgatg acttctccca gcacggtggt cttgccaagg tcggtgctgc cctccagcag    1140
   ggtatggtcc tcgtcctctc cctctgggat gactacgctg ctcagatgct ctggttggac    1200
   tccgactacc ccaccaacgc ctctgccacc actcccggtg ttgctcgtgg aacttgcccc    1260
   accgacagcg gtgttccctc ccaggttgag agccagtctc ccaacagcta cgtgacctac    1320
   tccaacatca agttcggtcc catcaactcg accttcactg cctcctaa                 1368
<210> 60
   <211> 1368
   <212> DNA
   <213> Artificial sequence
<220>
   <223> Cellobiohydrolase 1 variant
<400> 60
   atgctccgcc gtgccttgct tctgtcttct tctgccatcc tggccgtcaa ggcccagcag 60
   gctggtactg ccactgctga gaaccaccct cctctgacct ggcaggagtg cactgctcct 120
   ggctcttgca ccacccagaa tggtgctgtt gttctcgact cgaactggag atgggttcac 180
   aacgttggtg gatacaccaa ctgctacact ggcaacacct ggaaccccac ctactgcccc 240
   gacgatgtca cctgcgcgga gaactgcgct cttgacggtg ccgactacga gggcacctac 300
   ggtgtcacct cctccggcag cgaactccgt ctgaacttcg tcaccggaag caacgtcggc 360
   tctcgtctgt accttatgca ggatgacgaa acctaccaga tgttcaagct gctcaaccgc 420
   gagttcacct tcgatgtcga tgtctccaac cttccttgcg gtctgaacgg tgccctctac 480
   ttcgtcgcca tggatgcgga cggtggtatg agcaagtacc ccaacaacaa ggccggtgcc 540
   aagtacggca ctggatactg cgactctcag tgcccccgtg acctcaagtt cattgatggc 600
   gagggcaacg tggaaggctg gcagccctcc tccaacaacg ccaacactgg tatcggtgac 660
   cacggatcct gctgcgctga gatggatgtc tgggaggcca actcgatctc caacgccgtc 720
   actcctcacc cttgcgacac ccccggccag accatgtgcg atggagatga ctgcggtgga 780
   acctacagca ccaaccgcta cgctggtgag tgcgaccccg acggctgcga cttcaacccc 840
   taccgcatgg gtaacacctc gttctacggc cccggcaaga tcattgacac cactcagcct 900
   ttcactgttg tcacccagtt cctgaccgat gatggcaccg acaccggcac cctcagcgaa 960
   atcaagcgct tctacatcca gaacggcaag gtcattcctc agcccaactc cgacatctcc 1020
   ggtgtgaccg gcaactccat caccactgag ttctgcaccg ctcagaagca ggctttcggt 1080
   gacaccgatg acttctccca gcacggtggt atggccaaga tgggtgctgc catgcagcag 1140
   ggtatggtcc tcgtcatgtc cctctgggat gactacgctg ctcagatgct ctggttggac 1200
   tccgactacc ccaccaacgc ctctgccacc actcccggtg ttgctcgtgg aacttgcccc 1260
   accgacagcg gtgttccctc ccaggttgag agccagtctc ccaacagcta cgtgacctac 1320
   tccaacatca agttcggtcc catcaactcg accttcactg cctcctaa 1368
<210> 61
   <211> 1368
   <212> DNA
   <213> Artificial sequence
<220>
   <223> Cellobiohydrolase 1 variant
<400> 61
   atgctccgcc gtgccttgct tctgtcttct tctgccatcc tggccgtcaa ggcccagcag      60
   gctggtactg ccactgctga gaaccaccct cctatgacct ggcaggagtg cactgctcct     120
   ggctcttgca ccacccagaa tggtgctgtt gttctcgact cgaactggag atgggttcac     180
   aacgttggtg gatacaccaa ctgctacact ggcaacacct ggaaccccac ctactgcccc     240
   gacgatgtca cctgcgcgga gaactgcgct cttgacggtg ccgactacga gggcacctac     300
   ggtgtcacct cctccggcag cgaactccgt ctgaacttcg tcaccggaag caacgtcggc     360
   tctcgtatgt accttatgca ggatgacgaa acctaccaga tgttcaagct gctcaaccgc     420
   gagttcacct tcgatgtcga tgtctccaac cttccttgcg gtctgaacgg tgccctctac     480
   ttcgtcgcca tggatgcgga cggtggtatg agcaagtacc ccaacaacaa ggccggtgcc     540
   aagtacggca ctggatactg cgactctcag tgcccccgtg acctcaagtt cattgatggc     600
   gagggcaacg tggaaggctg gcagccctcc tccaacaacg ccaacactgg tatcggtgac     660
   cacggatcct gctgcgctga gatggatgtc tgggaggcca actcgatctc caacgccgtc     720
   actcctcacc cttgcgacac ccccggccag accatgtgcg atggagatga ctgcggtgga     780
   acctacagca ccaaccgcta cgctggtgag tgcgaccccg acggctgcga cttcaacccc     840
   taccgcatgg gtaacacctc gttctacggc cccggcaaga tcattgacac cactcagcct     900
   ttcactgttg tcacccagtt cctgaccgat gatggcaccg acaccggcac cctcagcgaa     960
   atcaagcgct tctacatcca gaacggcaag gtcattcctc agcccaactc cgacatctcc    1020
   ggtgtgaccg gcaactccat caccactgag ttctgcaccg ctcagaagca ggctttcggt    1080
   gacaccgatg acttctccca gcacggtggt cttgccaaga tgggtgctgc catgcagcag    1140
   ggtatggtcc tcgtcatgtc cctctgggat gactacgctg ctcagatgct ctggttggac    1200
   tccgactacc ccaccaacgc ctctgccacc actcccggtg ttgctcgtgg aacttgcccc    1260
   accgacagcg gtgttccctc ccaggttgag agccagtctc ccaacagcta cgtgacctac    1320
   tccaacatca agttcggtcc catcaactcg accttcactg cctcctaa                  1368
<210> 62
   <211> 1368
   <212> DNA
   <213> Artificial sequence
<220>
   <223> Cellobiohydrolase 1 variant
<400> 62
   atgctccgcc gtgccttgct tctgtcttct tctgccatcc tggccgtcaa ggcccagcag      60
   gctggtactg ccactgctga gaaccaccct cctatgacct ggcaggagtg cactgctcct     120
   ggctcttgca ccacccagaa tggtgctgtt gttctcgact cgaactggag atgggttcac     180
   aacgttggtg gatacaccaa ctgctacact ggcaacacct ggaaccccac ctactgcccc     240
   gacgatgtca cctgcgcgga gaactgcgct cttgacggtg ccgactacga gggcacctac     300
   ggtgtcacct cctccggcag cgaactccgt ctgaacttcg tcaccggaag caacgtcggc     360
   tctcgtatgt accttatgca ggatgacgaa acctaccaga tgttcaagct gctcaaccgc     420
   gagttcacct tcgatgtcga tgtctccaac cttccttgcg gtctgaacgg tgccctctac     480
   ttcgtcgcca tggatgcgga cggtggtatg agcaagtacc ccaacaacaa ggccggtgcc     540
   aagtacggca ctggatactg cgactctcag tgcccccgtg acctcaagtt cattgatggc     600
   gagggcaacg tggaaggctg gcagccctcc tccaacaacg ccaacactgg tatcggtgac     660
   cacggatcct gctgcgctga gatggatgtc tgggaggcca actcgatctc caacgccgtc     720
   actcctcacc cttgcgacac ccccggccag accatgtgcg atggagatga ctgcggtgga     780
   acctacagca ccaaccgcta cgctggtgag tgcgaccccg acggctgcga cttcaacccc     840
   taccgcatgg gtaacacctc gttctacggc cccggcatga tcattgacac cactcagcct     900
   ttcactgttg tcacccagtt cctgaccgat gatggcaccg acaccggcac cctcagcgaa     960
   atcaagcgct tctacatcca gaacggcaag gtcattcctc agcccaactc cgacatctcc    1020
   ggtgtgaccg gcaactccat caccactgag ttctgcaccg ctcagaagca ggctttcggt    1080
   gacaccgatg acttctccca gcacggtggt atggccaaga tgggtgctgc catgcagcag    1140
   ggtatggtcc tcgtcatgtc cctctgggat gactacgctg ctcagatgct ctggttggac    1200
   tccgactacc ccaccaacgc ctctgccacc actcccggtg ttgctcgtgg aacttgcccc    1260
   accgacagcg gtgttccctc ccaggttgag agccagtctc ccaacagcta cgtgacctac    1320
   tccaacatca agttcggtcc catcaactcg accttcactg cctcctaa                 1368
<210> 63
   <211> 1368
   <212> DNA
   <213> Artificial sequence
<220>
   <223> Cellobiohydrolase 1 variant
<400> 63
   atgctccgcc gtgccttgct tctgtcttct tctgccatcc tggccgtcaa ggcccagcag 60
   gctggtactg ccactgctga gaaccaccct cctctgacct ggcaggagtg cactgctcct 120
   ggctcttgca ccacccagaa tggtgctgtt gttctcgact cgaactggag atgggttcac 180
   aacgttggtg gatacaccaa ctgctacact ggcaacacct ggaaccccac ctactgcccc 240
   gacgatgtca cctgcgcgga gaactgcgct cttgacggtg ccgactacga gggcacctac 300
   ggtgtcacct cctccggcag cgaactccgt ctgaacttcg tcaccggaag caacgtcggc 360
   tctcgtctgt accttctcca ggatgacgaa acctaccaga tcttcaacct gctcaaccgc 420
   gagttcacct tcgatgtcga tgtctccaac cttccttgcg gtctgaacgg tgccctctac 480
   ttcgtcgcca tggatgcgga cggtggtgtc agcaagtacc ccaacaacaa ggccggtgcc 540
   aagtacggca ctggatactg cgactctcag tgcccccgtg acctcaagtt cattgatggc 600
   gagggcaacg tggaaggctg gcagccctcc tccaacaacg ccaacactgg tatcggtgac 660
   cacggatcct gctgcgctga gatggatgtc tgggaggcca actcgatctc caacgccgtc 720
   actcctcacc cttgcgacac ccccggccag accatgtgcg atggagatga ctgcggtgga 780
   acctacagca ccaaccgcta cgctggtgag tgcgaccccg acggctgcga cttcaacccc 840
   taccgcatgg gtaacacctc gttctacggc cccggcaaga tcattgacac cactcagcct 900
   ttcactgttg tcacccagtt cctgaccgat gatggcaccg acaccggcac cctcagcgaa 960
   atccgtcgct tctacatcca gaacggcaac gtcattcctc agcccaactc cgacatctcc 1020
   ggtgtgaccg gcaactccat caccactgag ttctgcaccg ctcagaagca ggctttcggt 1080
   gacaccgatg acttctccca gcacggtggt cttgccggta tgggtgctgc catgcagcag 1140
   ggtatggtcc tcgtcatgtc cctctgggat gactacgctg ctcagatgct ctggttggac 1200
   tccgactacc ccaccaacgc ctctgccacc actcccggtg ttgctcgtgg aacttgcccc 1260
   accgacagcg gtgttccctc ccaggttgag agccagtctc ccaacagcta cgtgacctac 1320
   tccaacatca agttcggtcc catcaactcg accttcactg cctcctaa 1368
<210> 64
   <211> 1368
   <212> DNA
   <213> Artificial sequence
<220>
   <223> Cellobiohydrolase 1 variant
<400> 64
   atgctccgcc gtgccttgct tctgtcttct tctgccatcc tggccgtcaa ggcccagcag        60
   gctggtactg ccactgctga gaaccaccct cctctgacct ggcaggagtg cactgctcct       120
   ggctcttgca ccacccagaa tggtgctgtt gttctcgact cgaactggag atgggttcac       180
   aacgttggtg gatacaccaa ctgctacact ggcaacacct ggaaccccac ctactgcccc       240
   gacgatgtca cctgcgcgga gaactgcgct cttgacggtg ccgactacga gggcacctac       300
   ggtgtcacct cctccggcag cgaactccgt ctgaacttcg tcaccggaag caacgtcggc       360
   tctcgtctgt accttctcca ggatgacgaa acctaccaga tcttcaacct gctcaaccgc       420
   gagttcacct tcgatgtcga tgtctccaac cttccttgcg gtctgaacgg tgccctctac       480
   ttcgtcgcca tggatgcgga cggtggtgtc agccgttacc ccaacaacaa ggccggtgcc       540
   aagtacggca ctggatactg cgactctcag tgcccccgtg acctcaagtt cattgatggc       600
   gagggcaacg tggaaggctg gcagccctcc tccaacaacg ccaacactgg tatcggtgac       660
   cacggatcct gctgcgctga gatggatgtc tgggaggcca actcgatctc caacgccgtc       720
   actcctcacc cttgcgacac ccccggccag accatgtgcg atggagatga ctgcggtgga       780
   acctacagca ccaaccgcta cgctggtgag tgcgaccccg acggctgcga cttcaacccc       840
   taccgcatgg gtaacacctc gttctacggc cccggcaaga tcattgacac cactcagcct       900
   ttcactgttg tcacccagtt cctgaccgat gatggcaccg acaccggcac cctcagcgaa       960
   atccgtcgct tctacatcca gaacggcaac gtcattcctc agcccaactc cgacatctcc      1020
   ggtgtgaccg gcaactccat caccactgag ttctgcaccg ctcagaagca ggctttcggt      1080
   gacaccgatg acttctccca gcacggtggt cttgccggta tgggtgctgc catgcagcag      1140
   ggtatggtcc tcgtcatgtc cctctgggat gactacgctg ctcagatgct ctggttggac      1200
   tccgactacc ccaccaacgc ctctgccacc actcccggtg ttgctcgtgg aacttgcccc      1260
   accgacagcg gtgttccctc ccaggttgag agccagtctc ccaacagcta cgtgacctac      1320
   tccaacatcc gtttcggtcc catcaactcg accttcactg cctcctaa                   1368
<210> 65
   <211> 1368
   <212> DNA
   <213> Artificial sequence
<220>
   <223> Cellobiohydrolase 1 variant
<400> 65
   atgctccgcc gtgccttgct tctgtcttct tctgccatcc tggccgtcaa ggcccagcag      60
   gctggtactg ccactgctga gaaccaccct cctctgacct ggcaggagtg cactgctcct     120
   ggctcttgca ccacccagaa tggtgctgtt gttctcgact cgaactggag atgggttcac     180
   aacgttggtg gatacaccaa ctgctacact ggcaacacct ggaaccccac ctactgcccc     240
   gacgatgtca cctgcgcgga gaactgcgct cttgacggtg ccgactacga gggcacctac     300
   ggtgtcacct cctccggcag cgaactccgt ctgaacttcg tcaccggaag caacgtcggc     360
   tctcgtctgt accttctcca ggatgacgaa acctaccaga tcttcaacct gctcaaccgc     420
   gagttcacct tcgatgtcga tgtctccaac cttccttgcg gtctgaacgg tgccctctac     480
   ttcgtcgcca tggatgcgga cggtggtgtc agcaagtacc ccaacaacaa ggccggtgcc     540
   aagtacggca ctggatactg cgactctcag tgcccccgtg acctcaagtt cattgatggc     600
   gagggcaacg tggaaggctg gcagccctcc tccaacaacg ccaacactgg tatcggtgac     660
   cacggatcct gctgcgctga gatggatgtc tgggaggcca actcgatctc caacgccgtc     720
   actcctcacc cttgcgacac ccccggccag accatgtgcg atggagatga ctgcggtgga     780
   acctacagca ccaaccgcta cgctggtgag tgcgaccccg acggctgcga cttcaacccc     840
   taccgcatgg gtaacacctc gttctacggc cccggcctga tcattgacac cactcagcct     900
   ttcactgttg tcacccagtt cctgaccgat gatggcaccg acaccggcac cctcagcgaa     960
   atccgtcgct tctacatcca gaacggcaac gtcattcctc agcccaactc cgacatctcc    1020
   ggtgtgaccg gcaactccat caccactgag ttctgcaccg ctcagaagca ggctttcggt    1080
   gacaccgatg acttctccca gcacggtggt cttgccggta tgggtgctgc catgcagcag    1140
   ggtatggtcc tcgtcatgtc cctctgggat gactacgctg ctcagatgct ctggttggac    1200
   tccgactacc ccaccaacgc ctctgccacc actcccggtg ttgctcgtgg aacttgcccc    1260
   accgacagcg gtgttccctc ccaggttgag agccagtctc ccaacagcta cgtgacctac    1320
   tccaacatca agttcggtcc catcaactcg accttcactg cctcctaa                 1368
<210> 66
   <211> 1368
   <212> DNA
   <213> Artificial sequence
<220>
   <223> Cellobiohydrolase 1 variant
<400> 66
   atgctccgcc gtgccttgct tctgtcttct tctgccatcc tggccgtcaa ggcccagcag        60
   gctggtactg ccactgctga gaaccaccct cctctgacct ggcaggagtg cactgctcct       120
   ggctcttgca ccacccagaa tggtgctgtt gttctcgact cgaactggag atgggttcac       180
   aacgttggtg gatacaccaa ctgctacact ggcaacacct ggaaccccac ctactgcccc       240
   gacgatgtca cctgcgcgga gaactgcgct cttgacggtg ccgactacga gggcacctac       300
   ggtgtcacct cctccggcag cgaactccgt ctgaacttcg tcaccggaag caacgtcggc       360
   tctcgtctgt accttctcca ggatgacgaa acctaccaga tcttcaacct gctcaaccgc       420
   gagttcacct tcgatgtcga tgtctccaac cttccttgcg gtctgaacgg tgccctctac       480
   ttcgtcgcca tggatgcgga cggtggtgtc agccgttacc ccaacaacac cgccggtgcc       540
   cagtacggca ctggatactg cgactctcag tgcccccgtg acctcaagtt cattgatggc       600
   gagggcaacg tggaaggctg gcagccctcc tccaacaacg ccaacactgg tatcggtgac       660
   cacggatcct gctgcgctga gatggatgtc tgggaggcca actcgatctc caacgccgtc       720
   actcctcacc cttgcgacac ccccggccag accatgtgcg atggagatga ctgcggtgga       780
   acctacagca ccaaccgcta cgctggtgag tgcgaccccg acggctgcga cttcaacccc       840
   taccgcatgg gtaacacctc gttctacggc cccggcaaga tcattgacac cactcagcct       900
   ttcactgttg tcacccagtt cctgaccgat gatggcaccg acaccggcac cctcagcgaa       960
   atccgtcgct tctacatcca gaacggcaac gtcattcctc agcccaactc cgacatctcc      1020
   ggtgtgaccg gcaactccat caccactgag ttctgcaccg ctcagaagca ggctttcggt      1080
   gacaccgatg acttctccca gcacggtggt cttgccggta tgggtgctgc catgcagcag      1140
   ggtatggtcc tcgtcatgtc cctctgggat gactacgctg ctcagatgct ctggttggac      1200
   tccgactacc ccaccaacgc ctctgccacc actcccggtg ttgctcgtgg aacttgcccc      1260
   accgacagcg gtgttccctc ccaggttgag agccagtctc ccaacagcta cgtgacctac      1320
   tccaacatcc gtttcggtcc catcaactcg accttcactg cctcctaa                   1368
<210> 67
   <211> 1368
   <212> DNA
   <213> Artificial sequence
<220>
   <223> Cellobiohydrolase 1 variant
<400> 67
   atgctccgcc gtgccttgct tctgtcttct tctgccatcc tggccgtcaa ggcccagcag 60
   gctggtactg ccactgctga gaaccaccct cctctgacct ggcaggagtg cactgctcct 120
   ggctcttgca ccacccagaa tggtgctgtt gttctcgact cgaactggag atgggttcac 180
   aacgttggtg gatacaccaa ctgctacact ggcaacacct ggaaccccac ctactgcccc 240
   gacgatgtca cctgcgcgga gaactgcgct cttgacggtg ccgactacga gggcacctac 300
   ggtgtcacct cctccggcag cgaactccgt ctgaacttcg tcaccggaag caacgtcggc 360
   tctcgtctgt accttctcca ggatgacgaa acctaccaga tcttcaacct gctcaaccgc 420
   gagttcacct tcgatgtcga tgtctccaac cttccttgcg gtctgaacgg tgccctctac 480
   ttcgtcgcca tggatgcgga cggtggtgtc agccgttacc ccaacaacac cgccggtgcc 540
   cagtacggca ctggatactg cgactctcag tgcccccgtg acctcaagtt cattgatggc 600
   gagggcaacg tggaaggctg gcagccctcc tccaacaacg ccaacactgg tatcggtgac 660
   cacggatcct gctgcgctga gatggatgtc tgggaggcca actcgatctc caacgccgtc 720
   actcctcacc cttgcgacac ccccggccag accatgtgcg atggagatga ctgcggtgga 780
   acctacagca ccaaccgcta cgctggtgag tgcgaccccg acggctgcga cttcaacccc 840
   taccgcatgg gtaacacctc gttctacggc cccggcctga tcattgacac cactcagcct 900
   ttcactgttg tcacccagtt cctgaccgat gatggcaccg acaccggcac cctcagcgaa 960
   atccgtcgct tctacatcca gaacggcaac gtcattcctc agcccaactc cgacatctcc 1020
   ggtgtgaccg gcaactccat caccactgag ttctgcaccg ctcagaagca ggctttcggt 1080
   gacaccgatg acttctccca gcacggtggt cttgccggta tgggtgctgc catgcagcag 1140
   ggtatggtcc tcgtcatgtc cctctgggat gactacgctg ctcagatgct ctggttggac 1200
   tccgactacc ccaccaacgc ctctgccacc actcccggtg ttgctcgtgg aacttgcccc 1260
   accgacagcg gtgttccctc ccaggttgag agccagtctc ccaacagcta cgtgacctac 1320
   tccaacatcc gtttcggtcc catcaactcg accttcactg cctcctaa 1368
<210> 68
   <211> 1368
   <212> DNA
   <213> Artificial sequence
<220>
   <223> Cellobiohydrolase 1 variant
<400> 68
   atgctccgcc gtgccttgct tctgtcttct tctgccatcc tggccgtcaa ggcccagcag 60
   gctggtactg ccactgctga gaaccaccct cctctgacct ggcagaagtg cactgctcct 120
   ggctcttgca ccacccagaa tggtaaggtt gttctcgact cgaactggag atgggttcac 180
   aacgttggtg gatacaccaa ctgctacact ggcaacacct ggaaccccac ctactgcccc 240
   gacgatgtca cctgcgcgga gaactgcgct cttgacggtg ccgactacga gggcacctac 300
   ggtgtcacct cctccggcag cgaactcaag ctgaacttcg tcaccggaag caacgtcggc 360
   tctcgtctgt accttctcca ggatgacgaa acctaccaga tcttcaagct gctcaaccgc 420
   gagttcacct tcgatgtcga tgtctccaac cttccttgcg gtctgaacgg tgccctctac 480
   ttcgtcgcca tggatgcgga cggtggtgtc agcaagtacc ccaacaacaa ggccggtgcc 540
   aagtacggca ctggatactg cgactctcag tgcccccgtg acctcaagtt cattgatggc 600
   gagggcaacg tggaaggctg gcagccctcc tccaacaacg ccaacactgg tatcggtgac 660
   cacggatcct gctgcgctga gatggatgtc tgggaggcca actcgatctc caacgccgtc 720
   actcctcacc cttgcgacac ccccggccag accatgtgcg atggagatga ctgcggtgga 780
   acctacagca ccaaccgcta cgctggtgag tgcgaccccg acggctgcga cttcaacccc 840
   taccgcatgg gtaacacctc gttctacggc cccggcaaga tcattgacac cactaagcct 900
   ttcactgttg tcacccagtt cctgaccgat gatggcaccg acaccggcac cctcagcgaa 960
   atcaagcgct tctacatcca gaacggcaag gtcattcctc agcccaactc cgacatctcc 1020
   ggtgtgaccg gcaactccat caccactgag ttctgcaccg ctcagaagca ggctttcggt 1080
   gacaccgatg acttctccca gcacggtggt cttgccaaga tgggtgctgc catgcagcag 1140
   ggtatggtcc tcgtcatgtc cctctgggat gactacgctg ctcagatgct ctggttggac 1200
   tccgactacc ccaccaacgc ctctgccacc actcccggtg ttgctcgtgg aacttgcccc 1260
   accgacagcg gtgttccctc ccaggttgag agccagtctc ccaacagcta cgtgacctac 1320
   tccaacatca agttcggtcc catcaactcg accttcactg cctcctaa 1368
<210> 69
   <211> 1368
   <212> DNA
   <213> Artificial sequence
<220>
   <223> Cellobiohydrolase 1 variant
<400> 69
   atgctccgcc gtgccttgct tctgtcttct tctgccatcc tggccgtcaa ggcccagcag      60
   gctggtactg ccactgctga gaaccaccct cctctgacct ggcaggagtg cactgctcct     120
   ggctcttgca ccacccagaa tggtaaggtt gttctcgact cgaactggag atgggttcac     180
   aacgttggtg gatacaccaa ctgctacact ggcaacacct ggaaccccac ctactgcccc     240
   gacgatgtca cctgcgcgga gaactgcgct cttgacggtg ccgactacga gggcacctac     300
   ggtgtcacct cctccggcag cgaactcaag ctgaacttcg tcaccggaag caacgtcggc     360
   tctcgtctgt accttctcca ggatgacgaa acctaccaga tcttcaagct gctcaacaag     420
   gagttcacct tcgatgtcga tgtctccaac cttccttgcg gtctgaacgg tgccctctac     480
   ttcgtcgcca tggatgcgga cggtggtgtc agcaagtacc ccaacaacaa ggccggtgcc     540
   aagtacggca ctggatactg cgactctcag tgcccccgtg acctcaagtt cattgatggc     600
   gagggcaacg tggaaggctg gcagccctcc tccaacaacg ccaacactgg tatcggtgac     660
   cacggatcct gctgcgctga gatggatgtc tgggaggcca actcgatctc caacgccgtc     720
   actcctcacc cttgcgacac ccccggccag accatgtgcg atggagatga ctgcggtgga     780
   acctacagca ccaaccgcta cgctggtgag tgcgaccccg acggctgcga cttcaacccc     840
   taccgcatgg gtaacacctc gttctacggc cccggcaaga tcattgacac cactaagcct     900
   ttcactgttg tcacccagtt cctgaccgat gatggcaccg acaccggcac cctcaaggaa     960
   atcaagcgct tctacatcca gaacggcaag gtcattcctc agcccaactc cgacatctcc    1020
   ggtgtgaccg gcaactccat caccactgag ttctgcaccg ctcagaagca ggctttcggt    1080
   gacaccgatg acttctccca gaagggtggt cttgccaaga tgggtgctgc catgcagcag    1140
   ggtatggtcc tcgtcatgtc cctctgggat gactacgctg ctcagatgct ctggttggac    1200
   tccgactacc ccaccaacgc ctctgccacc actcccggtg ttgctcgtgg aacttgcccc    1260
   accgacagcg gtgttccctc ccaggttgag agccagtctc ccaacagcta cgtgacctac    1320
   tccaacatca agttcggtcc catcaactcg accttcactg cctcctaa                  1368
<210> 70
   <211> 1368
   <212> DNA
   <213> Artificial sequence
<220>
   <223> Cellobiohydrolase 1 variant
<400> 70
   atgctccgcc gtgccttgct tctgtcttct tctgccatcc tggccgtcaa ggcccagcag      60
   gctggtactg ccactgctga gaaccaccct cctctgacct ggcagaagtg cactgctcct     120
   ggctcttgca ccacccagaa tggtgctgtt gttctcgact cgaactggag atgggttcac     180
   aacgttggtg gatacaccaa ctgctacact ggcaacacct ggaaccccac ctactgcccc     240
   gacgatgtca cctgcgcgga gaactgcgct cttgacggtg ccgactacga gggcacctac     300
   ggtgtcacct cctccggcag cgaactcaag ctgaacttcg tcaccggaag caacgtcggc     360
   tctcgtctgt accttctcca ggatgacgaa acctaccaga tcttcaagct gctcaaccgc     420
   gagttcacct tcgatgtcga tgtctccaac cttccttgcg gtctgaacgg tgccctctac     480
   ttcgtcgcca tggatgcgga cggtggtgtc agcaagtacc ccaacaacaa ggccggtgcc     540
   aagtacggca ctggatactg cgactctcag tgcccccgtg acctcaagtt cattgatggc     600
   gagggcaacg tggaaggctg gaagccctcc tccaacaacg ccaacactgg tatcggtgac     660
   cacggatcct gctgcgctga gatggatgtc tgggaggcca actcgatctc caacgccgtc     720
   actcctcacc cttgcgacac ccccggccag accatgtgcg atggagatga ctgcggtgga     780
   acctacagca ccaaccgcta cgctggtgag tgcgaccccg acggctgcga cttcaacccc     840
   taccgcatgg gtaacaagtc gttctacggc cccggcaaga tcattgacac cactaagcct     900
   ttcactgttg tcacccagtt cctgaccgat gatggcaccg acaccggcac cctcagcgaa     960
   atcaagcgct tctacatcca gaacggcaag gtcattcctc agcccaactc cgacatctcc    1020
   ggtgtgaccg gcaactccat caccactgag ttctgcaccg ctcagaagca ggctttcggt    1080
   gacaccgatg acttctccca gcacggtggt cttgccaaga tgggtgctgc catgcagcag    1140
   ggtatggtcc tcgtcatgtc cctctgggat gactacgctg ctcagatgct ctggttggac    1200
   tccgactacc ccaccaacgc ctctgccacc actcccggtg ttgctcgtgg aacttgcccc    1260
   accgacagcg gtgttccctc ccaggttgag agccagtctc ccaacagcta cgtgacctac    1320
   tccaacatca agttcggtcc catcaactcg accttcactg cctcctaa                 1368
<210> 71
   <211> 1368
   <212> DNA
   <213> Artificial sequence
<220>
   <223> Cellobiohydrolase 1 variant
<400> 71
   atgctccgcc gtgccttgct tctgtcttct tctgccatcc tggccgtcaa ggcccagcag      60
   gctggtactg ccactgctga gaaccaccct cctctgacct ggcagaagtg cactgctcct     120
   ggctcttgca ccacccagaa tggtaaggtt gttctcgact cgaactggag atgggttcac     180
   aacgttggtg gatacaccaa ctgctacact ggcaacacct ggaaccccac ctactgcccc     240
   gacgataaga cctgcgcgga gaactgcgct cttgacggtg ccgactacga gggcacctac     300
   ggtgtcacct cctccggcag cgaactcaag ctgaacttcg tcaccggaag caacgtcggc     360
   tctcgtctgt accttctcca ggatgacgaa acctaccaga tcttcaagct gctcaaccgc     420
   gagttcacct tcgatgtcga tgtctccaac cttccttgcg gtctgaacgg tgccctctac     480
   ttcgtcgcca tggatgcgga cggtggtgtc agcaagtacc ccaacaacaa ggccggtgcc     540
   aagtacggca ctggatactg cgactctcag tgcccccgtg acctcaagtt cattgatggc     600
   gagggcaacg tggaaggctg gcagccctcc tccaacaacg ccaacactgg tatcggtgac     660
   cacggatcct gctgcgctga gatggatgtc tgggaggcca actcgatctc caacgccgtc     720
   actcctcacc cttgcgacac ccccggccag accatgtgcg atggagatga ctgcggtgga     780
   acctacagca ccaaccgcta cgctggtgag tgcgaccccg acggctgcga cttcaacccc     840
   taccgcatgg gtaacacctc gttctacggc cccggcaaga tcattgacac cactaagcct     900
   ttcactgttg tcacccagtt cctgaccgat gatggcaccg acaccggcac cctcagcgaa     960
   atcaagcgct tctacatcca gaacggcaag gtcattcctc agcccaactc cgacatctcc    1020
   ggtgtgaccg gcaactccat caccactgag ttctgcaccg ctcagaagaa ggctttcggt    1080
   gacaccgatg acttctccca gcacggtggt cttgccaaga tgggtaaggc catgcagcag    1140
   ggtatggtcc tcgtcatgtc cctctgggat gactacgctg ctcagatgct ctggttggac    1200
   tccgactacc ccaccaacgc ctctgccacc actcccggtg ttgctcgtgg aacttgcccc    1260
   accgacagcg gtgttccctc ccaggttgag agccagtctc ccaacagcta cgtgacctac    1320
   tccaacatca agttcggtcc catcaactcg accttcactg cctcctaa                 1368
<210> 72
   <211> 1368
   <212> DNA
   <213> Artificial sequence
<220>
   <223> Cellobiohydrolase 1 variant
<400> 72
   atgctccgcc gtgccttgct tctgtcttct tctgccatcc tggccgtcaa ggcccagcag      60
   gctggtactg ccactgctga gaaccaccct cctctgacct ggcagaagtg cactgctcct     120
   ggctcttgca ccacccagaa tggtaaggtt gttctcgact cgaactggag atgggttcac     180
   aacgttggtg gatacaccaa ctgctacact ggcaacacct ggaaccccac ctactgcccc     240
   gacgatgtca cctgcgcgga gaactgcgct cttgacggtg ccgactacga gggcacctac     300
   ggtgtcacct cctccggcag cgaactcaag ctgaacttcg tcaccggaag caacgtcggc     360
   tctcgtctgt accttctcca ggatgacgaa acctaccaga tcttcaagct gctcaacaag     420
   gagttcacct tcgatgtcga tgtctccaac cttccttgcg gtctgaacgg tgccctctac     480
   ttcgtcgcca tggatgcgga cggtggtgtc agcaagtacc ccaacaacaa ggccggtgcc     540
   aagtacggca ctggatactg cgactctcag tgcccccgtg acctcaagtt cattgatggc     600
   gagggcaacg tggaaggctg gaagccctcc tccaacaacg ccaacactgg tatcggtgac     660
   cacggatcct gctgcgctga gatggatgtc tgggaggcca actcgatctc caacgccgtc     720
   actcctcacc cttgcgacac ccccggccag accatgtgcg atggagatga ctgcggtgga     780
   acctacagca ccaaccgcta cgctggtgag tgcgaccccg acggctgcga cttcaacccc     840
   taccgcatgg gtaacaagtc gttctacggc cccggcaaga tcattgacac cactaagcct     900
   ttcactgttg tcacccagtt cctgaccgat gatggcaccg acaccggcac cctcaaggaa     960
   atcaagcgct tctacatcca gaacggcaag gtcattcctc agcccaactc cgacatctcc    1020
   ggtgtgaccg gcaactccat caccactgag ttctgcaccg ctcagaagca ggctttcggt    1080
   gacaccgatg acttctccca gaagggtggt cttgccaaga tgggtgctgc catgcagcag    1140
   ggtatggtcc tcgtcatgtc cctctgggat gactacgctg ctcagatgct ctggttggac    1200
   tccgactacc ccaccaacgc ctctgccacc actcccggtg ttgctcgtgg aacttgcccc    1260
   accgacagcg gtgttccctc ccaggttgag agccagtctc ccaacagcta cgtgacctac    1320
   tccaacatca agttcggtcc catcaactcg accttcactg cctcctaa                1368
<210> 73
   <211> 1368
   <212> DNA
   <213> Artificial sequence
<220>
   <223> Cellobiohydrolase 1 variant
<400> 73
   atgctccgcc gtgccttgct tctgtcttct tctgccatcc tggccgtcaa ggcccagcag      60
   gctggtactg ccactgctga gaaccaccct cctctgacct ggcaggagtg cactgctcct     120
   ggctcttgca ccacccagaa tggtgctgtt gttctcgact cgaactggag atgggttcac     180
   aacgttggtg gatacaccaa ctgctacact ggcaacacct ggaaccccac ctactgcccc     240
   gacgatgtca cctgcgcgga gaactgcgct cttgacggtg ccgactacga gggcacctac     300
   ggtgtcacct cctccggcag cgaactccgt ctgaacttcg tcaccggaag caacgtcggc     360
   tctcgtctgt accttctcca ggatgacgaa acctaccaga tcttcaacct gctcaaccgc     420
   gagttcacct tcgatgtcga tgtctccaac cttccttgcg gtctgaacgg tgccctctac     480
   ttcgtcgcca tggatgcgga cggtggtgtc agcaagtacc ccaacaacaa ggccggtgcc     540
   aagtacggca ctggatactg cgactctcag tgcccccgtg acctcaagtt cattgatggc     600
   gagggcaacg tggaaggctg gcagccctcc tccaacaacg ccaacactgg tatcggtgac     660
   cacggatcct gctgcgctga gatggatgtc tgggaggcca actcgatctc caacgccgtc     720
   actcctcacc cttgcgacac ccccggccag acccgctgcg atggagatga ctgcggtgga     780
   acctacagca ccaaccgcta cgctggtgag tgcgaccccg acggctgcga cttcaacccc     840
   taccgcctcg gtaacacctc gttctacggc cccggcaaga tcattgacac cactcagcct     900
   ttcactgttg tcacccagtt cctgaccgat gatggcaccg acaccggcac cctcagcgaa     960
   atccgtcgct tctacatcca gaacggcaac gtcattcctc agcccaactc cgacatctcc    1020
   ggtgtgaccg gcaactccat caccactgag ttctgcaccg ctcagaagca ggctttcggt    1080
   gacaccgatg acttctccca gcacggtggt cttgccggta tgggtgctgc catgcagcag    1140
   ggtatggtcc tcgtcctctc cctctgggat gactacgctg ctcagatgct ctggttggac    1200
   tccgactacc ccaccaacgc ctctgccacc actcccggtg ttgctcgtgg aacttgcccc    1260
   accgacagcg gtgttccctc ccaggttgag agccagtctc ccaacagcta cgtgacctac    1320
   tccaacatca agttcggtcc catcaactcg accttcactg cctcctaa                  1368
<210> 74
   <211> 1368
   <212> DNA
   <213> Artificial sequence
<220>
   <223> Cellobiohydrolase 1 variant
<400> 74
   atgctccgcc gtgccttgct tctgtcttct tctgccatcc tggccgtcaa ggcccagcag       60
   gctggtactg ccactgctga gaaccaccct cctctgacct ggcaggagtg cactgctcct      120
   ggctcttgca ccacccagaa tggtgctgtt gttctcgact cgaactggag atgggttcac      180
   aacgttggtg gatacaccaa ctgctacact ggcaacacct ggaaccccac ctactgcccc      240
   gacgatgtca cctgcgcgga gaactgcgct cttgacggtg ccgactacga gggcacctac      300
   ggtgtcacct cctccggcag cgaactccgt ctgaacttcg tcaccggaag caacgtcggc      360
   tctcgtctgt accttctcca ggatgacgaa acctaccaga tcttcaacct gctcaaccgc      420
   gagttcacct tcgatgtcga tgtctccaac cttccttgcg gtctgaacgg tgccctctac      480
   ttcgtcgcca tggatgcgga cggtggtgtc agccgttacc ccaacaacaa ggccggtgcc      540
   aagtacggca ctggatactg cgactctcag tgcccccgtg acctcaagtt cattgatggc      600
   gagggcaacg tggaaggctg gcagccctcc tccaacaacg ccaacactgg tatcggtgac      660
   cacggatcct gctgcgctga gatggatgtc tgggaggcca actcgatctc caacgccgtc      720
   actcctcacc cttgcgacac ccccggccag acccgctgcg atggagatga ctgcggtgga      780
   acctacagca ccaaccgcta cgctggtgag tgcgaccccg acggctgcga cttcaacccc      840
   taccgcctcg gtaacacctc gttctacggc cccggcctga tcattgacac cactcagcct      900
   ttcactgttg tcacccagtt cctgaccgat gatggcaccg acaccggcac cctcagcgaa      960
   atccgtcgct tctacatcca gaacggcaac gtcattcctc agcccaactc cgacatctcc     1020
   ggtgtgaccg gcaactccat caccactgag ttctgcaccg ctcagaagca ggctttcggt     1080
   gacaccgatg acttctccca gcacggtggt cttgccggtg tcggtgctgc cctccagcag     1140
   ggtatggtcc tcgtcctctc cctctgggat gactacgctg ctcagatgct ctggttggac     1200
   tccgactacc ccaccaacgc ctctgccacc actcccggtg ttgctcgtgg aacttgcccc     1260
   accgacagcg gtgttccctc ccaggttgag agccagtctc ccaacagcta cgtgacctac     1320
   tccaacatcc gtttcggtcc catcaactcg accttcactg cctcctaa                 1368
<210> 75
   <211> 1485
   <212> **DNA**
   <213> **Artificial** sequence
<220>
   <223> Fungal amylase variant
<400> 75
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatgtac ctgatggttg atgttgttgc caaccacatg     420
   ggatacgatg gagcgggcag cagcgttgac tactccgtct tcaagccctt ctcctcccag     480
   gactacttcc accccttctg cttcatccag aattacgagg accagaccca ggttgaggac     540
   tgctggttgg gtgacaacac tgtctctctg cccgatctcg acaccaccaa ggatgttgtc     600
   aagaacgaat ggtacgactg ggtgggctct cttgtctcca actactccat cgatggcctc     660
   cgtatcgaca ctgtcaagca cgtccagaag gacttctggc ctggatacaa caaggctgct     720
   ggtgtctact gcattggtga ggtcctcgat ggtgaccctg cctacacctg cccctaccag     780
   aacgtcctgg atggtgtcct caactaccct atctactacc ctctgctcaa cgccttcaag     840
   tccacctccg gcagcatgga tgacctgtac aacatgatca acaccgtcaa gtccgactgc     900
   cccgactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaacg ccatccgcaa ctacgccatc tccaaggaca ccggtttcgt cacctacaag    1200
   aactggccca tctacaagga tgataccacc attgccctcc gcaagggcac cgatggctcc    1260
   cagatcgtca ccattttgtc gaacaagggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccctcgccg gtggtcttcc tcgtgtcctc    1440
   taccccactg agaagctggc tggaagcaag atctgctcga gcagc                     1485
<210> 76
   <211> 1485
   <212> DNA
   <213> Artificial sequence
<220>
   <223> Fungal amylase variant
<400> 76
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatgtac ctgatggttg atgttgttgc caaccacatg     420
   ggatacgatg gagcgggcag cagcgttgac tactccgtct tcaagccctt ctcctcccag     480
   gactacttcc accccttctg cttcatccag aattacgagg accagaccca ggttgaggac     540
   tgctggttgg gtgacaacac tgtctctctg cccgatctcg acaccaccaa ggatgttgtc     600
   aagaacgaat ggtacgactg ggtgggctct cttgtctcca actactccat cgatggcctc     660
   cgtatcgaca ctgtcaagca cgtccagaag gacttctggc ctggatacaa caaggctgct     720
   ggtgtctact gcattggtga ggtcctcgat ggtgaccctg cctacacctg cccctaccag     780
   aacgtcctgg atggtgtcct caactaccct atctactacc ctctgctcaa cgccttcaag     840
   tccacctccg gcagcatcga tgacctgtac aacatgatca acaccgtcaa gtccgactgc     900
   cccgactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaacg ccatccgcaa ctacgccatc tccaaggaca ccggtttcgt cacctacaag    1200
   aactggccca tctacaagga tgataccacc attgccatgc gcaagggcac cgatggctcc    1260
   cagatcgtca ccattttgtc gaacaagggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccctcgccg gtggtcttcc tcgtgtcctc    1440
   taccccactg agaagctggc tggaagcaag atctgctcga gcagc                    1485
<210> 77
   <211> 1485
   <212> DNA
   <213> Artificial sequence
<220>
   <223> Fungal amylase variant
<400> 77
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatgtac ctgatggttg atgttgttgc caaccacatg     420
   ggatacgatg gagcgggcag cagcgttgac tactccgtct tcaagccctt ctcctcccag     480
   gactacttcc accccttctg cttcatccag aattacgagg accagaccca ggttgaggac     540
   tgctggttgg gtgacaacac tgtctctctg cccgatctcg acaccaccaa ggatgttgtc     600
   aagaacgaat ggtacgactg ggtgggctct cttgtctcca actactccat cgatggcctc     660
   cgtatcgaca ctgtcaagca cgtccagaag gacttctggc ctggatacaa caaggctgct     720
   ggtgtctact gcattggtga ggtcctcgat ggtgaccctg cctacacctg cccctaccag     780
   aacgtcctgg atggtgtcct caactaccct atctactacc ctctgctcaa cgccttcaag     840
   tccacctccg gcagcatgga tgacctgtac aacgagatca acaccgtcaa gtccgactgc     900
   cccgactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaacg ccatccgcaa ctacgccatc tccaaggaca ccggtttcgt cacctacaag    1200
   aactggccca tctacaagga tgataccacc attgccctcc gcaagggcac cgatggctcc    1260
   cagatcgtca ccattttgtc gaacaagggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccatggccg gtggtcttcc tcgtgtcctc    1440
   taccccactg agaagctggc tggaagcaag atctgctcga gcagc                    1485
<210> 78
   <211> 1485
   <212> DNA
   <213> Artificial sequence
<220>
   <223> Fungal amylase variant
<400> 78
   atggtcaagt ccatcctggc ctccgtcttc ttcgctgcca ctgctcttgc tgccaccccc 60
   gccgactggc gctcccagtc catctacttc ctgctcaccg accgtttcgc tcgcaccgac 120
   ggctccacta ctgccacctg caacactgcg gaccagaagt actgcggtgg cacctggcag 180
   ggtatcattg acaagctcga ctacatccag ggtatgggtt tcactgccat ctggatcacc 240
   cccgtcactg ctcagctccc ccagaccact gcctacggtg atgcctacca cggatactgg 300
   cagcaggata tctactctct gaacgagaac tacggtactg ccgatgacct caaggccctt 360
   tcttctgctc tgcacgagcg tggcatgtac ctgatggttg atgttgttgc caaccacatg 420
   ggatacgatg gagcgggcag cagcgttgac tactccgtct tcaagccctt ctcctcccag 480
   gactacttcc accccttctg cttcatccag aattacgagg accagaccca ggttgaggac 540
   tgctggttgg gtgacaacac tgtctctctg cccgatctcg acaccaccaa ggatgttgtc 600
   aagaacgaat ggtacgactg ggtgggctct cttgtctcca actactccat cgatggcctc 660
   cgtatcgaca ctgtcaagca cgtccagaag gacttctggc ctggatacaa caaggctgct 720
   ggtgtctact gcattggtga ggtcctcgat ggtgaccctg cctacacctg cccctaccag 780
   aacgtcctgg atggtgtcct caactaccct atctactacc ctctgctcaa cgccttcaag 840
   tccacctccg gcagcatcga tgacctgtac aacgagatca acaccgtcaa gtccgactgc 900
   cccgactcga ccctccttgg caccttcgtc gagaaccacg acaaccctcg cttcgcctcc 960
   tacaccaacg acattgccct tgccaagaac gtcgctgctt tcatcatcct caacgacggt 1020
   atccccatca tctatgctgg ccaggagcag cactacgctg gtggcaacga ccccgccaac 1080
   cgtgaggcca cctggctctc cggctaccct accgacagcg aactctacaa gttgattgcc 1140
   tccgccaacg ccatccgcaa ctacgccatc tccaaggaca ccggtttcgt cacctacaag 1200
   aactggccca tctacaagga tgataccacc attgccctcc gcaagggcac cgatggctcc 1260
   cagatcgtca ccattttgtc gaacaagggt gcttctggtg acagctacac tctgtctctt 1320
   tctggtgctg gctacactgc cggtcagcag ctgaccgaag tcattggatg caccaccgtc 1380
   accgttggct ccgatggcaa cgtccccgtg cccctcgccg gtggtcttcc tcgtgtcctc 1440
   taccccactg agaagctggc tggaagcaag atctgctcga gcagc 1485
<210> 79
   <211> 1485
   <212> DNA
   <213> Artificial sequence
<220>
   <223> Fungal amylase variant
<400> 79
   atggtcaagt ccatcctggc ctccgtcttc ttcgctgcca ctgctcttgc tgccaccccc 60
   gccgactggc gctcccagtc catctacttc ctgctcaccg accgtttcgc tcgcaccgac 120
   ggctccacta ctgccacctg caacactgcg gaccagaagt actgcggtgg cacctggcag 180
   ggtatcattg acaagctcga ctacatccag ggtatgggtt tcactgccat ctggatcacc 240
   cccgtcactg ctcagctccc ccagaccact gcctacggtg atgcctacca cggatactgg 300
   cagcaggata tctactctct gaacgagaac tacggtactg ccgatgacct caaggccctt 360
   tcttctgctc tgcacgagcg tggcatgtac ctgatggttg atgttgttgc caaccacttt 420
   ggatacgatg gagcgggcag cagcgttgac tactccgtct tcaagccctt ctcctcccag 480
   gactacttcc accccttctg cttcatccag aattacgagg accagaccca ggttgaggac 540
   tgctggttgg gtgacaacac tgtctctctg cccgatctcg acaccaccaa ggatgttgtc 600
   aagaacgaat ggtacgactg ggtgggctct cttgtctcca actactccat cgatggcctc 660
   cgtatcgaca ctgtcaagca cgtccagaag gacttctggc ctggatacaa caaggctgct 720
   ggtgtctact gcattggtga ggtcctcgat ggtgaccctg cctacacctg cccctaccag 780
   aacgtcatgg atggtgtcct caactaccct atctactacc ctctgctcaa cgccttcaag 840
   tccacctccg gcagcatcga tgacctgtac aacgagatca acaccgtcaa gtccgactgc 900
   cccgactcga ccctccttgg caccttcgtc gagaaccacg acaaccctcg cttcgcctcc 960
   tacaccaacg acattgccct tgccaagaac gtcgctgctt tcatcatcct caacgacggt 1020
   atccccatca tctatgctgg ccaggagcag cactacgctg gtggcaacga ccccgccaac 1080
   cgtgaggcca cctggctctc cggctaccct accgacagcg aactctacaa gttgattgcc 1140
   tccgccaacg ccatccgcaa ctacgccatc tccaaggaca ccggtttcgt cacctacaag 1200
   aactggccca tctacaagga tgataccacc attgccctcc gcaagggcac cgatggctcc 1260
   cagatcgtca ccattttgtc gaacaagggt gcttctggtg acagctacac tctgtctctt 1320
   tctggtgctg gctacactgc cggtcagcag ctgaccgaag tcattggatg caccaccgtc 1380
   accgttggct ccgatggcaa cgtccccgtg cccctcgccg gtggtcttcc tcgtgtcctc 1440
   taccccactg agaagctggc tggaagcaag atctgctcga gcagc 1485
<210> 80
   <211> 1485
   <212> DNA
   <213> Artificial sequence
<220>
   <223> Fungal amylase variant
<400> 80
   atggtcaagt ccatcctggc ctccgtcttc ttcgctgcca ctgctcttgc tgccaccccc        60
   gccgactggc gctcccagtc catctacttc ctgctcaccg accgtttcgc tcgcaccgac       120
   ggctccacta ctgccacctg caacactgcg gaccagaagt actgcggtgg cacctggcag       180
   ggtatcattg acaagctcga ctacatccag ggtcttggtt tcactgccat ctggatcacc       240
   cccgtcactg ctcagctccc ccagaccact gcctacggtg atgcctacca cggatactgg       300
   cagcaggata tctactctct gaacgagaac tacggtactg ccgatgacct caaggccctt       360
   tcttctgctc tgcacgagcg tggcatgtac ctgatggttg atgttgttgc caaccacttt       420
   ggatacgatg gagcgggcag cagcgttgac tactccgtct tcaagccctt ctcctcccag       480
   gactacttcc accccttctg cttcatccag aattacgagg accagaccca ggttgaggac       540
   tgctggttgg gtgacaacac tgtctctctg cccgatctcg acaccaccaa ggatgttgtc       600
   aagaacgaat ggtacgactg ggtgggctct cttgtctcca actactccat cgatggcctc       660
   cgtatcgaca ctgtcaagca cgtccagaag gacttctggc ctggatacaa caaggctgct       720
   ggtgtctact gcattggtga ggtcctcgat ggtgaccctg cctacacctg cccctaccag       780
   aacgtcctgg atggtgtcct caactaccct atctactacc ctctgctcaa cgccttcaag       840
   tccacctccg gcagcatgga tgacctgtac aacatgatca acaccgtcaa gtccgactgc       900
   cccgactcga ccctccttgg caccttcgtc gagaaccacg acaaccctcg cttcgcctcc       960
   tacaccaacg acattgccct tgccaagaac gtcgctgctt tcatcatcct caacgacggt      1020
   atccccatca tctatgctgg ccaggagcag cactacgctg gtggcaacga ccccgccaac      1080
   cgtgaggcca cctggctctc cggctaccct accgacagcg aactctacaa gttgattgcc      1140
   tccgccaacg ccatccgcaa ctacgccatc tccaaggaca ccggtttcgt cacctacaag      1200
   aactggccca tctacaagga tgataccacc attgccctcc gcaagggcac cgatggctcc      1260
   cagatcgtca ccattttgtc gaacaagggt gcttctggtg acagctacac tctgtctctt      1320
   tctggtgctg gctacactgc cggtcagcag ctgaccgaag tcattggatg caccaccgtc      1380
   accgttggct ccgatggcaa cgtccccgtg cccctcgccg gtggtcttcc tcgtgtcctc      1440
   taccccactg agaagctggc tggaagcaag atctgctcga gcagc                      1485
<210> 81
   <211> 1485
   <212> DNA
   <213> Artificial sequence
<220>
   <223> Fungal amylase variant
<400> 81
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtcttggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatgtac ctgatggttg atgttgttgc caaccacttt     420
   ggatacgatg gagcgggcag cagcgttgac tactccgtct tcaagccctt ctcctcccag     480
   gactacttcc accccttctg cttcatccag aattacgagg accagaccca ggttgaggac     540
   tgctggttgg gtgacaacac tgtctctctg cccgatctcg acaccaccaa ggatgttgtc     600
   aagaacgaat ggtacgactg ggtgggctct cttgtctcca actactccat cgatggcctc     660
   cgtatcgaca ctgtcaagca cgtccagaag gacttctggc ctggatacaa caaggctgct     720
   ggtgtctact gcattggtga ggtcctcgat ggtgaccctg cctacacctg cccctaccag     780
   aacgtcctgg atggtgtcct caactaccct atctactacc ctctgctcaa cgccttcaag     840
   tccacctccg gcagcatcga tgacctgtac aacgagatca acaccgtcaa gtccgactgc     900
   cccgactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaacg ccatccgcaa ctacgccatc tccaaggaca ccggtttcgt cacctacaag    1200
   aactggccca tctacaagga tgataccacc attgccatgc gcaagggcac cgatggctcc    1260
   cagatcgtca ccattttgtc gaacaagggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccatggccg gtggtcttcc tcgtgtcctc    1440
   taccccactg agaagctggc tggaagcaag atctgctcga gcagc                    1485
<210> 82
   <211> 1485
   <212> DNA
   <213> Artificial sequence
<220>
   <223> Fungal amylase variant
<400> 82
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtcttggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatctac ctgcttgttg atgttgttgc caaccacttt     420
   ggatacgatg gagcgggcag cagcgttgac tactccgtct tcaagccctt ctcctcccag     480
   gactacttcc accccttctg cttcatccag aattacgagg accagaccca ggttgaggac     540
   tgctggttgg gtgacaacac tgtctctctg cccgatctcg acaccaccaa ggatgttgtc     600
   aagaacgaat ggtacgactg ggtgggctct cttgtctcca actactccat cgatggcctc     660
   cgtatcgaca ctgtcaagca cgtccagaag gacttctggc ctggatacaa caaggctgct     720
   ggtgtctact gcattggtga ggtcctcgat ggtgaccctg cctacacctg cccctaccag     780
   aacgtcctgg atggtgtcct caactaccct atctactacc ctctgctcaa cgccttcaag     840
   tccacctccg gcagcatcga tgacctgtac aacgagatca acaccgtcaa gtccgactgc     900
   cccgactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaacg ccatccgcaa ctacgccatc tccaaggaca ccggtttcgt cacctacaag    1200
   aactggccca tctacaagga tgataccacc attgccctcc gcaagggcac cgatggctcc    1260
   cagatcgtca ccattttgtc gaacaagggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccctcgccg gtggtcttcc tcgtgtcctc    1440
   taccccactg agaagctggc tggaagcaag atctgctcga gcagc                    1485
<210> 83
   <211> 1485
   <212> DNA
   <213> Artificial sequence
<220>
   <223> Fungal amylase variant
<400> 83
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatgtac ctgatggttg atgttgttgc caaccacatg     420
   ggatacgatg gagcgggcag cagcgttgac tactccgtct tcaagccctt ctcctcccag     480
   gactacttcc accccttctg cttcatccag aattacgagg accagaccca ggttgaggac      540
   tgctggttgg gtgacaacac tgtctctctg cccgatctcg acaccaccaa ggatgttgtc     600
   aagaacgaat ggtacgactg ggtgggctct cttgtctcca actactccat cgatggcctc     660
   cgtatcgaca ctgtcaagca cgtccagaag gacttctggc ctggatacaa caaggctgct     720
   ggtgtctact gcattggtga ggtcctcgat ggtgaccctg cctacacctg cccctaccag     780
   aacgtcatgg atggtgtcct caactaccct atgtactacc ctctgctcaa cgccttcaag     840
   tccacctccg gcagcatgga tgacctgtac aacatgatca acaccatgaa gtccgactgc     900
   cccgactcga ccctccttgg caccttcgtc gagaaccacg acaaccctcg cttcgcctcc     960
   tacaccaacg acatggccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaacg ccatccgcaa ctacgccatc tccaaggaca ccggtttcgt cacctacaag    1200
   aactggccca tctacaagga tgataccacc attgccatgc gcaagggcac cgatggctcc    1260
   cagatcgtca ccattttgtc gaacaagggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccatggccg gtggtcttcc tcgtgtcctc    1440
   taccccactg agaagctggc tggaagcaag atctgctcga gcagc                   1485
<210> 84
   <211> 1485
   <212> DNA
   <213> Artificial sequence
<220>
   <223> Fungal amylase variant
<400> 84
   atggtcaagt ccatcctggc ctccgtcttc ttcgctgcca ctgctcttgc tgccaccccc 60
   gccgactggc gctcccagtc catctacttc ctgctcaccg accgtttcgc tcgcaccgac 120
   ggctccacta ctgccacctg caacactgcg gaccagaagt actgcggtgg cacctggcag 180
   ggtatcattg acaagctcga ctacatccag ggtatgggtt tcactgccat ctggatcacc 240
   cccgtcactg ctcagctccc ccagaccact gcctacggtg atgcctacca cggatactgg 300
   cagcaggata tctactctct gaacgagaac tacggtactg ccgatgacct caaggccctt 360
   tcttctgctc tgcacgagcg tggcatgtac ctgatggttg atgttgttgc caaccacatg 420
   ggatacgatg gagcgggcag cagcgttgac tactccgtct tcaagccctt ctcctcccag 480
   gactacttcc accccttctg cttcatccag aattacgagg accagaccca ggttgaggac 540
   tgctggttgg gtgacaacac tgtctctctg cccgatctcg acaccaccaa ggatgttgtc 600
   aagaacatgt ggtacgactg ggtgggctct cttgtctcca actactccat cgatggcctc 660
   cgtatcgaca ctgtcaagca cgtccagaag gacttctggc ctggatacaa caaggctgct 720
   ggtgtctact gcattggtga ggtcctcgat ggtgaccctg cctacacctg cccctaccag 780
   aacgtcatgg atggtgtcct caactaccct atctactacc ctctgctcaa cgccttcaag 840
   tccacctccg gcagcatgga tgacctgtac aacatgatca acaccgtcaa gtccgactgc 900
   cccgactcga ccctccttgg caccttcatg gagaaccacg acaaccctcg cttcgcctcc 960
   tacaccaacg acattgccct tgccaagaac gtcgctgctt tcatcatcct caacgacggt 1020
   atccccatca tctatgctgg ccaggagcag cactacgctg gtggcaacga ccccgccaac 1080
   cgtgaggcca cctggctctc cggctaccct accgacagcg aactctacaa gttgattgcc 1140
   tccgccaacg ccatccgcaa ctacgccatc tccaaggaca ccggtttcgt cacctacaag 1200
   aactggccca tctacaagga tgataccacc attgccatgc gcaagggcac cgatggctcc 1260
   cagatcgtca ccattttgtc gaacaagggt gcttctggtg acagctacac tctgtctctt 1320
   tctggtgctg gctacactgc cggtcagcag ctgatggaag tcattggatg caccaccgtc 1380
   accgttggct ccgatggcaa cgtccccgtg cccatggccg gtggtcttcc tcgtgtcctc 1440
   taccccactg agaagctggc tggaagcaag atctgctcga gcagc 1485
<210> 85
   <211> 1485
   <212> DNA
   <213> Artificial sequence
<220>
   <223> Fungal amylase variant
<400> 85
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatgtac ctgatggttg atgttgttgc caaccacatg     420
   ggatacgatg gagcgggcag cagcgttgac tactccgtct tcaagccctt ctcctcccag     480
   gactacttcc accccttctg cttcatccag aattacgagg accagaccca ggttgaggac     540
   tgctggttgg gtgacaacac tgtctctctg cccgatctcg acaccaccaa ggatgttgtc     600
   aagaacatgt ggtacgactg ggtgggctct cttgtctcca actactccat cgatggcctc     660
   cgtatcgaca ctgtcaagca cgtccagaag gacttctggc ctggatacaa caaggctgct     720
   ggtgtctact gcattggtga ggtcctcgat ggtgaccctg cctacacctg cccctaccag     780
   aacgtcatgg atggtgtcct caactaccct atgtactacc ctctgctcaa cgccttcaag     840
   tccacctccg gcagcatgga tgacctgtac aacatgatca acaccatgaa gtccgactgc     900
   cccgactcga ccctccttgg caccttcatg gagaaccacg acaaccctcg cttcgcctcc     960
   tacaccaacg acatggccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaacg ccatccgcaa ctacgccatc tccaaggaca ccggtttcgt cacctacaag    1200
   aactggccca tctacaagga tgataccacc attgccatgc gcaagggcac cgatggctcc    1260
   cagatcgtca ccattttgtc gaacaagggt gcttctggtg acagctacac tctgtctctt    1320
   tctggtgctg gctacactgc cggtcagcag ctgatggaag tcattggatg caccaccgtc    1380
   accgttggct ccgatggcaa cgtccccgtg cccatggccg gtggtcttcc tcgtgtcctc    1440
   taccccactg agaagctggc tggaagcaag atctgctcga gcagc                    1485
<210> 86
   <211> 1485
   <212> DNA
   <213> Artificial sequence
<220>
   <223> Fungal amylase variant
<400> 86
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatgtac ctgatggttg atgttgttgc caaccacatg     420
   ggatacgatg gagcgggcag cagcgttgac tactccgtct tcaagccctt ctcctcccag     480
   gactacttcc accccttctg cttcatccag aattacgagg accagaccat ggttgaggac     540
   tgctggttgg gtgacaacac tgtctctctg cccgatctcg acaccaccaa ggatgttgtc     600
   aagaacatgt ggtacgactg ggtgggctct atggtgtcca actactccat cgatggcctc     660
   cgtatcgaca ctgtcaagca cgtccagaag gacttctggc ctggatacaa caaggctgct     720
   ggtgtctaca tgattggtga ggtcctcgat ggtgaccctg cctacacctg cccctaccag     780
   aacgtcatgg atggtgtcct caactaccct atgtactacc ctctgctcaa cgccttcaag     840
   tccacctccg gcagcatgga tgacctgtac aacatgatca acaccatgaa gtccgactgc     900
   cccgactcga ccctccttgg caccttcatg gagaaccacg acaaccctcg cttcgcctcc     960
   tacaccaacg acatggccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaacg ccatccgcaa ctacgccatc tccaaggaca ccggtttcgt cacctacaag    1200
   aactggccca tctacaagga tgataccacc attgccatgc gcaagggcac cgatggctcc    1260
   cagatcgtca ccattttgtc gaacaagggt gcttctggtg acagctacac tctgtctctt    1320
   tctggtgctg gctacactgc cggtcagcag ctgatggaag tcattggatg caccaccgtc    1380
   accgttggct ccgatggcaa cgtccccgtg cccatggccg gtggtcttcc tcgtgtcctc    1440
   taccccactg agaagctggc tggaagcaag atctgctcga gcagc                    1485
<210> 87
   <211> 1485
   <212> DNA
   <213> Artificial sequence
<220>
   <223> Fungal amylase variant
<400> 87
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtatgggtt tcactgccat ctggatcacc     240
   cccgtcacta agcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatgtac ctgatggttg atgttgttgc caaccacatg     420
   ggatacgatg gagcgggcag cagcgttgac tactccgtct tcaagccctt ctcctcccag     480
   gactacttcc accccttctg cttcatccag aattacgagg accagaccca ggttgaggac     540
   tgctggttgg gtgacaacac tgtctctctg cccgatctcg acaccaccaa ggatgttgtc     600
   aagaacgaat ggtacgactg ggtgaagtct cttgtctcca actactccat cgatggcctc     660
   cgtatcgaca ctgtcaagca cgtccagaag gacttctggc ctggatacaa caaggctgct     720
   ggtgtctact gcattggtga ggtcctcgat ggtgaccctg cctacacctg cccctaccag     780
   aacgtcatgg atggtgtcct caactaccct atctactacc ctctgctcaa cgccttcaag     840
   tccacctccg gcagcatgga tgacctgtac aacatgatca acaccgtcaa gtccgactgc     900
   aaggactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaaca agatccgcaa ctacgccatc tccaaggaca ccggtttcgt cacctacaag    1200
   aactggccca tctacaagga tgataccacc attgccatgc gcaagggcac cgatggctcc    1260
   cagatcgtca ccattttgtc gaacaagggt gcttctggtg acagctacac tctgtctctt    1320
   tctggtgctg gctacactgc cggtcagaag ctgaccgaag tcattggatg caccaccgtc    1380
   accgttggct ccgatggcaa cgtccccgtg cccatggccg gtggtcttcc tcgtgtcctc    1440
   taccccactg agaagctggc tggaagcaag atctgctcga gcagc                    1485
<210> 88
   <211> 1485
   <212> DNA
   <213> Artificial sequence
<220>
   <223> Fungal amylase variant
<400> 88
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtatgggtt tcactgccat ctggatcacc     240
   cccgtcacta agcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatgtac ctgatggttg atgttgttgc caaccacatg     420
   ggatacgatg gagcgggcag cagcgttgac tactccgtct tcaagccctt ctcctcccag       480
   gactacttcc accccttctg cttcatccag aattacgagg accagaccca ggttgaggac       540
   tgctggttgg gtgacaacac tgtctctctg cccgatctcg acaccaccaa ggatgttgtc     600
   aagaacgaat ggtacgactg ggtgaagtct cttgtctcca actactccat cgatggcctc     660
   cgtatcgaca ctgtcaagca cgtccagaag gacttctggc ctggatacaa caaggctgct     720
   ggtgtctact gcattggtga ggtcctcgat ggtgaccctg cctacacctg cccctaccag     780
   aacgtcatgg atggtgtcct caactaccct atctactacc ctctgctcaa cgccttcaag     840
   tccacctccg gcagcatgga tgacctgtac aacatgatca acaccgtcaa gtccgactgc     900
   aaggactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaaca agatccgcaa ctacgccatc tccaaggaca ccggtttcgt cacctacaag    1200
   aactggccca tctacaagga tgataccacc attgccatga agaagggcac cgatggctcc    1260
   cagatcgtca ccattttgtc gaacaagggt gcttctggtg acagctacac tctgtctctt    1320
   tctggtgctg gctacactgc cggtcagaag ctgaccgaag tcattggatg caccaccgtc    1380
   accgttggct ccgatggcaa cgtccccgtg cccatggccg gtggtcttcc taaggtcctc    1440
   taccccactg agaagctggc tggaagcaag atctgctcga gcagc                    1485
<210> 89
   <211> 1485
   <212> DNA
   <213> Artificial sequence
<220>
   <223> Fungal amylase variant
<400> 89
   atggtcaagt ccatcctggc ctccgtcttc ttcgctgcca ctgctcttgc tgccaccccc 60
   gccgactgga agtcccagtc catctacttc ctgctcaccg accgtttcgc tcgcaccgac 120
   ggctccacta ctgccacctg caacactgcg gaccagaagt actgcggtgg cacctggaag 180
   ggtatcattg acaagctcga ctacatccag ggtatgggtt tcactgccat ctggatcacc 240
   cccgtcacta agcagctccc ccagaccact gcctacggtg atgcctacca cggatactgg 300
   cagcaggata tctactctct gaacgagaac tacggtactg ccgatgacct caaggccctt 360
   tcttctgctc tgcacgagcg tggcatgtac ctgatggttg atgttgttgc caaccacatg 420
   ggatacgatg gagcgggcag cagcgttgac tactccgtct tcaagccctt ctcctcccag 480
   gactacttcc accccttctg cttcatccag aattacgagg accagaccca ggttgaggac 540
   tgctggttgg gtgacaacac tgtctctctg cccgatctcg acaccaccaa ggatgttgtc 600
   aagaacgaat ggtacgactg ggtgaagtct cttgtctcca actactccat cgatggcctc 660
   cgtatcgaca ctgtcaagca cgtccagaag gacttctggc ctggatacaa caaggctgct 720
   ggtgtctact gcattggtga ggtcctcgat ggtgaccctg cctacacctg cccctaccag 780
   aacgtcatgg atggtgtcct caactaccct atctactacc ctctgctcaa cgccttcaag 840
   tccacctccg gcagcatgga tgacctgtac aacatgatca acaccgtcaa gtccgactgc 900
   aaggactcga ccctccttgg caccttcgtc gagaaccacg acaaccctcg cttcgcctcc 960
   tacaccaacg acattgccct tgccaagaac gtcgctgctt tcatcatcct caacgacggt 1020
   atccccatca tctatgctgg ccaggagcag cactacgctg gtggcaacga ccccgccaac 1080
   cgtgaggcca cctggctctc cggctaccct accgacagcg aactctacaa gttgattgcc 1140
   aaggccaaca agatccgcaa gtacgccatc tccaaggaca ccggtttcgt cacctacaag 1200
   aactggccca tctacaagga tgataccacc attgccatga agaagggcac cgatggctcc 1260
   cagatcgtca ccattttgtc gaacaagggt gcttctggtg acagctacac tctgtctctt 1320
   tctggtgctg gctacactgc cggtcagaag ctgaccgaag tcattggatg caccaccgtc 1380
   accgttggct ccgatggcaa ggtccccgtg cccatggccg gtggtcttcc taaggtcctc 1440
   taccccactg agaagctggc tggaagcaag atctgctcga gcagc 1485
<210> 90
   <211> 1485
   <212> DNA
   <213> Artificial sequence
<220>
   <223> Fungal amylase variant
<400> 90
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatgtac ctgatggttg atgttgttgc caaccacatg     420
   ggatacgatg gagcgggcag cagcgttgac tactccgtct tcaacccctt ctcctcccag     480
   gactacttcc accccttctg cttcatccag aattacgagg accagaccca ggttgaggac     540
   tgctggttgg gtgacaacac tgtctctctg cccgatctcg acaccaccaa ggatgttgtc     600
   cgcaacgaat ggtacgactg ggtgggctct cttgtctcca actactccat cgatggcctc     660
   cgtatcgaca ctgtcaagca cgtccagaag gacttctggc ctggatacaa cgacgctgct     720
   ggtgtctact gcattggtga ggtcctcgat ggtgaccctg cctacacctg cccctaccag     780
   aacgtcatgg atggtgtcct caactaccct atctactacc ctctgctcaa cgccttccag     840
   tccacctccg gcagcatgga tgacctgtac aacatgatca acaccgtcaa gtccgactgc     900
   cccgactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaacg ccatccgcaa ctacgccatc tccaaggaca ccggtttcgt cacctacaag    1200
   aactggccca tctaccagga tgataccacc attgccatgc gcaagggcac cgatggctcc    1260
   cagatcgtca ccattttgtc gaacaagggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccatggccg gtggtcttcc tcgtgtcctc    1440
   taccccactg agaagctggc tggaagcggt atctgctcga gcagc                    1485
<210> 91
   <211> 1485
   <212> DNA
   <213> Artificial sequence
<220>
   <223> Fungal amylase variant
<400> 91
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg accacctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatgtac ctgatggttg atgttgttgc caaccacatg     420
   ggatacgatg gagcgggcag cagcgttgac tactccgtct tcaacccctt ctcctcccag     480
   gactacttcc accccttctg cttcatccag aattacgagg accagaccca ggttgaggac     540
   tgctggttgg gtgacaacac tgtctctctg cccgatctcg acaccaccaa ggatgttgtc     600
   aagaacgaat ggtacgactg ggtgggctct cttgtctcca actactccat cgatggcctc     660
   cgtatcgaca ctgtcaagca cgtccagaag gacttctggc ctggatacaa ctccgctgct     720
   ggtgtctact gcattggtga ggtcctcgat ggtgaccctg cctacacctg cccctaccag     780
   aacgtcatgg atggtgtcct caactaccct atctactacc ctctgctcaa cgccttcaag     840
   tccacctccg gcagcatgga tgacctgtac aacatgatca acaccgtcgc ctccgactgc     900
   cccgactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacca attgattgcc    1140
   tccgccaacg ccatccgcaa ctacgccatc tccaaggaca ccggtttcgt cacctacaag    1200
   aactggccca tctacaagga tgataccacc attgccatgc gcaagggcac cgatggctcc    1260
   cagatcgtca ccattttgtc gaacaagggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccatggccg gtggtcttcc tcgtgtcctc    1440
   taccccactg agaagctggc tggaagcggt atctgctcga gcagc                    1485
<210> 92
   <211> 1485
   <212> DNA
   <213> Artificial sequence
<220>
   <223> Fungal amylase variant
<400> 92
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct ccgtgccctt     360
   tcttctgctc tgcacgagcg tggcatgtac ctgatggttg atgttgttgc caaccacatg     420
   ggatacgatg gagcgggcag cagcgttgac tactccgtct tcaagccctt ctcctcccag     480
   gactacttcc accccttctg cttcatccag aattacgagg accagaccca ggttgaggac      540
   tgctggttgg gtgacaacac tgtctctctg cccgatctcg acaccaccaa ggatgttgtc     600
   aagaacgaat ggtacgactg ggtgggctct cttgtctcca actactccat cgatggcctc     660
   cgtatcgaca ctgtcaagca cgtccagaag gacttctggc ctggatacaa caaggctgct     720
   ggtgtctact gcattggtga ggtcctcgat ggtgaccctg cctacacctg cccctaccag     780
   aacgtcatgg atggtgtcct caactaccct atctactacc ctctgctcaa cgccttccag     840
   tccacctccg gcagcatgga tgacctgtac aacatgatca acaccgtcaa gtccgactgc     900
   cccgactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaacg ccatccgcaa ctacgccatc tcccaggaca ccggtttcgt cacctaccag    1200
   aactggccca tctaccagga tgataccacc attgccatgc gcaagggcac cgatggctcc    1260
   cagatcgtca ccattttgtc gaacctcggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccatggccg gtggtcttcc tcgtgtcctc    1440
   taccccactg agaagctggc tggaagcaag atctgctcga gcagc                    1485
<210> 93
   <211> 1485
   <212> DNA
   <213> Artificial sequence
<220>
   <223> Fungal amylase variant
<400> 93
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg accacctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct caaggccctt     360
   tcttctgctc tgcacgagcg tggcatgtac ctgatggttg atgttgttgc caaccacatg     420
   ggatacgatg gagcgggcag cagcgttgac tactccgtct tcaacccctt ctcctcccag    480
   gactacttcc accccttctg cttcatccag aattacgagg accagaccca ggttgaggac    540
   tgctggttgg gtgacaacac tgtctctctg cccgatctcg acaccaccaa ggatgttgtc     600
   cgcaacgaat ggtacgactg ggtgggctct cttgtctcca actactccat cgatggcctc     660
   cgtatcgaca ctgtcaagca cgtccagaag gacttctggc ctggatacaa cgacgctgct     720
   ggtgtctact gcattggtga ggtcctcgat ggtgaccctg cctacacctg cccctaccag     780
   aacgtcatgg atggtgtcct caactaccct atctactacc ctctgctcaa cgccttccag     840
   tccacctccg gcagcatgga tgacctgtac aacatgatca acaccgtcaa gtccgactgc     900
   cccgactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacca attgattgcc    1140
   tccgccaacg ccatccgcaa ctacgccatc tccgccgaca ccggtttcgt cacctacaag    1200
   aactggccca tctaccagga tgataccacc attgccatgc gcaagggcac cgatggctcc    1260
   cagatcgtca ccattttgtc gaacctcggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccatggccg gtggtcttcc tcgtgtcctc    1440
   taccccactg agaagctggc tggaagcggt atctgctcga gcagc                     1485
<210> 94
   <211> 1485
   <212> DNA
   <213> Artificial sequence
<220>
   <223> Fungal amylase variant
<400> 94
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg acaagctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct ccgtgccctt     360
   tcttctgctc tgcacgagcg tggcatgtac ctgatggttg atgttgttgc caaccacatg     420
   ggatacgatg gagcgggcag cagcgttgac tactccgtct tcaacccctt ctcctcccag     480
   gactacttcc accccttctg cttcatccag aattacgagg accagaccca ggttgaggac     540
   tgctggttgg gtgacaacac tgtctctctg cccgatctcg acaccaccaa ggatgttgtc     600
   aagaacgaat ggtacgactg ggtgggctct cttgtctcca actactccat cgatggcctc     660
   cgtatcgaca ctgtcaagca cgtccagaag gacttctggc ctggatacaa ctccgctgct     720
   ggtgtctact gcattggtga ggtcctcgat ggtgaccctg cctacacctg cccctaccag     780
   aacgtcatgg atggtgtcct caactaccct atctactacc ctctgctcaa cgccttccag     840
   tccacctccg gcagcatgga tgacctgtac aacatgatca acaccgtcgc ctccgactgc     900
   cccgactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacca attgattgcc    1140
   tccgccaacg ccatccgcaa ctacgccatc tcccaggaca ccggtttcgt cacctaccag    1200
   aactggccca tctaccagga tgataccacc attgccatgc gcaagggcac cgatggctcc    1260
   cagatcgtca ccattttgtc gaacaagggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccatggccg gtggtcttcc tcgtgtcctc    1440
   taccccactg agaagctggc tggaagcggt atctgctcga gcagc                    1485
<210> 95
   <211> 1485
   <212> DNA
   <213> Artificial sequence
<220>
   <223> Fungal amylase variant
<400> 95
   atggtcaagt ccatcctggc ctccgtcttc ttcgctgcca ctgctcttgc tgccaccccc 60
   gccgactggc gctcccagtc catctacttc ctgctcaccg accgtttcgc tcgcaccgac 120
   ggctccacta ctgccacctg caacactgcg gaccagaagt actgcggtgg cacctggcag 180
   ggtatcattg accacctcga ctacatccag ggtatgggtt tcactgccat ctggatcacc 240
   cccgtcactg ctcagctccc ccagaccact gcctacggtg atgcctacca cggatactgg 300
   cagcaggata tctactctct gaacgagaac tacggtactg ccgatgacct ccgtgccctt 360
   tcttctgctc tgcacgagcg tggcatgtac ctgatggttg atgttgttgc caaccacatg 420
   ggatacgatg gagcgggcag cagcgttgac tactccgtct tcaacccctt ctcctcccag 480
   gactacttcc accccttctg cttcatccag aattacgagg accagaccca ggttgaggac 540
   tgctggttgg gtgacaacac tgtctctctg cccgatctcg acaccaccaa ggatgttgtc 600
   cgcaacgaat ggtacgactg ggtgggctct cttgtctcca actactccat cgatggcctc 660
   cgtatcgaca ctgtcaagca cgtccagaag gacttctggc ctggatacaa ctccgctgct 720
   ggtgtctact gcattggtga ggtcctcgat ggtgaccctg cctacacctg cccctaccag 780
   aacgtcatgg atggtgtcct caactaccct atctactacc ctctgctcaa cgccttccag 840
   tccacctccg gcagcatgga tgacctgtac aacatgatca acaccgtcgc ctccgactgc 900
   cccgactcga ccctccttgg caccttcgtc gagaaccacg acaaccctcg cttcgcctcc 960
   tacaccaacg acattgccct tgccaagaac gtcgctgctt tcatcatcct caacgacggt 1020
   atccccatca tctatgctgg ccaggagcag cactacgctg gtggcaacga ccccgccaac 1080
   cgtgaggcca cctggctctc cggctaccct accgacagcg aactctacca attgattgcc 1140
   tccgccaacg ccatccgcaa ctacgccatc tcccaggaca ccggtttcgt cacctaccag 1200
   aactggccca tctaccagga tgataccacc attgccatgc gcaagggcac cgatggctcc 1260
   cagatcgtca ccattttgtc gaacctcggt gcttctggtg acagctacac tctgtctctt 1320
   tctggtgctg gctacactgc cggtcagcag ctgaccgaag tcattggatg caccaccgtc 1380
   accgttggct ccgatggcaa cgtccccgtg cccatggccg gtggtcttcc tcgtgtcctc 1440
   taccccactg agaagctggc tggaagcggt atctgctcga gcagc 1485
<210> 96
   <211> 1485
   <212> DNA
   <213> Artificial sequence
<220>
   <223> Fungal amylase variant
<400> 96
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg accacctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct ccgtgccctt     360
   tcttctgctc tgcacgagcg tggcatgtac ctgatggttg atgttgttgc caaccacatg     420
   ggatacgatg gagcgggcag cagcgttgac tactccgtct tcaacccctt ctcctcccag     480
   gactacttcc accccttctg cttcatccag aattacgagg accagaccca ggttgaggac     540
   tgctggttgg gtgacaacac tgtctctctg cccgatctcg acaccaccaa ggatgttgtc     600
   cgcaacgaat ggtacgactg ggtgggctct cttgtctcca actactccat cgatggcctc     660
   cgtatcgaca ctgtcaagca cgtccagccc gacttctggc ctggatacaa ctccgctgct     720
   ggtgtctact gcattggtga ggtcctcgat ggtgaccctg cctacacctg cccctaccag     780
   aacgtcatgg atggtgtcct caactaccct atctactacc ctctgctcaa cgccttccag     840
   tccacctccg gcagcatgga tgacctgtac aacatgatca acaccgtcgc ctccgactgc     900
   cccgactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacca attgattgcc    1140
   tccgccaacg ccatccgcaa ctacgccatc tcccaggaca ccggtttcgt cacctaccag    1200
   aactggccca tctaccagga tgataccacc attgccatgc gcaagggcac cgatggctcc    1260
   cagatcgtca ccattttgtc gaacctcggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccatggccg gtggtcttcc tcgtgtcctc    1440
   taccccactg agcgtctggc tggaagcggt atctgctcga gcagc                    1485
<210> 97
   <211> 1485
   <212> DNA
   <213> Artificial sequence
<220>
   <223> Fungal amylase variant
<400> 97
   atggtcaagt ccatcctggc ctccgtcttc ttcgctgcca ctgctcttgc tgccaccccc 60
   gccgactggc gctcccagtc catctacttc ctgctcaccg accgtttcgc tcgcaccgac 120
   ggctccacta ctgccacctg caacactgcg gaccagatct actgcggtgg cacctggcag 180
   ggtatcattg accacctcga ctacatccag ggtatgggtt tcactgccat ctggatcacc 240
   cccgtcactg ctcagctccc ccagaccact gcctacggtg atgcctacca cggatactgg 300
   cagcaggata tctactctct gaacgagaac tacggtactg ccgatgacct ccgtgccctt 360
   tcttctgctc tgcacgagcg tggcatgtac ctgatggttg atgttgttgc caaccacatg 420
   ggatacgatg gagcgggcag cagcgttgac tactccgtct tcaacccctt ctcctcccag 480
   gactacttcc accccttctg cttcatccag aattacgagg accagaccca ggttgaggac 540
   tgctggttgg gtgacaacac tgtctctctg cccgatctcg acaccacctc cgatgttgtc 600
   cgcaacgaat ggtacgactg ggtgggctct cttgtctcca actactccat cgatggcctc 660
   cgtatcgaca ctgtccgtca cgtccagccc gacttctggc ctggatacaa ctccgctgct 720
   ggtgtctact gcattggtga ggtcctcgat ggtgaccctg cctacacctg cccctaccag 780
   aacgtcatgg atggtgtcct caactaccct atctactacc ctctgctcaa cgccttccag 840
   tccacctccg gcagcatgga tgacctgtac aacatgatca acaccgtcgc ctccgactgc 900
   cccgactcga ccctccttgg caccttcgtc gagaaccacg acaaccctcg cttcgcctcc 960
   tacaccaacg acattgccct tgcccagaac gtcgctgctt tcatcatcct caacgacggt 1020
   atccccatca tctatgctgg ccaggagcag cactacgctg gtggcaacga ccccgccaac 1080
   cgtgaggcca cctggctctc cggctaccct accgacagcg aactctacca attgattgcc 1140
   tccgccaacg ccatccgcaa ctacgccatc tcccaggaca ccggtttcgt cacctaccag 1200
   aactggccca tctaccagga tgataccacc attgccatgc gccgtggcac cgatggctcc 1260
   cagatcgtca ccattttgtc gaacctcggt gcttctggtg acagctacac tctgtctctt 1320
   tctggtgctg gctacactgc cggtcagcag ctgaccgaag tcattggatg caccaccgtc 1380
   accgttggct ccgatggcaa cgtccccgtg cccatggccg gtggtcttcc tcgtgtcctc 1440
   taccccactg agcgtctggc tggaagcggt atctgctcga gcagc 1485
<210> 98
   <211> 1485
   <212> DNA
   <213> Artificial sequence
<220>
   <223> Fungal amylase variant
<400> 98
   atggtcaagt ccatcctggc ctccgtcttc ttcgctgcca ctgctcttgc tgccaccccc 60
   gccgactggc gctcccagtc catctacttc ctgctcaccg accgtttcgc tcgcaccgac 120
   ggctccacta ctgccacctg caacactgcg gaccagaagt actgcggtgg cacctggcag 180
   ggtatcattg acaagctcga ctacatccag ggtatgggtt tcactgccat ctggatcacc 240
   cccgtcactg ctcagctccc ccagaccact gcctacggtg atgcctacca cggatactgg 300
   cagcaggata tctactctct gaacgagaac tacggtactg ccgatgacct caaggccctt 360
   tcttctgctc tgcacgagcg tggcatgtac ctgatggttg atgttgttgc caaccacatg 420
   ggatacgatg gagcgggcag cagcgttgac tactccgtct tcaacccctt ctcctcccag     480
   gactacttcc accccttctg cttcatccag aattacgagg accagaccca ggttgaggac     540
   tgctggttgg gtgacaacac tgtctctctg cccgatctcg acaccaccaa ggatgttgtc 600
   cgcaacgaat ggtacgactg ggtgggctct cttgtctcca actactccat cgatggcctc 660
   cgtatcgaca ctgtcaagca cgtccagaag gacttctggc ctggatacaa cgacgctgct 720
   ggtgtctact gcattggtga ggtcctcgat ggtgaccctg cctacacctg cccctaccag 780
   aacgtcctgg atggtgtcct caactaccct atctactacc ctctgctcaa cgccttccag 840
   tccacctccg gcagcatgga tgacctgtac aacatgatca acaccgtcaa gtccgactgc 900
   cccgactcga ccctccttgg caccttcgtc gagaaccacg acaaccctcg cttcgcctcc 960
   tacaccaacg acattgccct tgccaagaac gtcgctgctt tcatcatcct caacgacggt 1020
   atccccatca tctatgctgg ccaggagcag cactacgctg gtggcaacga ccccgccaac 1080
   cgtgaggcca cctggctctc cggctaccct accgacagcg aactctacaa gttgattgcc 1140
   tccgccaacg ccatccgcaa ctacgccatc tccaaggaca ccggtttcgt cacctacaag 1200
   aactggccca tctaccagga tgataccacc attgccctcc gcaagggcac cgatggctcc 1260
   cagatcgtca ccattttgtc gaacaagggt gcttctggtg acagctacac tctgtctctt 1320
   tctggtgctg gctacactgc cggtcagcag ctgaccgaag tcattggatg caccaccgtc 1380
   accgttggct ccgatggcaa cgtccccgtg cccctcgccg gtggtcttcc tcgtgtcctc 1440
   taccccactg agaagctggc tggaagcggt atctgctcga gcagc 1485
<210> 99
   <211> 1485
   <212> DNA
   <213> Artificial sequence
<220>
   <223> Fungal amylase variant
<400> 99
   atggtcaagt ccatcctggc ctccgtcttc ttcgctgcca ctgctcttgc tgccaccccc      60
   gccgactggc gctcccagtc catctacttc ctgctcaccg accgtttcgc tcgcaccgac     120
   ggctccacta ctgccacctg caacactgcg gaccagaagt actgcggtgg cacctggcag     180
   ggtatcattg accacctcga ctacatccag ggtatgggtt tcactgccat ctggatcacc     240
   cccgtcactg ctcagctccc ccagaccact gcctacggtg atgcctacca cggatactgg     300
   cagcaggata tctactctct gaacgagaac tacggtactg ccgatgacct ccgtgccctt     360
   tcttctgctc tgcacgagcg tggcatgtac ctgatggttg atgttgttgc caaccacatg     420
   ggatacgatg gagcgggcag cagcgttgac tactccgtct tcaacccctt ctcctcccag     480
   gactacttcc accccttctg cttcatccag aattacgagg accagaccca ggttgaggac    540
   tgctggttgg gtgacaacac tgtctctctg cccgatctcg acaccaccaa ggatgttgtc     600
   cgcaacgaat ggtacgactg ggtgggctct cttgtctcca actactccat cgatggcctc     660
   cgtatcgaca ctgtcaagca cgtccagaag gacttctggc ctggatacaa ctccgctgct     720
   ggtgtctact gcattggtga ggtcctcgat ggtgaccctg cctacacctg cccctaccag     780
   aacgtcctgg atggtgtcct caactaccct atctactacc ctctgctcaa cgccttccag     840
   tccacctccg gcagcatcga tgacctgtac aacgagatca acaccgtcaa gtccgactgc     900
   cccgactcga ccctccttgg caccttcgtc gagaaccacg acaaccctcg cttcgcctcc     960
   tacaccaacg acattgccct tgccaagaac gtcgctgctt tcatcatcct caacgacggt    1020
   atccccatca tctatgctgg ccaggagcag cactacgctg gtggcaacga ccccgccaac    1080
   cgtgaggcca cctggctctc cggctaccct accgacagcg aactctacaa gttgattgcc    1140
   tccgccaacg ccatccgcaa ctacgccatc tccaaggaca ccggtttcgt cacctacaag    1200
   aactggccca tctacaagga tgataccacc attgccatgc gcaagggcac cgatggctcc    1260
   cagatcgtca ccattttgtc gaacaagggt gcttctggtg acagctacac tctgtctctt    1320
   tctggtgctg gctacactgc cggtcagcag ctgaccgaag tcattggatg caccaccgtc    1380
   accgttggct ccgatggcaa cgtccccgtg cccatggccg gtggtcttcc tcgtgtcctc    1440
   taccccactg agaagctggc tggaagcaag atctgctcga gcagc                    1485

## Description

### Field

The present disclosure relates to a method for the production of a polypeptide of interest in a host cell. The disclosure also relates to a modified polypeptide. Also, the invention relates to a method for improving the expression level of a polypeptide and to the use of a polypeptide of interest which is modified so as to increase the expression level of that polypeptide in a host cell.

### Background

The production of recombinant polypeptides in bacterial, yeast and fungal host cells is known in the art. Current production of polypeptides is performed in various ways.

The state of the art process for the production of recombinant polypeptides is by means of fermentation of a host cell comprising an expression construct, said expression construct comprising inter alia a promoter operably linked to a polynucleotide encoding the polypeptide of interest. The resulting polypeptide might accumulate intracellularly or may be further secreted via the secretory pathway of the host cell. In the latter case, the polypeptide of interest typically comprises a signal sequence. In Broekhuijsen et al (Journal of Biotechnology, 31 (1993) 135-145, Broekhuijsen et al; Secretion of heterologous proteins by Aspergillus niger: Production of active human interleukin-6 in a protease deficient mutant by KEX2-like processing of a glucoamylase-hIL6 fusion protein), a recombinant protein is expressed in *Aspergillus niger* using the signal sequence of the secreted polypeptide glucoamylase.

The yield of production of the recombinant polypeptide of interest may be enhanced by increasing the production and secretion efficiency, for example by using modified Kozak sequences (WO2008000632), codon-pair optimization (WO2008000632), introduction of improved signal sequences for secretion
(WO2010/121933). These methods do not change the amino acid composition of the mature polypeptide.

US 2004/142429 A1 discloses a method for expression, purification, and structure recovery and handling of hydrophobic peptides in prokaryotes. The method includes the steps of designing a fusion protein which contains in its amino acid sequence a hexa-His tag, a cyanogen bromide cleavage and subsequently expressing the fusion protein using a vector in such a way as to target expressed peptides to inclusion bodies. The inclusion bodies are then isolated and undergo cleavage of the fusion protein (with cyanogen bromide) to release the desired product and at the same time to expose the hexa-His tag. The recombinant peptides are then purified and recovery of the natural conformation and secondary structure of the peptides and their dispersion in un-aggregated form occurs by dissolution of the peptides in an acidic organic solvent that is compatible with common lipid. It is known that cyanogen bromide hydrolyses peptide bonds at the C-terminus of methionine residues. When the desired hydrophobic peptide sequence contains a methionine residue(s) anywhere but where eventual cyanogen bromide cleavage is desired, the methionine residue(s) is/are either removed or substituted, by known methods before expression of the fusion protein.

Recently, protein feature optimization (PFO) was introduced as a novel method for improving the secretion of a polypeptide of interest by a eukaryotic host cell by modifying the value of a set of relevant protein features in the amino acid backbone of the polypeptide to fall within an optimal range or to become more close to an optimal value for one or more protein features in the eukaryotic host. The protein features are properties that can be computationally derived from the protein amino acid sequence and DNA sequence (WO2010/102982).

In an industrial context, high yields of polypeptides produced are required. Consequently, to enhance the yield of production of a polypeptide of interest still further, there is a need to improve secretion efficiency. It is an object of the disclosure to provide an improved process for the production of a recombinant polypeptide.

### Summary

Knowledge of factors influencing protein production may be employed to improve enzyme production rates in industrial settings. High production yields can in some cases be obtained for homologous gene expression, but yields are often limited for heterologous gene expression. We have applied sequence-based machine learning techniques to identify relevant protein sequence features. The amino-acid composition of the protein sequence was found to be most predictive and interpretation revealed that, for both homologous and heterologous gene expression, the same features are important. In particular, methionine (M) and Lysine (K) were found to have a negative contribution to high-level production.

Accordingly, the present disclosure relates to a method for improving the secretion of a polypeptide of interest by a host cell, such as a eukaryotic host cell, by reducing the number of methionine, or lysine, or methionine and lysine amino acids in the amino acid backbone of the mature polypeptide.

One advantage is that reduction of the lysine (K), methionine (M), or lysine (L) and methionine (M) content contributes to improved protein production and leads to the development of lower cost processes.

According to the invention, there is thus provided a method for the production of a polypeptide of interest in a host cell, which method comprises:
a. providing a host cell which harbours a nucleic acid encoding a polypeptide of interest, wherein the polypeptide of interest is modified so that it comprises fewer methionine and/or lysine residues than a reference polypeptide, excluding any initial methionine amino acid located at the N-terminal end of the polypeptide sequence, wherein methionine and/or lysine amino acids in the reference polypeptide have been substituted with non-methionine and/or non-lysine amino acids and wherein modification occurs in the mature polypeptide backbone;
b. cultivating the host cell under conditions suitable for production of the polypeptide; and, optionally,
c. recovering the compound of interest,
wherein the number of methionine and/or lysine amino acids in the polypeptide of interest is reduced by at least 25% in comparison with the reference polypeptide, wherein the host cell is a yeast cell or a filamentous fungus cell, wherein the reference polypeptide is a polypeptide the expression of which is desired to increase in the host cell, wherein the substrate specificity or binding specificity of the modified polypeptide is maintained or its specific activity is the same as before improvement.

Typically, the polypeptide may be modified with respect to the coding sequence, including or excluding one or more or all control sequences (such as a signal sequence). The modification excludes the N-terminal amino acid.

The disclosure also relates to:
- a modified polypeptide which comprises fewer methionine and/or lysine residues than a reference polypeptide, excluding any initial methionine amino acid located at the N-terminal end of the polypeptide sequence;
- use of a polypeptide of interest which is modified so that it comprises fewer methionine and/or lysine residues than a reference polypeptide, excluding any initial methionine amino acid located at the N-terminal end of the polypeptide sequence, to increase the expression level of the polypeptide of interest in a host cell; and
a polypeptide having the amino acid sequence set out in SEQ ID NOs: 16, 17, 18, 19, 20, 8, 9, 10, 11, 12, 26, 27, 43, 44, 45, 28 or 29.

The invention also relates to:
- a method for improving the expression level of a polypeptide in a host cell, which method comprises reducing the number of methionine and/or lysine amino acids in the polypeptide as compared with a reference polypeptide, excluding any initial methionine amino acid located at the N-terminal end of the polypeptide sequence, wherein the number of methionine and/or lysine amino acids in the amino acid backbone of the polypeptide is reduced by at least 25%, wherein methionine and/or lysine amino acids in the reference polypeptide have been substituted with non-methionine and/or non-lysine amino acids and wherein modification occurs in the mature polypeptide backbone, wherein the host cell is a yeast cell or a filamentous fungus cell, wherein the reference polypeptide is a polypeptide the expression of which is desired to increase in the host cell, wherein the substrate specificity or binding specificity of the modified polypeptide is maintained or its specific activity is the same as before improvement.

### Brief description of the figures

Figure 1 shows the weights of the classifier. For each (negatively correlated) amino acid (x-axis), the bar indicates the importance / weight of the frequency of occurrence of this amino acid in a protein for obtaining a high production after overexpression.
Figure 2 shows a comparison of hom and het classifiers. Amino acid contributions obtained from hom and het classifiers are the x- and y-values, respectively. Contributions are normalized per classifier (axis): each contribution is divided by the maximum absolute contribution. The plot shows the contributions obtained from classifiers trained using the protein amino acid composition.
Figure 3 depicts a plasmid map of expression vector pGBFINEBA205 (construction described in Example 1). Indicated are the *gla*A flanking regions relative to the *amd*S selection marker cassette. In addition are indicated sequences of the *gla*A promoter and the EBA205 sequences encoding variant enzymes according a method of the disclosure. The *E. coli* DNA can be removed by digestion with restriction enzyme *Not*I, prior to transformation of the *A. niger* strains.
Figure 4 depicts a plasmid map of expression vector pGBFINFUA (construction described in Example 1). Indicated are the *gla*A flanking regions relative to the *amd*S selection marker cassette. In addition are indicated sequences of the *gla*A promoter and the EBA205 sequences encoding variant enzymes according a method of the disclosure. The *E. coli* DNA can be removed by digestion with restriction enzyme *Not*I, prior to transformation of the *A. niger* strains.
Figure 5 sets out EBA205 protein M-variant expression in shake-flask fermentation measured as FUA activity in supernatant; normalized to M-content 100% and protein concentartion (mg/ml) WT at 100%; data-points are average 1-3 independently selected strains and 1 x standard deviation is shown.
Figure 6 sets out EBA205 protein K-variant expression in shake-flask fermentation measured as FUA activity in supernatant; normalized to K-content 100% and protein concentration (mg/ml) WT at 100%; data-points are average 1-3 independently selected strains and 1 x standard deviation is shown.
Figure 7 sets out FUA protein M-variant expression in shake-flask fermentation measured as fungal alpha-amylase activity in supernatant; normalized to M-content 100% and activity WT at 100%; data-points are average 1-3 independently selected strains and 1 x standard deviation is shown.
Figure 8 sets out FUA protein K-variants expression in shake-flask fermentation measured as fungal alpha-amylase activity in supernatant; normalized to K-content 100% and activity WT at 100%; data-points are average 1-3 independently selected strains and 1 x standard deviation is shown.

### Description of the sequence listing

SEQ ID NO: 1: PEPTIDE: SS_EBA205 signal sequence EBA205; 18 amino acids .
SEQ ID NO: 2: PEPTIDE: SS_FUA signal sequence FUA; 20 amino acids.
SEQ ID NO: 3: PEPTIDE: SS_PmeA signal sequence; 17 amino acids.
SEQ ID NO: 4: DNA: SS_PmeA signal sequence; 51 base pairs: codon-pair optimized.
SEQ ID NO: 5: PROTEIN: EBA205 wild-type sequence, including signal sequence (SEQ ID NO: 1).
SEQ ID NO: 6: PROTEIN: FUA wild-type sequence, including signal sequence (SEQ ID NO: 2).
SEQ ID NO: 7: PROTEIN: FUA wild-type sequence, where signal sequence (SEQ ID NO: 2) is replaced by (SEQ ID NO: 3).
SEQ ID NO: 8-27: PROTEIN: EBA205 variant sequences based on SEQ ID NO: 5.
SEQ ID NO: 28-52: PROTEIN: FUA variant sequences based on SEQ ID NO: 7
SEQ ID NO: 53: DNA: codon-pair optimized (CPO): EBA205 sequence for SEQ ID NO: 5.
SEQ ID NO: 54: DNA: codon-pair optimized (CPO): FUA sequence for SEQ ID NO: 7.
SEQ ID NO: 55-74: DNA EBA205 sequence variants for SEQ ID NO: 8-27 based on template SEQ ID NO: 53.
SEQ ID NO: 75-99: DNA FUA sequence variants for SEQ ID NO: 28-52 based on template SEQ ID NO: 54.

### Detailed description

Throughout the present specification and the accompanying claims, the words "comprise", "include" and "having" and variations such as "comprises", "comprising", "includes" and "including" are to be interpreted inclusively. That is, these words are intended to convey the possible inclusion of other elements or integers not specifically recited, where the context allows.

The articles "a" and "an" are used herein to refer to one or to more than one (i.e. to one or at least one) of the grammatical object of the article. By way of example, "an element" may mean one element or more than one element

In the current disclosure, a polypeptide redesign method is applied to alter sequence properties without affecting enzymatic function. The rationale behind this is the observed characteristic amino acid compositions for proteins that provide high yields after over-expression compared to those that do not; thereby indicating that alteration of this characteristic can be used to improve secretion yields. However, the enzymatic activity should remain similar to the original activity to make the method useful for the enzyme industry. A characteristic difference in amino acid composition can be obtained by comparing individual amino acids composition of successful with less successful examples.

Protein engineering, using both rationalized and randomized approaches, has been used to e.g. increase thermostability, change by-product spectrum, let enzymes work in organic solvent, etc., avoid inclusion bodies, or form inclusion bodies, etc. Recently one approach focused on protein-feature optimization for increasing the expression of proteins (WO2010/102982). Recently, others (Goltermann et al (2010) approached protein evolution via amino acid and codon elimination and showed that it was possible to prepare a GFP free of phenylalanine. Others have looked at reduced amino-acid alphabets that will sustain life. Natural evolution produces complex protein folds with a 20-amino acid alphabet. Primordial protein synthesis, however, is believed to have involved only a handful of amino acids. Several studies have demonstrated that considerably reduced amino acid alphabets may be sufficient to encode native-like proteins (Tanaka et al. 2011, v6(3), e18034; Walter et al., 2005, The Journal of Biological Chemistry vol. 280, no. 45, pp. 37742-37746). for example Walter et al. 2005 (The Journal of Biological Chemistry vol. 280, NO. 45, pp. 37742-37746) constructed an active enzyme from a 9-amino acid alphabet, basically aiming at novel structures and functions. The simplified 9-amino acid alphabet (Asp, Glu, Asn, Lys/Phe, Ile, Leu, Met plus Arg) contains methionine and lysine. Tanaka et al. (Protein Science 2010 Vol. 19:786-795) evaluated comparative characterization of random sequence proteins consisting of 5, 12, and 20 kinds of amino acids. The 12-amino acid table contained lysine and methionine; the 5-amino acid alphabet (Ala, Gly, Val, Asp, and Glu) did not yield proteins exhibiting extensively well-folded structure.

We have observed, however, that, the Methionine (M) and Lysine (K) compositional fraction of a mature polypeptide is negatively correlated with expressivity. Accordingly, the disclosure relates to polypeptides and polypeptide expression methods, wherein the number of methionine and/or lysine amino acids is modified in a polypeptide, in particular reduced in comparison with a reference/starting polypeptide.

That is to say, the current disclosure takes the approach of modification, in particular reduction, of the amino acids methionine or lysine, or methionine and lysine (taken together) to increase expression of a protein, and, in the case of a catalytic enzyme, without losing its catalytic activity. This may lead to improved protein production levels and to consequent lower cost processes. The method according to the disclosure may be referred to a Protein Sequence Optimisation (PSO).

The reduction in the number of methionine and/or lysine amino acids in a polypeptide of interest may be carried out on the basis of the polypeptide including all of its control sequences (such as a signal sequence) or on the basis of the polypeptide excluding one or more or all of its control sequences (such as a signal sequence). The N-terminal amino acid is excluded.

According to the invention, there is thus provided a method for the production of a polypeptide of interest in a host cell, which method comprises:
a. providing a host cell which harbours a nucleic acid encoding a polypeptide of interest, wherein the polypeptide of interest is modified so that it comprises fewer methionine and/or lysine residues than a reference polypeptide, excluding any initial methionine amino acid located at the N-terminal end of the polypeptide sequence, wherein methionine and/or lysine amino acids in the reference polypeptide have been substituted with non-methionine and/or non-lysine amino acids and wherein modification occurs in the mature polypeptide backbone;
.b cultivating the host cell under conditions suitable for production of the polypeptide; and, optionally,
c. recovering the compound of interest,
wherein the number of methionine and/or lysine amino acids in the polypeptide of interest is reduced by at least 25% in comparison with the reference polypeptide, wherein the host cell is a yeast cell or a filamentous fungus cell, wherein the reference polypeptide is a polypeptide the expression of which is desired to increase in the host cell, wherein the substrate specificity or binding specificity of the modified polypeptide is maintained or its specific activity is the same as before improvement.

Thus, in the method of the invention, a polypeptide is selected and modified so that its expression level in a host cell may be increased. Typically, the nucleic acid sequence encoding the polypeptide is modified so that the resulting polypeptide is modified as desired. The nucleic acid that is harboured by the host cell may be in the form of a nucleic acid construct.

The term "nucleic acid construct" is herein referred to as a nucleic acid molecule, either single-or double-stranded, which is isolated from a naturally occurring gene or which has been modified to contain segments of nucleic acid which are combined and juxtaposed in a manner which would not otherwise exist in nature. The term nucleic acid construct is synonymous with the term "expression cassette" when the nucleic acid construct contains all the control sequences required for expression of a coding sequence, wherein said control sequences are operably linked to said coding sequence.

The term "operably linked" is defined herein as a configuration in which a control sequence is appropriately placed at a position relative to the coding sequence of the DNA sequence such that the control sequence directs the production of a polypeptide.

The term "control sequences" is defined herein to include all components, which are necessary or advantageous for the expression of mRNA and / or a polypeptide, either *in vitro* or in a host cell. Each control sequence may be native or foreign to the nucleic acid sequence encoding the polypeptide. Such control sequences include, but are not limited to, a leader, Shine-Delgarno sequence, optimal translation initiation sequences (as described in Kozak, 1991, J. Biol. Chem. 266:19867-19870), a polyadenylation sequence, a pro-peptide sequence, a pre-pro-peptide sequence, a promoter, a signal sequence, and a transcription terminator. At a minimum, the control sequences include a promoter, and transcriptional and translational stop signals. Control sequences may be optimized to their specific purpose. Preferred optimized control sequences used in the present disclosure are those described in WO2006/077258.

A signal sequence (sometimes referred to as a signal peptide) may be a short (for example from about 5 to about 30 amino acids long) peptide present at the N-terminus of the majority of newly synthesized proteins that are destined towards the secretory pathway. These proteins include those that reside either inside certain organelles (the endoplasmic reticulum, golgi or endosomes), secreted from the cell, or inserted into most cellular membranes. Although most type I membrane-bound proteins have signal peptides, the majority of type II and multi-spanning membrane-bound proteins are targeted to the secretory pathway by their first transmembrane domain, which biochemically resembles a signal sequence except that it is not cleaved.

The control sequences may be provided with linkers for the purpose of introducing specific restriction sites facilitating ligation of the control sequences with the coding region of the nucleic acid sequence encoding a polypeptide.

The control sequence may be an appropriate promoter sequence (promoter).

The control sequence may also be a suitable transcription terminator (terminator) sequence, a sequence recognized by a filamentous fungal cell to terminate transcription. The terminator sequence is operably linked to the 3'-terminus of the nucleic acid sequence encoding the polypeptide. Any terminator, which is functional in the cell, may be used in the present disclosure.

Preferred terminator sequences for filamentous fungal cells are obtained from the polynucleotides encoding *A. oryzae* TAKA amylase, *A. niger* glucoamylase (glaA), *A. nidulans* anthranilate synthase, *A. niger* alpha-glucosidase, trpC and *Fusarium oxysporum* trypsin-like protease.

The control sequence may also be a suitable leader sequence (leaders), a non-translated region of a mRNA which is important for translation by the filamentous fungal cell. The leader sequence is operably linked to the 5'-terminus of the nucleic acid sequence encoding the polypeptide. Any leader sequence, which is functional in the cell, may be used in the present disclosure.

Preferred leaders for filamentous fungal cells are obtained from the polynucleotides encoding *A. oryzae* TAKA amylase and *A. nidulans* triose phosphate isomerase and *A. niger* glaA and phytase.

Other control sequences may be isolated from the *Penicillium* IPNS gene, or pcbC gene, the beta tubulin gene. Control sequences are disclosed in WO 01/21779.

The control sequence may also be a polyadenylation sequence, a sequence which is operably linked to the 3'-terminus of the nucleic acid sequence and which, when transcribed, is recognized by the filamentous fungal cell as a signal to add polyadenosine residues to transcribed mRNA. Any polyadenylation sequence, which is functional in the cell, may be used in the present disclosure.

Preferred polyadenylation sequences for filamentous fungal cells are obtained from the polynucleotides encoding *A. oryzae* TAKA amylase, *A. niger* glucoamylase, *A. nidulans* anthranilate synthase, *Fusarium oxysporum* trypsin-like protease and *A. niger* alpha-glucosidase.

The term "promoter" is defined herein as a DNA sequence that binds RNA polymerase and directs the polymerase to the correct downstream transcriptional start site of a nucleic acid sequence encoding a biological compound to initiate transcription. RNA polymerase effectively catalyzes the assembly of messenger RNA complementary to the appropriate DNA strand of a coding region. The term "promoter" will also be understood to include the 5'-non-coding region (between promoter and translation start) for translation after transcription into mRNA, cis-acting transcription control elements such as enhancers, and other nucleotide sequences capable of interacting with transcription factors. The promoter may be any appropriate promoter sequence suitable for a eukaryotic or prokaryotic host cell, which shows transcriptional activity, including mutant, truncated, and hybrid promoters, and may be obtained from polynucleotides encoding extracellular or intracellular polypeptides either homologous (native) or heterologous (foreign) to the cell. The promoter may be a constitutive or inducible promoter.

Modification of a polypeptide is herein intended to encompass any event resulting in a change in the amino acid sequence of the polypeptide. A modification is construed as one or more modifications. Modification may be accomplished by the introduction (insertion), substitution or removal (deletion) of one or more amino acids in the polypeptide backbone. Clearly, such modification may readily be effected by modifying the sequence coding for the polypeptide of interest such that the desired modifications are present.

Typically, modification in the disclosure is reduction of methionine and/or lysine residues. The number of methionine residues or lysine residues or methionine and lysine residues (taken together) may be reduced.

Modification according to the disclosure may be carried out on the basis of the polypeptide of interest including control sequences (such as a signal sequence) or may be carried out on the basis of the polypeptide of interest minus one or more or all of its control sequences (such as a signal sequence).

In the method of the invention, the methionine and/or lysine amino acids in the reference polypeptide may be substituted with non-methionine and/or non-lysine amino acids. That is to say, all or some of the methionine and/or lysine amino acids in the reference polypeptide may be substituted.

In the present invention, a polypeptide may be modified in order to increase its expression level in a host cell. An increase in expression level encompasses any way in which the amount of resulting polypeptide may be increased. Typically, an increase in secretion will be preferred. The term 'secretion' refers to the appearance of a polypeptide in the extracellular medium, typically the growth medium or production medium. The polypeptide which is secreted is free from the biomass. The level of secretion may be measured by methods known in the art, including by activity assays (units of activity), specific activity (units per weight protein), quantitative PAGE analysis, quantitative mass spectrometry and antibody assays.

Thus, the method of the disclosure may be used to increase/improve secretion of a polypeptide. Accordingly, in a method of the disclosure, the polypeptide of interest may be a secreted protein.

The expression 'improvement of the secretion of a polypeptide' refers to an increase in the amount of polypeptide which is secreted in the extracellular medium of a cell. The improvement may be reflected by the fact that a polypeptide which is normally not secreted, such as for example an intracellular polypeptide, is now secreted. The improvement may also reside in the fact that a polypeptide which is expected to be secreted, for example because it contains a signal sequence, and which is not secreted, is now secreted. Improvement is of course always measured with reference to identical host genetic background and identical culture or fermentation conditions. In these cases, improved secretion may be clear from, for example, the appearance of a protein band in a polyacrylamide gel, where there was no band visible before improvement.

Alternatively, the improvement may be reflected by the fact that a polypeptide which is secreted in very low amounts, shows increased levels of secretion.

The amount of polypeptide secreted may be determined by measuring the activity of the polypeptide in the extracellular medium. In comparison to the situation before improvement, the activity in the extracellular medium may be increased by at least 5%, at least 10%, at least 15% or at least 20%. Preferably the activity is increased by at least 25%, at least 30%, at least 35% or at least 40%. In a more preferred embodiment, the activity is at least 45%, at least 50%, at least 60%, at least 70%, at least 80%, at least 90%, at least 100%, at least 200%, at least 500% or at least 1000% increased. The activity may be increased from no activity to some activity in the extracellular medium. "Activity" in this context typically refers to the total activity of the polypeptide in the extracellular medium which is used as a measure of an increased amount of protein in the extracellular medium. "Activity" is not intended to be a measure of specific activity - that is to say, "activity" is used to identify proteins which are secreted better than non-modified polypeptides (the intrinsic enzymatic activity of the polypeptide itself may be unaffected).

According to the method of the disclosure, the number of a set of relevant amino acids to be chosen from methionine and/or lysine in the amino acid backbone is modified to be reduced by at least about 1%, at least about 2%, at least about 5%, at least about 10%, at least about 15%, at least about 20%, at least about 25%, at least about 30%, at least about 40%, at least about 50%, at least about 60%, at least about 70%, at least about 80%, at least about 90%, at least about 95%, at least about 99% or more. These percentages refer to the amount of modification with respect to the total number of methionine or lysine residues (depending on which is being modified). If methionine and lysine residues are modified, these percentages typically refer to the total modifications of both types of residue taken together. However, they can refer to the total modifications made with respect to one of the residues.

According to the method of the disclosure, the number of a set of relevant amino acids to be chosen from methionine and/or lysine in the amino acid backbone is modified to be reduced by at two, three, four, five, six, seven, eight, nine, ten, about fifteen or about twenty, or about twenty-five, or about 30 or more. That is to say, in the method of the disclosure a reference polypeptide may be modified by carrying out modifications to two, three, four, five, six, seven, eight, nine, ten, about fifteen or about twenty, or about twenty-five, or about 30 or more methionine residues and/or by carrying out modifications to two, three, four, five, six, seven, eight, nine, ten, about fifteen or about twenty, or about twenty-five, or about 30 or more lysine residues.

Such modifications may be, for example, substitutions, or replacements. A substitution will be with an amino acid other than methionine or lysine. A preferred substitution may be with an amino acid other than methionine or lysine which occurs at a corresponding position in a related polypeptide (for example the corresponding polypeptide from another species from which the reference polypeptide is derived).

Any initial methionine amino acid located at the N-terminal end of the polypeptide sequence is excluded from modification. Also, any methionines and/or lysine amino acids in one or more control sequences, in particular one or more signal sequences, is excluded from modification.

Also, one or more methionine and/or lysine residues may be essential for the functionality of a polypeptide of interest. Typically, such an amino acid will not be modified in the disclosure. Such an amino acid may be indicated if it is shared between a set of related polypeptides (for example the cognate polypeptide from different species), i.e. appears at the same corresponding position within that set of polypeptides.

On the other hand, a polypeptide which may be preferentially modified may be one where there appears to be variation in nature, i.e. different amino acids appear at the same corresponding position within a related set of polypeptides.

The number of modifications is determined in relation to a reference polypeptide. The reference polypeptide may be any polypeptide the expression of which it is desired to increase in a host cell. In particular, a reference polypeptide may be a wild-type polypeptide. Wild-type herein may refer to the typical form of the polypeptide as it appears in nature. It is of course the case that gene loci may exist in a variety of allelic forms. Typically, the most prevalent allele may be considered the wild-type. However, for the purposes of the disclosure, any naturally-occurring allele may be the wild-type. However, the reference polypeptide may be any polypeptide, such as a modified wild-type or a hybrid polypeptide composed of two or more wild-type sequences (e.g. a consensus sequence) or a mixture of wild-type and non-wild-type sequences. Also, the reference polypeptide may be a non-wild type sequence. Such a polypeptide may be modified according to the disclosure so that its expression level in a host cell in increased.

The reference polypeptide could be a polypeptide which has been modified according to the disclosure. That is to say, the method according to the disclosure may be an iterative process. Such an iterative process could be carried out by first modifying the number of one or methionine or lysine and then carrying out a second modification based on the other of methionine or lysine. One round of modification of methionine and/or lysine could be followed by one or more additional rounds of modification of methionine and/or lysine.

Accordingly, in the disclosure, a polypeptide, that is to say a reference polypeptide, is provided. That polypeptide is then modified according to the disclosure, that is to say, the number of methionine and/or lysine residues in the polypeptide is reduced. In this way, the expression level of the polypeptide may be increased.

A reference polypeptide may be a polypeptide sequence having at least about 70%, at least about 75%, at least about 80%, at least about 85%, at least about 90%, at least about 95%, at least about 98%, at least about 99% identity with one or more corresponding wild-type polypeptides.

Further, the number of methionine and/or lysine amino acids in the reference polypeptide may be determined as the average number of methionine and/or lysine amino acids in two or more reference polypeptides. Thus, the number of methionine and/or lysine residues could be averaged over two or more corresponding wild-type sequences obtained from two or more different species. That is to say, the reference polypeptide could be a consensus sequence. The polypeptide of interest would be
modified in that it would contain fewer methionine and/or lysine residues than this average.

In the method of the invention, the reduction in methionine and/or lysine amino acids in polypeptide of interest may be determined over a length of at least about 80 or more contiguous amino acids. The reduction in methionine and/or lysine amino acids may be determined within one functional domain of the polypeptide, in particular if the polypeptide of interest is a fusion protein or a chimeric protein.

Typically, the reduction of methionine and/or lysine amino acids in the polypeptide of interest is determined without taking into account signal sequences that may be processed in the host cell. That is to say, the reduction of methionine and/or lysine amino acids in the polypeptide of interest may typically be determined with reference to a mature polypeptide sequence, for example after processing of pre- and/or pro- sequences, for example, has occurred.

The disclosure also relates to a polypeptide modified as set out herein. That is to say, the disclosure concerns a modified polypeptide which comprises fewer methionine and/or lysine residues than a reference polypeptide, excluding any initial methionine amino acid located at the N-terminal end of the polypeptide sequence. The reference polypeptide may be a corresponding wild-type polypeptide.

Accordingly, the disclosure relates to a polypeptide having the amino acid sequence set out in SEQ ID NOs: 16, 17, 18, 19, 20, 8, 9, 10, 11, 12, 26, 27, 43, 44, 45, 28 or 29.

The disclosure relates to variants of these polypeptides in which one or more amino acids other than lysine or methionine residues are modified and which has at least about 35% identity, more preferably at least 40% identity, more preferably at least 45% identity, more preferably at least 50% identity, even more preferably at least 55% identity, even more preferably at least 60% identity, even more preferably at least 65% identity, even more preferably at least 70% identity, even more preferably at least 75% identity, even more preferably at least 80% identity, even more preferably at least 85% identity, even more preferably at least 90% identity, for example at least 91% identity, for example at least 92% identity, for example at least 93% identity, for example at least 94% identity, for example at least 95% identity, for example at least 96% identity, for example at least 97% identity, for example at least 98% identity, for example at least 99% identity, for example 100% identity with a sequence as set out in any one of SEQ ID NOs: 16, 17, 18, 19, 20, 8, 9, 10, 11, 12, 26, 27, 43, 44, 45, 28 or 29.

The invention also relates to a method for improving the expression level of a polypeptide in a host cell, which method comprises reducing the number of methionine and/or lysine amino acids in the polypeptide as compared with a reference polypeptide, excluding any initial methionine amino acid located at the N-terminal end of the polypeptide sequence, wherein the number of methionine and/or lysine amino acids in the amino acid backbone of the polypeptide is reduced by at least 25%, wherein methionine and/or lysine amino acids in the reference polypeptide have been substituted with non-methionine and/or non-lysine amino acids and wherein modification occurs in the mature polypeptide backbone, wherein the host cell is a yeast cell or a filamentous fungus cell, wherein the reference polypeptide is a polypeptide the expression of which is desired to increase in the host cell, wherein the substrate specificity or binding specificity of the modified polypeptide is maintained or its specific activity is the same as before improvement.

Also, the disclosure relates to use of a polypeptide of interest which is modified so that it comprises fewer methionine and/or lysine residues than a reference polypeptide, typically excluding any initial methionine amino acid located at the N-terminal end of the polypeptide sequence, and/or optionally excluding one or more control sequences (such as) to increase the expression level of the polypeptide of interest in a host cell.

According to the method of the disclosure, modifications, in particular reductions of the numbers of methionine and/or lysine residues, are made to the polypeptide backbone. In this context, the term "backbone" refers to the regular structure which is formed when amino acids are linked together through peptide bonds and form a sequence of covalently linked amino acids. In the present invention, the backbone of the mature polypeptide is modified. In the context of the present disclosure "mature polypeptide" is defined herein as a polypeptide that is in its final functional form following translation and any post-translational modifications, such as N-terminal processing, C-terminal truncation, glycosylation, phosphorylation, etc.

The polypeptide before modification is referred to as the parent or reference (for example a wild-type polypeptide) to distinguish it from the modified polypeptide which results from it, which may be referred to as the polypeptide of interest. The terms "parent-", and "reference-polypeptide" are used interchangeably herein. When the polypeptide is a chimeric polypeptide, i.e. a translational fusion with an efficiently secreted polypeptide, preferably a polypeptide native to the host cell, the entire chimeric polypeptide may be modified according to the disclosure. When the chimeric polypeptide comprises an efficiently secreted polypeptide as a leader polypeptide fused to polypeptide of interest, the polypeptide of interest is preferably modified. A reference polypeptide may be a variant of a different parent polypeptide.

As is known to the person skilled in the art it is possible that the N-termini of the mature polypeptide might be heterogeneous as well as the C-terminus of the mature polypeptide due to processing errors during maturation. In particular such processing errors might occur upon overexpression of the polypeptide. In addition, exo-protease activity might give rise to heterogeneity. The extent to which heterogeneity occurs depends also on the host and fermentation protocols that are used. Such N-terminal and C-terminal processing artefacts might lead to shorter polypeptides or longer polypeptides compared with the expected mature polypeptide.

Methods to identify amino acids crucial for essential functional properties of interest are known in the art. Suitable tools include using a 3D structure or a 3D model of the protein of interest, mutagenesis studies of the protein of interest or of homologous proteins, the use of site saturated libraries to establish functionally neutral substitutions versus functional substitutions.

When introducing amino acid sequence features, such as methionine and/or lysine substitutions/deletions according to the disclosure, substitutions may preferably be chosen in such a way that at the given position the required amino acid sequence characteristic are selected from the group of amino acids which is observed in homologous sequences. State of the art modeling techniques may be applied to identify allowable substitutions which are not observed in natural homologues. Preferred references for modelling techniques which allow the generation of new sequences adopting a given fold are:
Kuhlman B, Dantas G, Ireton GC, Varani G, Stoddard BL, Baker D (2003). Design of a novel globular protein fold with atomic-level accuracy Science 302, 1364-8.

Baker D (2006). Prediction and design of macromolecular structures and interactions. Philos. Trans. R. Soc. Lond., B, Biol. Sci. 361, 459-63 De Novo protein design: towards fully automated sequence selection Journal of Molecular Biology, Volume 273, Issue 4, 7 November 1997, Pages 789-796 Bassil I. Dahiyat, Catherine A. Sarisky, Stephen L. Mayo

State of the art computational method allow for the generation of numerous potential sequences which may adopt a given protein fold. By introducing protein sequence optimisation (PSO) into the scoring functions which are used to filter out the most optimal sequences the most optimal sequences for a given production host might be selected in a computational way.

The amino-acid fraction computed from the entire polypeptide or DNA sequence is an average value for the entire mature protein, which may not reveal local protein properties. For example, a protein could be on average hydrophilic but still contain a large internal hydrophobic region. Local protein properties can be computed using a sliding window method of 30, 50, and preferably 80 amino acids or more.

In order to modulate the hydrophilicity of the protein accessible surface a 3D structure or a 3D structural model may be required. The 3D structure of protein can be determined by X-ray crystallography and by NMR. In addition comparative modelling or template based modelling can be applied to construct reliable 3D models for a given sequences based on 3D structures of homologous proteins (http://en.wikipedia.org/wiki/Homology_modeling). Various servers and software packages for comparative modelling be found at: http://en.wikipedia.org/wiki/Protein_structure_prediction_software.
For a recent review on protein structure prediction and modelling see Yang Zhang, Current Opinion in Structural Biology 2008, 18:342-348.

Given the atomic coordinates of a 3D structure or 3D model the accessible surface can be calculated by methods known in the art. A well-known method is the calculation via a rolling-ball algorithm developed by Frederic Richards (1977, "Areas, volumes, packing and protein structure." Annu Rev Biophys Bioeng, 6:151-176). See also http://en.wikipedia.org/wiki/Accessible_surface_area.

For determination of the accessible surface the quaternary structure of the final mature protein should be considered in order to avoid that substitutions will disturb the interaction between the individual polypeptides (the monomers) in the multimer (e.g. dimer, trimer, tetramer etc).

According to the disclosure, there is provided a process for the production of a compound of interest. The process comprises cultivating cells capable of producing a polypeptide of interest, because they comprise a polynucleotide encoding the polypeptide of interest, in a reaction medium under conditions which allow for production of the polypeptide of interest.

The compound of interest may then be recovered from the medium (or from the cells in the event that the compound of interest is not secreted).

It is to be understood that the methods according to the present disclosure can conveniently be combined with a state of the art technique to increase levels of protein production or with combinations of one or more of these techniques. These include but are not limited to application of strong promoters, increase of copy number, optimal Kozak sequence, mRNA stabilizing elements and optimized codon usage (WO2008/000632).

The host cell used in the disclosure may be a eukaryotic cell.

The eukaryotic cell is a fungal cell, i.e. a yeast cell, such as *Candida, Hansenula, Kluyveromyces, Pichia, Saccharomyces, Schizosaccharomyces,* or *Yarrowia strain.* More preferably from *Kluyveromyces lactis, S. cerevisiae, Hansenula polymorpha, Yarrowia lipolytica* and *Pichia pastoris,* or a filamentous fungal cell. Most preferably, the eukaryotic cell is a filamentous fungal cell.

"Filamentous" fungi include all filamentous forms of the subdivision *Eumycota* and *Oomycota* (as defined by Hawksworth et al., In, Ainsworth and Bisby's Dictionary of The Fungi, 8th edition, 1995, CAB International, University Press, Cambridge, UK). The filamentous fungi are characterized by a mycelial wall composed of chitin, cellulose, glucan, chitosan, mannan, and other complex polysaccharides. Vegetative growth is by hyphal elongation and carbon catabolism is obligately aerobic. Filamentous fungal strains include, but are not limited to, strains of *Acremonium, Agaricus, Aspergillus, Aureobasidium, Chrysosporium*, *Coprinus, Cryptococcus, Filibasidium, Fusarium, Geosmithia, Humicola, Magnaporthe, Mucor, Myceliophthora, Neocallimastix, Neurospora, Paecilomyces, Penicillium, Piromyces, Phanerochaete, Pleurotus, Rasamsonia, Schizophyllum, Talaromyces, Thermoascus*, *Thermomyces, Thielavia, Tolypocladium,* and *Trichoderma.*

Preferred filamentous fungal cells belong to a species of an *Acremonium, Aspergillus, Chrysosporium, Myceliophthora, Penicillium, Rasamsonia, Talaromyces, Thielavia, Fusarium* or *Trichoderma* genus, and most preferably a species of *Aspergillus niger, Acremonium alabamense, Aspergillus awamori, Aspergillus foetidus, Aspergillus sojae, Aspergillus fumigatus, Talaromyces emersonii, Talaromyces thermophilus, Thermomyces lanuginosus, Thermoascus thermophilus, Thermoascus aurantiacus, Thermoascus crustaceus, Rasamsonia emersonii, Rasamsonia byssochlamyoides, Rasamsonia argillacea, Rasamsonia brevistipitata, Rasamsonia cylindrospora, Aspergillus oryzae, Chrysosporium lucknowense, Fusarium oxysporum, Myceliophthora thermophila, Trichoderma reesei, Thielavia terrestris* or *Penicillium chrysogenum.* A more preferred host cell belongs to the genus *Aspergillus,* more preferably the host cell belongs to the species *Aspergillus niger.* When the host cell according to the disclosure is an *Aspergillus niger* host cell, the host cell preferably is CBS 513.88, CBS124.903 or a derivative thereof. A more preferred host cell belongs to the genus *Penicillium,* more preferably the host cell belongs to the species *Penicillium chrysogenum.* When the host cell according to the disclosure is a *Penicillium chrysogenum* host cell, the host cell preferably is Wisconsin 54-1255 or a derivative thereof. A more preferred host cell belongs to the genus *Rasamsonia* also known as *Talaromyces,* more preferably the host cell belongs to the species *Talaromyces emersonii* also known as *Rasamsonia emersonii.*

Several strains of filamentous fungi are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), Agricultural Research Service Patent Culture Collection, Northern Regional Research Center (NRRL), and All-Russian Collection of Microorganisms of Russian Academy of Sciences, (abbreviation in Russian - VKM, abbreviation in English - RCM), Moscow, Russia. Useful strains in the context of the present disclosure may be *Aspergillus niger* CBS 513.88, CBS124.903, *Aspergillus oryzae* ATCC 20423, IFO 4177, ATCC 1011, CBS205.89, ATCC 9576, ATCC14488-14491, ATCC 11601, ATCC12892, *P. chrysogenum* CBS 455.95, *P. chrysogenum* Wisconsin 54-1255 (ATCC28089), P*enicillium citrinum* ATCC 38065, *Penicillium chrysogenum* P2, *Thielavia terrestris* NRRL8126, *Talaromyces emersonii* CBS 124.902, *Acremonium chrysogenum* ATCC 36225 or ATCC 48272, *Trichoderma reesei* ATCC 26921 or ATCC 56765 or ATCC 26921, *Aspergillus sojae* ATCC11906, *Myceliophthora thermophila* C1, Garg 27K, VKM-F 3500 D,
*Chrysosporium lucknowense* C1, Garg 27K, VKM-F 3500 D, ATCC44006 and derivatives thereof.

In one embodiment, the eukaryotic cell is a host cell in which the polypeptide is produced by recombinant technology. Suitable methods for transforming or transfecting host cells can be found in Sambrook, et al. (Molecular Cloning: A Laboratory Manual, 2nd,ed. Cold Spring Harbor Laboratory, Cold Spring Harbor Laboratory Press, Cold Spring Harbor, NY, 1989), Davis et al., Basic Methods in Molecular Biology (1986) and other laboratory manuals. Accordingly, the present disclosure also relates to a method for the production of a polypeptide of interest by applying a method according to the disclosure to improve the expression, for example secretion, of the polypeptide to the polypeptide of interest and producing the polypeptide modified according to the disclosure by recombinant technology. The present disclosure also relates to said recombinantly produced polypeptide. The present disclosure also relates to a polypeptide obtainable by a method according to the disclosure to improve the expression, for example secretion, of the polypeptide; preferably said polypeptide is obtained by a method according to the disclosure to improve the secretion of the polypeptide.

The polypeptide of interest of which the expression level is to be improved is improved according to a method of the disclosure may be any polypeptide having a biological activity of interest. The polypeptide may be a collagen or gelatin, or a variant or hybrid thereof. The polypeptide may be an antibody or parts thereof, an antigen, a clotting factor, an enzyme, a hormone or a hormone variant, a receptor or parts thereof, a regulatory protein, a structural protein, a reporter, or a transport protein such as serum albumin, e.g. Bovine Serum Albumin and Human Serum Albumin, or such as a transferrin, e.g. lactoferrin, a protein involved in secretion process, a protein involved in folding process, a chaperone, a peptide amino acid transporter, a glycosylation factor, a transcription factor, a synthetic peptide or oligopeptide, a protein which in its native form is an intracellular protein and is secreted by methods known in the art such as fusion to a signal peptide and fusion to a polypeptide that is already secreted in its native form. Such intracellular protein may be an enzyme such as a protease, ceramidases, epoxide hydrolase, aminopeptidase, acylases, aldolase, hydroxylase, aminopeptidase, lipase. The polypeptide may be an enzyme secreted extracellularly in its native form. Such enzymes may belong to the groups of oxidoreductase, transferase, hydrolase, lyase, isomerase, ligase, catalase, cellulase, chitinase, cutinase, deoxyribonuclease, dextranase, esterase. The enzyme may be a carbohydrase, e.g. cellulases such as endoglucanases, β-glucanases, cellobiohydrolases or β-glucosidases, hemicellulases or pectinolytic enzymes such as xylanases, xylosidases, mannanases, galactanases, galactosidases, pectin methyl esterases, pectin lyases, pectate lyases, endo polygalacturonases, exopolygalacturonases rhamnogalacturonases, arabanases, arabinofuranosidases, arabinoxylan hydrolases, galacturonases, lyases, or amylolytic enzymes; hydrolase, isomerase, or ligase, phosphatases such as phytases, esterases such as lipases, proteolytic enzymes, oxidoreductases such as oxidases, transferases, or isomerases. The enzyme may be a phytase. The enzyme may be an aminopeptidase, asparaginase, amylase, carbohydrase, carboxypeptidase, endo-protease, metallo-protease, serine-protease catalase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, protein deaminase, invertase, laccase, lipase, mannosidase, mutanase, oxidase, pectinolytic enzyme, peroxidase, phospholipase, polyphenoloxidase, ribonuclease, transglutaminase, or glucose oxidase, hexose oxidase, monooxygenase. The polypeptide of which the secretion is improved may be homologous or heterologous to the host cell. A suitable example of a homologous polypeptide is an *Aspergillus niger* protein which is cloned into and produced by an *Aspergillus niger.* Suitable examples of heterologous expression include a bacterial polypeptide, for example from *E. coli* or *Bacillus,* cloned into and produced by a filamentous fungus or a yeast, or a mammalian protein, for example from bovine or goat, which is cloned into and produced by a filamentous fungus or a yeast, or a filamentous fungal polypeptide which is cloned and produced by a yeast, or a filamentous fungal protein which is cloned into and produced by another fungus. Preferably, the nucleic acids encoding the polypeptides are optimized, for example by codon pair optimization, for expression in the relevant host cell. Codon-pair optimization is a method wherein the nucleotide sequences encoding a polypeptide have been modified with respect to their codon-usage, in particular the codon-pairs that are used, to obtain improved expression of the nucleotide sequence encoding the polypeptide and/or improved production of the encoded polypeptide. Codon pairs are defined as a set of two subsequent triplets (codons) in a coding sequence. Codon-pair optimization is preferably performed as described in WO2008/000632.

Preferably, the specificity of the modified polypeptide is substantially the same as before the improvement in expression level. This means for example that substrate specificity or binding specificity is substantially maintained. In this context, the term "substantially maintained" means that more than 60%, more than 65%, more than 70% or more than 75% of the specificity is maintained. Preferably more than 80%, 85% or 90% of the specificity is maintained. Most preferably, more than 95%, 96%, 97%, 98% or 99% of the specificity is maintained.

According to the method of the disclosure, the level of activity in the extracellular medium may be increased, which is an indication of improved secretion. However, specific activity of the modified polypeptide does not have to be increased, as long as it is not decreased. Therefore, specific activity is preferably substantially the same as or higher than before the improvement of secretion. In a preferred embodiment, specific activity is substantially the same as before improvement. In this context the phrase 'substantially the same level of activity' refers to a level of activity which differs less than 15%, preferably less than 12% or less than 10%, more preferably less than 8%, less than 6% or less than 4% from the level of activity of the parent polypeptide.

In the present context, the terms 'polypeptide' and protein' are used interchangeably. Any type of polypeptide may have its secretion improved by the method of the disclosure. In a preferred embodiment, the polypeptide is one of the list cited earlier herein. For the purpose of this disclosure, it is defined here that in order to determine the percent identity of two amino acid sequences or of two nucleic acid sequences, the complete sequences are aligned for optimal comparison purposes. In order to optimize the alignment between the two sequences gaps may be introduced in any of the two sequences that are compared. Such alignment may be carried out over the full length of the sequences being compared or on the basis of longest identity. The identity is the percentage of identical matches between the two sequences over the reported aligned region.

A comparison of sequences and determination of percent identity between two sequences can be accomplished using a mathematical algorithm. The homology or identity between two aligned sequences may be calculated as follows: Number of corresponding positions in the alignment showing an identical amino acid in both sequences divided by the total length of the alignment after subtraction of the total number of gaps in the alignment. The skilled person will be aware of the fact that several different computer programs are available to align two sequences and determine the homology between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley). The percent identity between two amino acid sequences can be determined using the Needleman and Wunsch algorithm for the alignment of two sequences. (Needleman, S. B. and Wunsch, C. D. (1970) J. Mol. Biol. 48, 443-453). The algorithm aligns amino acid sequences as well as nucleotide sequences. The Needleman-Wunsch algorithm has been implemented in the computer program NEEDLE. For the purpose of this disclosure the NEEDLE program from the EMBOSS package was used (version 2.8.0 or higher, EMBOSS: The European Molecular Biology Open Software Suite (2000) Rice,P. Longden,I. and Bleasby,A. Trends in Genetics 16, (6) pp276-277, http://emboss.bioinformatics.nl/). For protein sequences, EBLOSUM62 is used for the substitution matrix. For nucleotide sequences, EDNAFULL is used. Other matrices can be specified. For purpose of the disclosure, No brief is yes and the parameters used for alignment of amino acid sequences are a gap-open penalty of 10 and a gap extension penalty of 0.5. The skilled person will appreciate that all these different parameters will yield slightly different results but that the overall percentage identity of two sequences is not significantly altered when using different algorithms. The identity defined as herein may be obtained from NEEDLE by using the NOBRIEF option and is labelled in the output of the program as "longest-identity". For purposes of the disclosure the level of identity (homology) between two sequences (amino acid or nucleotide) may be calculated according to the definition of "longest-identity" as can be carried out by using the program NEEDLE.

The protein sequences mentioned herein can further be used as a "query sequence" to perform a search against sequence databases, for example to identify other family members or related sequences. Such searches can be performed using the BLAST programs. Software for performing BLAST analyses is publicly available through the National Center for Biotechnology Information (http://www.ncbi.nlm.nih.gov). BLASTP is used for amino acid sequences and BLASTN for nucleotide sequences. In the BLAST program, the default settings may be used:
- Cost to open gap: default = 5 for nucleotides/ 11 for proteins
- Cost to extend gap: default = 2 for nucleotides/ 1 for proteins
- Penalty for nucleotide mismatch: default = -3
- Reward for nucleotide match: default = 1
- Expect value: default = 10
- Wordsize: default = 11 for nucleotides/ 28 for megablast/ 3 for proteins

The nucleic acid sequences as mentioned herein can further be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, word-length = 12 to obtain nucleotide sequences homologous to the nucleic acid molecules of the disclosure.

The sequence information as provided herein should not be so narrowly construed as to require inclusion of erroneously identified bases. The specific sequences disclosed herein can be readily used to isolate the complete gene from filamentous fungi, in particular *A. niger* which in turn can easily be subjected to further sequence analyses thereby identifying sequencing errors.

Unless otherwise indicated, all nucleotide sequences determined by sequencing a DNA molecule herein were determined using an automated DNA sequencer and all amino acid sequences of polypeptides encoded by DNA molecules determined herein were predicted by translation of a nucleic acid sequence determined as above. Therefore, as is known in the art for any DNA sequence determined by this automated approach, any nucleotide sequence determined herein may contain some errors. Nucleotide sequences determined by automation are typically at least about 90% identical, more typically at least about 95% to at least about 99.9% identical to the actual nucleotide sequence of the sequenced DNA molecule. The actual sequence can be more precisely determined by other approaches including manual DNA sequencing methods well known in the art. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion.

The person skilled in the art is capable of identifying such erroneously identified bases and knows how to correct for such errors.

A reference herein to a patent document or other matter which is given as prior art is not to be taken as an admission that that document or matter was known or that the information it contains was part of the common general knowledge as at the priority date of any of the claims.

The invention has the following embodiments:
1. A method for the production of a polypeptide of interest in a host cell, which method comprises:
   a.providing a host cell which harbours a nucleic acid encoding a polypeptide of interest, wherein the polypeptide of interest is modified so that it comprises fewer methionine and/or lysine residues than a reference polypeptide, excluding any initial methionine amino acid located at the N-terminal end of the polypeptide sequence, wherein methionine and/or lysine amino acids in the reference polypeptide have been substituted with non-methionine and/or non-lysine amino acids and wherein modification occurs in the mature polypeptide backbone;
   b.cultivating the host cell under conditions suitable for production of the polypeptide; and, optionally,
   c. recovering the compound of interest,
   wherein the number of methionine and/or lysine amino acids in the polypeptide of interest is reduced by at least 25% in comparison with the reference polypeptide, wherein the host cell is a yeast cell or a filamentous fungus cell, wherein the reference polypeptide is a polypeptide the expression of which is desired to increase in the host cell, wherein the substrate specificity or binding specificity of the modified polypeptide is maintained or its specific activity is the same as before improvement.
2. A method according to embodiment 1, where the reference polypeptide is a corresponding wild-type polypeptide.
3. A method according embodiment 1 or 2, wherein the reference polypeptide is a polypeptide sequence having at least 70% identity with one or more corresponding wild-type polypeptides.
4. A method according to any one of the preceding embodiments, wherein the number of methionine and/or lysine amino acids in the reference polypeptide is determined as the average number of methionine and/or lysine amino acids in two or more reference polypeptides.
5. A method according to any one of the preceding embodiments, wherein the reduction in methionine and/or lysine amino acids in the polypeptide of interest is determined over a length of at least 80 or more contiguous amino acids.
6. A method according to any one of the preceding embodiments, wherein the reduction of methionine and/or lysine amino acids in the polypeptide of interest is determined without taking into account signal sequences that may be processed in the host cell.
7. A method according to any one of the preceding embodiments, wherein the polypeptide of interest is a secreted protein.
8. A method for improving the expression level of a polypeptide in a host cell, which method comprises reducing the number of methionine and/or lysine amino acids in the polypeptide as compared with a reference polypeptide, excluding any initial methionine amino acid located at the N-terminal end of the polypeptide sequence, wherein the number of methionine and/or lysine amino acids in the amino acid backbone of the polypeptide is reduced by at least 25%, wherein methionine and/or lysine amino acids in the reference polypeptide have been substituted with non-methionine and/or non-lysine amino acids and wherein modification occurs in the mature polypeptide backbone, wherein the host cell is a yeast cell or a filamentous fungus cell, wherein the reference polypeptide is a polypeptide the expression of which is desired to increase in the host cell, wherein the substrate specificity or binding specificity of the modified polypeptide is maintained or its specific activity is the same as before improvement.
9. The method of embodiment 8 wherein the reference polypeptide is a corresponding wild-type polypeptide.
10. The method of embodiment 8 wherein the reference polypeptide is a polypeptide sequence having at least 70% identity with one or more corresponding wild-type polypeptides.
11. The method of embodiment 8 wherein the number of methionine and/or lysine amino acids in the reference polypeptide is determined as the average number of methionine and/or lysine amino acids in two or more reference polypeptides.
12. The method of embodiment 8 wherein the reduction in methionine and/or lysine amino acids in polypeptide of interest is determined over a length of at least 80 or more contiguous amino acids.
13. The method of embodiment 8 wherein the number of methionine and/or lysine amino acids is reduced by at least 30% in comparison with the reference polypeptide.
14. The method of embodiment 8 wherein the reduction of methionine and/or lysine amino acids in the modified polypeptide is determined without taking into account signal sequences that may be processed in the host cell.

### Examples

### Strains

### A. niger strains:

WT 1: This *A. niger* strain is used as a wild-type strain. This strain was deposited on 10 August 1988 at the Centraalbureau voor Schimmelcultures, Utrecht, The Netherlands.
WT 2: This *A. niger* strain is a WT 1 strain comprising a deletion of the gene encoding glucoamylase (*gla*A). WT 2 was constructed by using the "MARKER-GENE FREE" approach as described in EP 0 635 574 B1. In this patent it is extensively described how to delete *gla*A specific DNA sequences in the genome of CBS 513.88. The procedure resulted in a MARKER-GENE FREE Δ*gla*A recombinant *A. niger* CBS 513.88 strain, possessing finally no foreign DNA sequences at all.
WT 3: To disrupt the *pep*A gene encoding the major extracellular aspartic protease PepA in WT 2, *pep*A specific DNA sequences in the genome of WT 2 were deleted, as described by van den Hombergh et al. (van den Hombergh JP, Sollewijn Gelpke MD, van de Vondervoort PJ, Buxton FP, Visser J. (1997) - Disruption of three acid proteases in Aspergillus niger--effects on protease spectrum, intracellular proteolysis, and degradation of target proteins - Eur J Biochem. 247(2): 605-13). The procedure resulted in a MARKER-GENE FREE WT 3 strain, with the *pep*A gene inactivated in the WT 2 strain background.
WT 4: To delete the *hdf*A gene in WT 3, the method as earlier described in detail in WO05/095624 was used to generate *Aspergillus niger* WT 4 (Δ*gla*A, Δ*pep*A, Δ*hdf*A)*.*
WT 5: This *A. niger* strain is a WT 4 strain comprising a deletion which results in an oxalate deficient *A. niger* strain. WT 5 was constructed by using the method as described in EP1157100 and US6,936,438, in which an oxalate deficient strain was obtained by deletion of the *oah*A gene, encoding oxaloacetate hydrolase, Strain WT 5 was selected as a representative strain with the oahA gene inactivated in the WT 4 strain background.
WT 6: This *A. niger* strain is a WT 5 strain comprising the deletion of three genes encoding alpha-amylases (*amy*B, *amy*BI and *amy*BII) in three subsequent steps. The construction of deletion vectors and genomic deletion of these three genes has been described in detail in WO2005095624. The vectors pDEL-AMYA, pDEL-AMYBI and pDEL-AMYBII, described in WO2005095624, have been used according the "MARKER-GENE FREE" approach as described in EP 0 635 574 B1. The procedure described above resulted in WT 6, an oxalate deficient, MARKER-GENE FREE Δ*gla*A, Δ*pep*A, Δ*hdf*A, Δ*amy*A, Δ*amy*BI and Δ*amy*BII amylase-negative recombinant *A. niger* CBS 513.88 strain, possessing finally no foreign DNA sequences at all. As such, strain WT 6 has a low amylase background, has a higher HR/NHR ratio for more efficient targeting of sequences and is more optimized for extracellular protein expression and detection compared to WT 1.

### Fungal alpha-amylase activity assay (Example 2)

To determine the alpha-amylase activity in A. niger culture broth, the Megazyme cereal alpha-amylase kit is used (Megazyme, CERALPHA alpha amylase assay kit, catalogue, ref. K- CERA, year 2000-2001), according protocol of the supplier. The measured activity is based on hydrolysis of non-reducing-end blocked p-nitrophenyl maltoheptaoside in the presence of excess glucoamylase and [alpha]-glucosidase. The amount of formed p-nitrophenol is a measure for alpha-amylase activity present in a sample.

### Fungal alpha-amylase activity assay (Example 3)

The assay to determine the alpha-amylase activity in A. niger culture broth was based on the Megazyme cereal alpha-amylase kit (Megazyme, CERALPHA alpha amylase assay kit, catalogue, ref. K- CERA, year 2000-2001),with some minor modifications to make it suitable for running on an auto-analyzer (Konelab Arena 30). The assay is executed at pH 5.20 taking into account the pH optima for α-glucosidase and amyloglucosidase (pH range 5 - 6). The measured activity is based on hydrolysis of non-reducing-end blocked p-nitrophenyl maltoheptaoside in the presence of excess glucoamylase and [alpha]-glucosidase. The amount of formed p-nitrophenol is a measure for alpha-amylase activity present in a sample. The change in absorption per minute of incubation in the linear part of the time curve is used as a measure for the activity of the enzyme.

### Cellobiohydrolase activity assay

25 µL of enzyme solution was incubated with 10 mM para-nitrophenyl-β-D-cellobioside and 10 mM gluconolactone in 0.2 M sodium acetate buffer of pH 4.5 at 40°C. Samples were taken at time intervals of 10 min up to 30 min. The reaction was stopped by addition of cold 1M sodium carbonate solution at a ratio of 1:1. The absorbance of the final solutions is measured at 405 nm. The change in absorption per minute of incubation in the linear part of the time curve is used as a measure for the activity of the enzyme.

### LC-90

The LabChip 90 system (Caliper) was used to perform 1D electrophoretic separation of supernatant (protein) samples. Sample preparation was performed according to supplier's manual (HT Protein express reagent kit Catno. 760328) using DTT as a denaturing agent. HT protein express LabChip (Catno. 760499) that was prepared according to supplier's manual was used in combination with the LabChip HT protein express 200 software program, for sizing of protein ranging from 14kDa to 200 kDa, to analyze the samples.

### SDS- PAGE electrophoresis (Example 2)

*Sample pre-treatment:* 30µl sample was added to 35µl water and 25 µl NuPAGE™ LDS sample buffer (4x) Invitrogen and 10 µl NuPAGE™ Sample Reducing agent (10x) Invitrogen. Samples were heated for ten minutes at 70°C in a thermo mixer.

SDS-PAGE was performed in duplicate according to the supplier's instructions (Invitrogen: Gel: 4-12% Bis-Tris gel, Buffer: MES SDS running buffer, Runtime: 35 minutes). One of the two gels was used for blotting, 10 µl of the sample solutions and 1 µl marker M12 (Invitrogen) were applied on the gels (NuPAGE™ BisTris, Invitrogen).

The gels were run at 200V, using the XCELL Surelock, with 600 ml 20 times diluted MES-SDS buffer in the outer buffer chamber and 200ml 20 times diluted MES-SDS buffer, containing 0.5 ml of antioxidant (NuPAGE™ Invitrogen) in the inner buffer chamber. After running, the gels were fixed for one hour with 50% Methanol/ 7% Acetic acid (50ml), rinsed twice with demineralised water and stained with Sypro Ruby (50ml, Invitrogen) overnight.

Images were made using the Typhoon 9200 (610 BP 30, Green (532 nm), PMT 600V, 100 micron) after washing the gel for ten minutes with demineralised water.

### Quantitative SDS-PAGE (Example 3)

To determine the concentration of the specific protein of interest in the presence of one or more other proteins with a different molecular weight on SDS-PAGE, a technique called quantitative SDS-PAGE (qPAGE) was used. For qPAGE a Bovine Serum Albumin protein standard from Sigma-Aldrich (P0914, 1 mg/ml) was used to create a calibration curve. The five calibration BSA samples ranged from 0.02 up to 0.10 mg/ml. The BSA calibration samples were applied together with the fermentation samples containing the protein of interest for which the protein concentration should be determined. From the calibration BSA standard samples, 65 µl of was added to 25 µl NuPAGE™ LDS sample buffer (4x) Invitrogen and 10 µl NuPAGE™ Sample Reducing Agent (10x) Invitrogen. The mixtures were heated in a thermomixer for 10 minutes at 70°C.

Every fermentation sample of the protein of interest was diluted in order to fit the calibration curve generated with the BSA standard samples. After this dilution, 65 µl of the diluted sample was added to 25 µl NuPAGE™ LDS sample buffer (4x) Invitrogen and 10 µl NuPAGE™ Sample Reducing Agent (10x) Invitrogen. The mixtures were heated in a thermomixer for 10 minutes at 70°C. Subsequently, 5 µl of the sample solutions; 5 ul of each of the BSA standards and 3 µl marker M12 (Invitrogen) were applied on the 17 wells gels (4-12% Bis-Tris NuPAGE™, Invitrogen). The gels were run for 55 minutes at 200V using the XCELL Surelock, with 600 ml 20x diluted MOPS SDS buffer in the outer buffer chamber and 200 ml 20x diluted SDS buffer, containing 0.5 ml of anti-oxidant (NuPAGE™ Invitrogen) in the inner buffer chamber. After running, the gels were fixed for one hour with 50% Methanol / 7% Acetic acid (50ml), rinsed twice with demineralized water and stained with Sypro Ruby (50 ml, Invitrogen) overnight. An image was made using the Typhoon 9200 (Amersham/GE-Healthcare; 610 BP 30, Green (532 nm), PMT 530V, 100 micron) after washing the gels with demineralized water.

Using the Typhoon with the program Image Quant Tools version 2003.02 (Amersham/GE-Healthcare), grey values were measured for the BSA and the band(s) corresponding to the protein of interest. Calibration lines were made with the five BSA dilutions (0.02, 0.04, 0.06, 0.08 and 0.10 mg/ml) and from these calibration lines the protein concentrations of the protein of interest (on the same gel) were calculated. It should be noted that a calibration curve was accepted if 1) it was constituted of at least 4 standards, 2) if for each standard, the bias with respect to the nominal concentration was within +/- 15.0%. Only the (diluted) sample concentrations that felt between the lowest and the highest standards of an accepted calibration curve were calculated.

### Molecular biology techniques

In the examples herein, using molecular biology techniques known to the skilled person (see: Sambrook & Russell, Molecular Cloning: A Laboratory Manual, 3rd Ed., CSHL Press, Cold Spring Harbor, NY, 2001), several genes were over expressed and others were down regulated as described below.

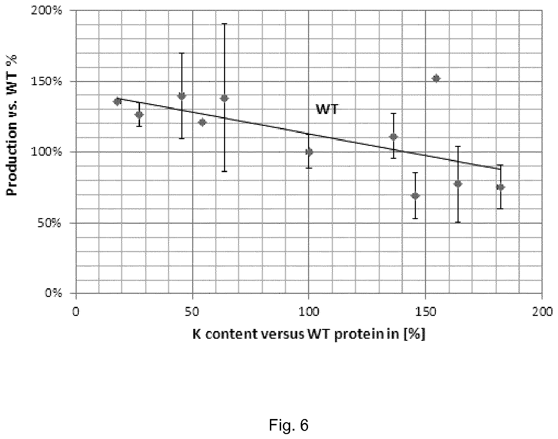

All gene replacement vectors described and used, were designed according to known principles and constructed according to routine cloning procedures. In essence, these vectors comprise approximately 1 - 2 kb flanking regions of the respective ORF sequences, to target for homologous recombination at the predestined genomic loci. In addition, they contain the *A. nidulans* bi-directional *amd*S selection marker for transformation, in-between direct repeats. The method applied for gene deletion in all examples herein uses linear DNA, which integrates into the genome at the homologous locus of the flanking sequences by a double cross-over, thus substituting the gene to be deleted by the *amd*S gene. After transformation, the direct repeats allow for the removal of the selection marker by a (second) homologous recombination event. The removal of the *amd*S marker can be done by plating on fluoro-acetamide media, resulting in the selection of marker-gene-free strains. Using this strategy of transformation and subsequent counter-selection, which is also described as the "MARKER-GENE FREE" approach in EP 0 635 574, the *amd*S marker can be used indefinitely in strain modification programs. The general procedure for gene disruption is depicted in Figure 6 of WO2006040312. The general design of deletion vectors was previously described in EP635574B and WO 98/46772 and the use of general cloning vector pGBDEL for constructing deletion vectors and the counter-selection procedure were a.o. described in WO06/040312.

Examples of the general design of expression vectors and specifically pGBFIN-expression vectors for gene over expression, transformation, use of markers and selective media can be found in WO199846772, WO199932617, WO2001121779, WO2005095624, EP 635574B and WO2005100573.

### Shake flask fermentations (Example 2)

*A. niger* strains were pre-cultured in 20 ml CSL pre-culture medium (100 ml flask, baffle) as described in the Examples: "Aspergillus niger shake flask fermentations" section of WO 99/32617. After growth for 18-24 hours at 34°C and 170 rpm, 10 ml of this culture is transferred to Fermentation Medium (FM). Fermentation in FM is performed in 500 ml flasks with baffle with 100 ml fermentation broth at 34°C and 170 rpm for the number of days indicated, generally as described in WO99/32617.
The CSL medium consisted of (in amount per litre): 100 g Corn Steep Solids (Roquette), 1 g NaH₂PO₄* H₂0, 0.5 g MgSO₄*7H₂O, 10 g glucose*H₂O and 0.25 g Basildon (antifoam). The ingredients were dissolved in demi-water and the pH was adjusted to pH 5.8 with NaOH or H₂SO₄; 100 ml flasks with baffle and foam ball were filled with 20 ml fermentation medium and sterilized for 20 minutes at 120°C.

The fermentation medium (FM) consisted of (in amount per liter): 150 g maltose*H₂O, 60 g Soytone (peptone), 1 g NaH₂PO₄*H2O, 15 g MgSO₄*7H2O, 0.08 g Tween 80, 0.02 g Basildon (antifoam), 20 g MES, 1 g L-arginine. The ingredients were dissolved in demi-water and the pH was adjusted to pH 6.2 with NaOH or H₂SO₄; 500 ml flasks with baffle and foam ball were filled with 100 ml fermentation broth and sterilized for 20 minutes at 120°C.

### Shake flask fermentations (Example 3)

*A. niger* strains were pre-cultured in 20 ml START pre-culture medium (100 ml flask, baffle) as described in the Examples: "Aspergillus niger shake flask fermentations" section of WO 99/32617. After growth for 18-24 hours at 34°C and 170 rpm, 10 ml of this culture is transferred to Fermentation Medium (FM). Fermentation in FM is performed in 500 ml flasks with baffle with 100 ml fermentation broth at 34°C and 170 rpm for the number of days indicated, generally as described in WO99/32617.

The START medium consisted of (in amount per litre): 30 g maltose, 10 g pepton aus casein (Merck 2239), 5 g yeast extract, 0.5 g MgSO4*7H2O, 1 g KH2PO4, 30 mg ZnCI2, 20 mg CaCI2, 10 mg MnSO4*4H2O, 0.3 g FeSO4*7H2O, 3 g Tween-80. The ingredients were dissolved in demi-water and the pH was adjusted to pH 5.5 with H2SO4; 100 ml flasks with baffle and foam ball were filled with 20 ml fermentation medium and sterilized for 15 minutes at 110°C.

The fermentation medium (FM) consisted of (in amount per liter): 60 g Glucose.H2O, 10 g maltose, 1 g KH2PO4, 12.5 g Yeast extract (Difco), 25 g Pepton aus casein (Merck 2239), 2 g K2SO4, 0.5 g MgSO4.7H2O, 3 ml ZnCl2 (10 mg/ml), 8 g CaCI2, 0.9 ml MnSO4.1H2O (10 mg/ml), 0.3 ml FeSO4.7H2O (10 mg/ml), 48.8 g MES. The ingredients were dissolved in demi-water and pH was adjusted to pH 5.6 with 6N KOH / H2SO4; 500 ml flasks with baffle and foam ball were filled with 100 ml fermentation medium and sterilized for 15 minutes at 110oC. Pre-culturing was done in START medium

Alternatively for the production of the EBA205 cellobiohydrolases the *A.niger* strains were pre-cultured using CSL medium. The CSL medium consisted of (in amount per litre): 100 g Corn Steep Solids (Roquette), 1 g NaH₂PO₄* H₂0, 0.5 g MgSO₄*7H₂O, 10 g glucose*H₂O and 0.25 g Basildon (antifoam). The ingredients were dissolved in demi-water and the pH was adjusted to pH 5.8 with NaOH or H₂SO₄; 100 ml flasks with baffle and foam ball were filled with 20 ml fermentation medium and sterilized for 20 minutes at 120°C.

The fermentation was performed in CSM/MES medium. The CSM/MES medium consisted of (in amount per litre): 150g Maltose.H2O, 60 g Soytone, 1 g NaH2PO4.H2O, 15 g (NH4)2SO4.H2O, 1 g MgSO4.7H2O. 0.08 g Tween80, 0.02 g Basildon, 20 g MES, 1 g L-arginine. The ingredients were dissolved in demi-water and pH was adjusted to pH 6.2 with NaOH / H2SO4; 500 ml flasks with baffle and foam ball were filled with 100 ml fermentation medium and sterilized for 15 minutes at 110°C.

### Example 1: Relating sequence characteristics to high-level protein production in Aspergillus niger

Knowledge on factors influencing protein production could be employed to improve enzyme production rates in industrial settings. High production yields can be obtained for homologous gene expression, but yields are often limited for heterologous gene expression. To learn about sequence properties that might influence production rates, we applied sequence-based machine learning techniques to identify relevant protein sequence features. The composition of the protein sequence was found to be most predictive and interpretation revealed that, for both homologous and heterologous gene expression, the same features are important. Methionine (M) and Lysine (K) were found to have a negative contribution to high-level production.

### Data

Two protein data sets were experimentally tested for high-level production and secretion, one for homologous gene expression and one for heterologous gene expression. Binary success scores were obtained by over-expression of the gene, introduced randomly into the A. niger genome behind the strong constitutive glucoamylase promoter. After growing in a shake flask, the filtered broth was put on gel. A positive success score was given when a clear visible band was present, negative otherwise. Proteins in the heterologous data set originated from 14 different fungal donor organisms (see Table 1). The resulting data from both hom and het data sets is set out in Table 2.

**Table 1. The names and abbreviations of the 14 fungal donor organisms for which there are proteins in the heterologous data set (het)**

| abbreviation | organism |
|---|---|
| Apul | Aureobasidium pullulans |
| Ares | Amorphotheca resinae |
| Ccin | Coprinus cinereus |
| Cele | Cunninghamella elegans |
| Clau | Cryptococcus laurentii |
| Gpan | Geomyces pannorum |
| Gtra | Gloeophyllum trabeum |
| Ledo | Lentinula edodes |
| Lsco | Leucosporidium scottii |
| Opil | Ophiostoma piliferum |
| Pchr | Phanerochaete chrysosporium |
| Sthe | Sporotrichum thermophile |
| Tlan | Thermomyces (Humicola) lanuginosa |
| Tver | Trametes versicolor |

**Table S2. The total number of proteins and the number of successfully (pos) and unsuccessfully (neg) high-level produced proteins in both hom and het, and per organism in het**

| | hom | het | Organism | | | | | | | | | | | | | |
|---|---|---|---|---|---|---|---|---|---|---|---|---|---|---|---|---|
| | | | Apul | Ares | Ccin | Cele | Clau | Gpan | Gtra | Ledo | Lsco | Opil | Pchr | Sthe | Tlan | Tver |
| # proteins | 345 | 991 | 87 | 68 | 140 | 39 | 14 | 83 | 81 | 169 | 60 | 39 | 55 | 65 | 16 | 75 |
| % | 1.0 | 1.0 | 0.09 | 0.07 | 0.14 | 0.04 | 0.01 | 0.08 | 0.08 | 0.17 | 0.06 | 0.04 | 0.06 | 0.07 | 0.02 | 0.08 |
| # pos | 178 | 163 | 13 | 7 | 29 | 2 | 2 | 12 | 20 | 9 | 4 | 9 | 18 | 17 | 6 | 15 |
| % pos | 0.52 | 0.16 | 0.1 5 | 0.1 | 0.21 | 0.05 | 0.14 | 0.14 | 0.25 | 0.05 | 0.07 | 0.23 | 0.33 | 0.36 | 0.38 | 0.2 |
| # neg | 167 | 828 | 74 | 61 | 111 | 37 | 12 | 71 | 61 | 160 | 56 | 30 | 37 | 48 | 10 | 60 |
| % neg | 0.48 | 0.84 | 0.85 | 0.9 | 0.79 | 0.95 | 0.86 | 0.86 | 0.75 | 0.95 | 0.93 | 0.77 | 0.67 | 0.74 | 0.62 | 0.8 |

All proteins have a signal peptide (length > 10 amino acids) as predicted by SignalP 3.0 [Dyrløv Bendtsen J, Nielsen H, von Heijne G, Brunak S (2004) Improved prediction of signal peptides: SignalP 3.0. Journal of molecular biology 340: 783-795] and a total sequence length longer than 100 amino acids. Proteins containing an ER retention signal (Cterminal KDEL) and proteins predicted to be transmembrane by both TMHMM [Krogh A, Larsson B, Von Heijne G, Sonnhammer E (2001) Predicting transmembrane protein topology with a hidden markov model: application to complete genomes. Journal of molecular biology 305: 567-580] and Phobius [. K"all L, Krogh A, Sonnhammer E (2004) A combined transmembrane topology and signal peptide prediction method. Journal of molecular biology 338: 1027-1036] were filtered out of the data set.

To avoid biasing subsequent analyses, sequence redundancy was reduced using BLASTCLUST [Dondoshansky I (2002) Blastclust (NCBI Software Development Toolkit). NCBI, Bethesda, Md]. Two sequences were considered redundant when the aligned sequences shared > 40% identity over a length of minimal 90% for at least one of the sequences. From the obtained protein clusters, we selected a representative protein, with the shortest average distance to all other proteins in the cluster, and removed the remainder. If a cluster contained proteins with both positive and negative labels, one positive and one negative protein was selected. This resulted in data sets *hom* and *het* containing 345 proteins (187 positives, 167 negatives) and 991 proteins (163 positives, 828 negatives), respectively.

To train a classifier on *hom* en test it on het, a data set *hetₕₒₘ* was constructed that contains the het data set without proteins that share > 40% identity with any protein in *hom.* This data set contained 906 (128 positives, 778 negatives) proteins.

### Classification

A linear support vector machine (LIBSVM [Chang C, Lin C (2011) LIBSVM: a library for support vector machines. ACM Transactions on Intelligent Systems and Technology (TIST) 2: 27]) was used for classification [Ben-Hur A, Ong C, Sonnenburg S, Sch¨olkopf B, R"atsch G (2008) Support vector machines and kernels for computational biology. PLoS computational biology 4: e1000173]. Parameter C was optimized using a simple grid search. In case of multiple kernels, both C and the kernel weights were optimized using a covariance matrix adaptation evolutionary algorithm (CMA) [Hulsman M, Reinders M, de Ridder D (2009) Evolutionary optimization of kernel weights improves protein complex comembership prediction. IEEE/ACM Transactions on Computational Biology and Bioinformatics (TCBB) 6: 427-437; and Hansen N (2006) The CMA evolution strategy: a comparing review. Towards a new evolutionary computation: 75-102]. Classifier performance on a data set was obtained by running a double 10-fold cross-validation (CV) loop, in which C and the kernel weights were optimized in an inner CV-loop on the training set. As performance measure we used the area under the receiver-operator characteristic curve (auroc) [Fawcett T (2006) An introduction to ROC analysis. Pattern recognition letters 27: 861-874]. Classifier performance is defined as the average auroc over the CV-loops. When separate training and test sets are used, a classifier was trained on the first data set, optimizing C and kernel weights in a 10-fold CV-loop, and tested on the second data set, again using the auroc as performance measure.

### Results

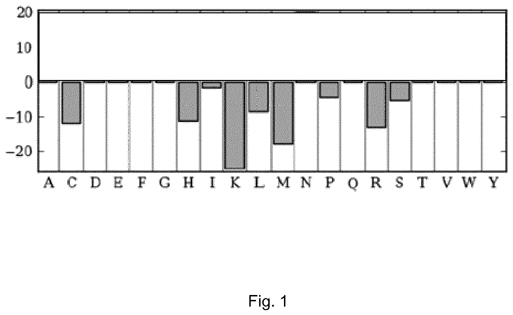

We exploited a dataset of proteins to predict successful high-level secretion, showing that the frequency of occurrence of a limited number amino acids are most predictive for high-yield secretion (see Fig. 1). Lysine and methione show the highest negative correlation.

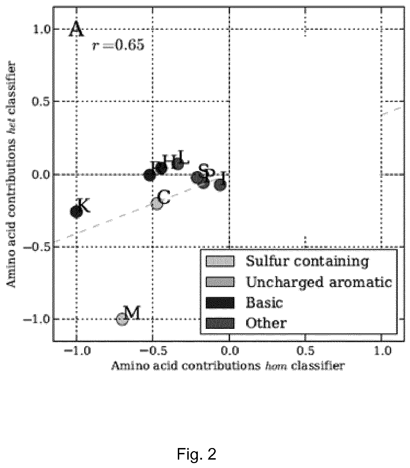

Fig. 2 shows the correlation between weights for both *het* and hom classifiers. The correlation shows that both classifiers are indeed similar. For both *hom* and *het,* a remarkable negative negative contribution of methionine (M) and lysine (K) was found. Considering amino acid properties, it was observed that the basic and the sulphur-containing amino acids have a negative contribution.

Prediction performance scores (AUROC) for amino acid composition were very good. Score 0.83 and 0.70 respectively for the mature protein AA composition, based on *hom* training and evaluation for *hom, het* training and evaluation on *het,* respectively.

Similar characteristics are important for both data sets, as derived from ROC-curves of the composition-based classifiers. Remarkably, similar weights resulted as the classifiers trained on *het,* suggesting that the homologous classifier generalizes well to predict high-level production for *het.* The good generalization suggests that classifiers trained on hom and het are similar, i.e. perform their predictions based on the same sequence characteristics.

### Example 2: Construction A. niger expression vectors for wild-type enzymes and enzyme variants according a method of the invention

In this example a number of expression vectors are constructed for variants of the enzymes of the invention. All variants for expression in *Aspergillus* are cloned in a pGBFIN-5 or a pGBTOP- expression vector. The construction, general layout and use of these vectors is described in detail in WO1999/32617.

### A. niger constructs

For expression of EBA205 and FUA (SEQ ID No. 5 and 7) in *A. niger,* the cDNA sequence was codon pair optimized using the method described in WO2008/000632 (SEQ ID No. 54 and 55) and is prepared synthetically (*e.g.* DNA2.0, USA, GeneArt, Germany).

The DNA sequence of the amyB gene encoding the alpha-amylase protein (FUA) was disclosed in J. Biochem. Mol. Biol. 37(4):429-438(2004) (Matsubara T., Ammar Y.B., Anindyawati T., Yamamoto S., Ito K., lizuka M., Minamiura N. "Molecular cloning and determination of the nucleotide sequence of raw starch digesting alpha-amylase from Aspergillus awamori KT-I I.") and also can be retrieved from EMBL Nucleotide Sequence Database (http://www.ebi.ac.uk/embl/index.html) under accession number AB083159. A. niger WT 6 is a strain where both the native 2 copies amyBI and amyBII were removed from the genome [An12g06930 and An05g02100] (*A. niger* genome sequence (EMBL: AM269948 - AM270415; Pel et al., "Genome sequencing and analysis of the versatile cell factory Aspergillus niger CBS 513.88"). Nat Biotechnol. 2007 Feb; 25 (2):221-231).

The *T. emersonii* EBA205, cellobiohydrolase 1 is identical to the one in Genbank [AAL33603]. In the case of FUA, the signal sequence for secretion (SEQ ID NO: 2) is replaced by an optimal signal sequence (SEQ ID NO: 3) (as detailed in WO2010121933).

For both proteins, cDNA sequences including the signal sequences were designed by codon-pair optimization directly from the amino acid sequence (as detailed in WO2008000632). The translational initiation sequence of the glucoamylase glaA promoter is modified into 5'-CACCGTCAAA ATG-3' in all expression constructs generated (as also detailed in WO2006/077258). In addition, an optimal translational termination sequence is used: 5'-TAAA-3' (as detailed in WO2006/077258) in all expression constructs.

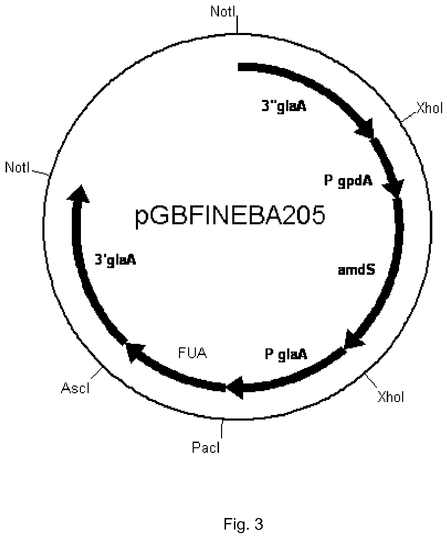

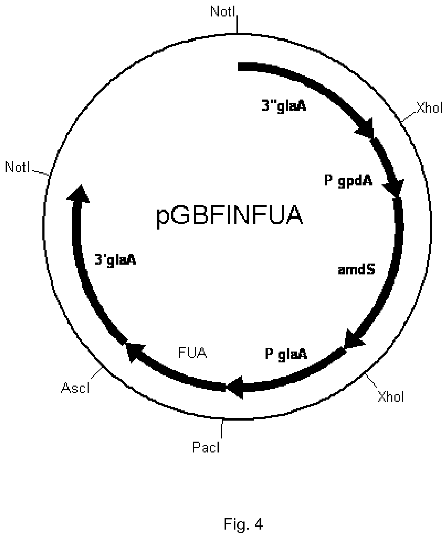

The *Talaromyces emersonii* EBA205 was codon pair optimized (as detailed in WO2008000632) and with all appropriate control elements is synthesized completely as *Pac*I *- Asc*I fragment, subcloned and sequence verified. The *Pac*I *- Asc*I restriction sites at the ends of the synthesized fragments are used to allow cloning in the large vector fragment of a *Pac*I - *Asc*I digested pGBFIN-5 expression vector, generating an expression vector pGBFINEBA205 (see Fig. 3). In addition and in a similar way as for the EBA205, an optimized FUA construct was codon pair optimized (as detailed in WO2008000632) and with all appropriate control elements is cloned as *Pac*I - *Asc*I fragment in pGBFIN-5, generating pGBFINFUA (Fig. 4).

Protein sequence optimization (PSO) is applied to the FUA, *A. niger* fungal amylase protein sequence [An12g06930; An05g02100], and the *T. emersonii* EBA205, cellobiohydrolase 1 [Genbank: AAL33603]. All proteins designed are obtained via gene-synthesis providers (*e.g.* DNA2.0, USA, GeneArt, Germany). The obtained fragments are cloned in a pGBFIN vector using the method essentially as described above, generating various pGBFIN- expression constructs. All relevant protein modification details for *A. niger* FUA and EBA205 constructs can be found in Tables 3 - 10. The complete overview with decrease/increase in methionine and lysine content for the mature protein is given in Table 11 and 12.

The experimental design is set-up in such a way that either M-residues, or K-residues, or both M and K residues are replaced in order to study the generic effect of reducing the compositional percentage of these amino-acids. For providing additional evidence on the specific effect of M or K, additional constructs with increased M or K content are used.

**Table 3: EBA205: decreased M content**

| | | | | | | | | | | |
|---|---|---|---|---|---|---|---|---|---|---|
| gene | 164 | 228 | 252 | 283 | 374 | 378 | 382 | 386 | 396 | M |
| EBA205 | M | M | M | M | M | M | M | M | M | reduction |
| mature | 146 | 210 | 234 | 265 | 356 | 360 | 364 | 368 | 378 | |
| substitute | L | L | R | L | | L | H | L | A | % |
| EBA205-M01 | | | R | L | | | | L | | 33% |
| EBA205-M02 | | | R | | I | L | | L | | 44% |
| EBA205-M03 | | | R | L | I | L | | | | 44% |
| EBA205-M04 | | | R | L | I | L | | L | | 56% |
| EBA205-M05 | | L | R | L | V | L | | L | | 67% |

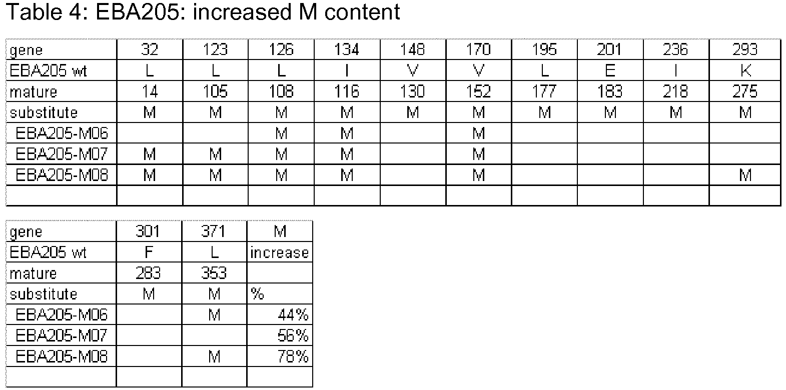

**Table 5: EBA205: decreased K content**

| | | | | | | | | | | | | |
|---|---|---|---|---|---|---|---|---|---|---|---|---|
| gene | 136 | 172 | 177 | 181 | 196 | 293 | 322 | 330 | 356 | 373 | 444 | |
| EBA205 | K | K | K | K | K | K | K | K | K | K | K | % |
| mature | 118 | 154 | 159 | 163 | 178 | 275 | 304 | 312 | 338 | 355 | 426 | |
| substitute | N | R | T | Q | | L | R | N | | G | R | |
| EBA205-K01 | N | | | | | | R | N | | G | | 36% |
| EBA205-K02 | N | R | | | | | R | N | | G | R | 55% |
| EBA205-K03 | N | | | | | L | R | N | | G | | 45% |
| EBA205-K04 | N | R | T | Q | | | R | N | | G | R | 73% |
| EEBA205-K05 | N | R | T | Q | | L | R | N | | G | R | 82% |

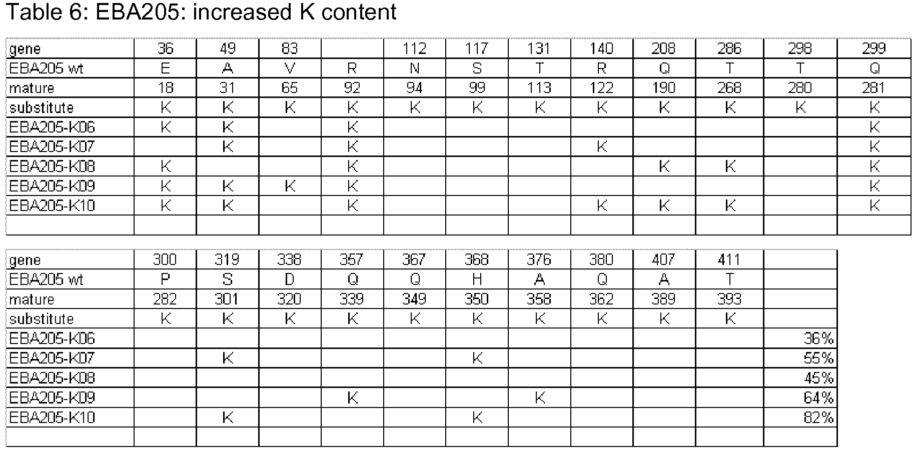

**Table 7: FUA: decreased M content**

| | | | | | | | | | | |
|---|---|---|---|---|---|---|---|---|---|---|
| gene | 75 | 132 | 135 | 143 | 266 | 289 | 295 | 416 | 475 | |
| wt | M | M | M | M | M | M | M | M | M | reduction |
| mature | 55 | 112 | 115 | 123 | 246 | 269 | 275 | 396 | 455 | M |
| substitute | L | I | L | F | L | I | E | L | L | % |
| FUA-M01 | | | | | x | | | x | x | 33% |
| FUA-M02 | | | | | x | x | | | x | 33% |
| FUA-M03 | | | | | x | | x | x | | 33% |
| FUA-M04 | | | | | x | x | x | x | x | 56% |
| FUA-M05 | | | | x | | x | x | x | x | 56% |
| FUA-M06 | x | | | x | x | | | x | x | 56% |
| FUA-M07 | x | | | x | x | x | x | | | 56% |
| FUA-M08 | x | x | x | x | x | x | x | x | x | 100% |

**Table 8: FUA: increased M content**

| gene | 180 | 206 | 214 | 247 | 274 | 299 | 313 | 328 | 455 | |
|---|---|---|---|---|---|---|---|---|---|---|
| wt | Q | E | L | C | I | V | V | I | T | increase |
| mature | 160 | 186 | 194 | 227 | 254 | 279 | 293 | 308 | 435 | M |
| substitute | M | M | M | M | M | M | M | M | M | % |
| FUA-M09 | | | | | x | x | | x | | 33% |
| FUA-M10 | | x | | | | | x | | x | 33% |
| FUA-M11 | | x | | | x | x | x | x | x | 67% |
| FUA-M12 | x | x | x | x | x | x | x | x | x | 100% |

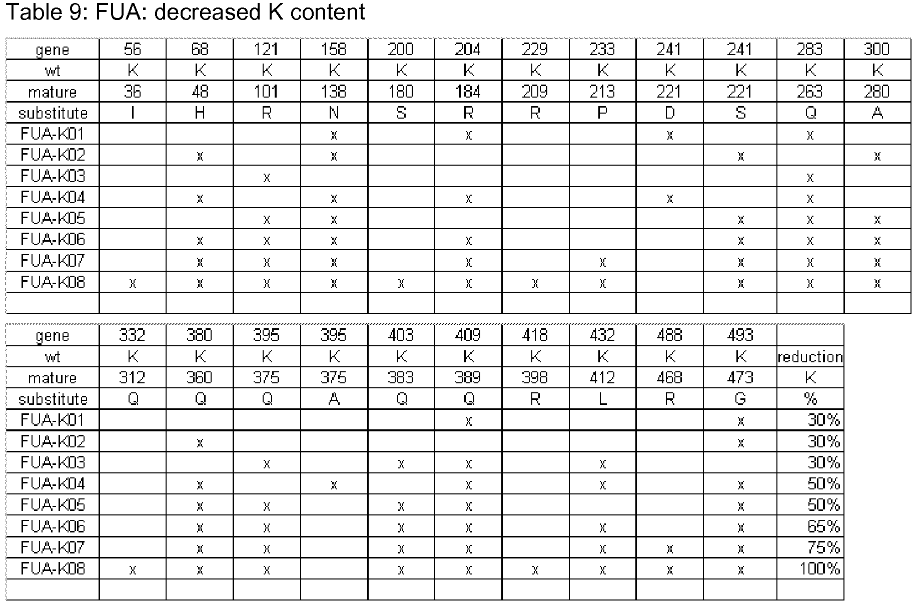

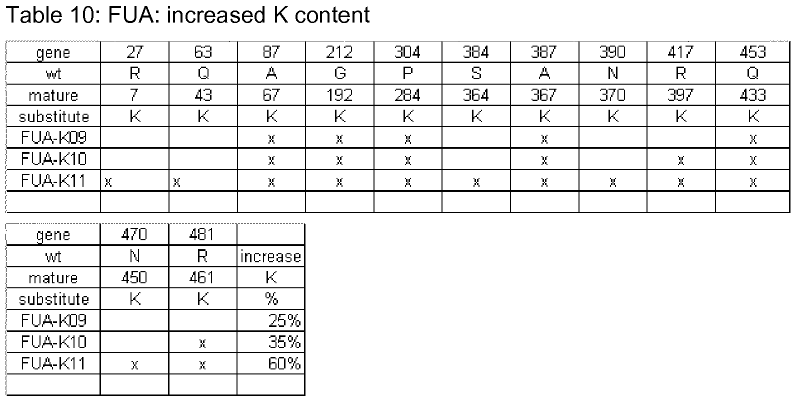

**Table 11: Overview EBA-205 experiment (21 constructs)**

| Protein | M- | M+ | K- | K+ | KM- |
|---|---|---|---|---|---|
| E BA-205 | | | | | |
| EBA205-M01 | 33% | | | | |
| EBA205-M02 | 44% | | | | |
| EBA205-M03 | 44% | | | | |
| EBA205-M04 | 56% | | | | |
| EBA205-M05 | 67% | | | | |
| EBA205-M06 | | 44% | | | |
| EBA205-M07 | | 56% | | | |
| EBA205-M08 | | 78% | | | |
| EBA205-K01 | | | 33% | | |
| EBA205-K02 | | | 44% | | |
| EBA205-K03 | | | 44% | | |
| EBA205-K04 | | | 56% | | |
| EBA205-K05 | | | 67 % | | |
| EBA205-K06 | | | | 36% | |
| EBA205-K07 | | | | 55% | |
| EBA205-K08 | | | | 45% | |
| EBA205-K09 | | | | 64% | |
| EBA205-K1 0 | | | | 82% | |
| EBA205-KM01 | 33% | | 45% | | 35% |
| EBA205-KM02 | 44% | | 64% | | 60% |

**Table 12: Overview FUA experiment (26 constructs)**

| Protein | M- | M+ | K- | K+ | KM- |
|---|---|---|---|---|---|
| FUA | | | | | |
| FUA-M01 | 33% | | | | |
| FUA-M02 | 33% | | | | |
| FUA-M03 | 33% | | | | |
| FUA-M04 | 56% | | | | |
| FUA-M05 | 56% | | | | |
| FUA-M06 | 56% | | | | |
| FUA-M07 | 56% | | | | |
| FUA-M08 | 100% | | | | |
| FUA-M09 | | 33% | | | |
| FUA-M10 | | 33% | | | |
| FUA-M11 | | 67% | | | |
| FUA-M12 | | 100% | | | |
| FUA-K01 | | | 30% | | |
| FUA-K02 | | | 30% | | |
| FUA-K03 | | | 30% | | |
| FUA-K04 | | | 50% | | |
| FUA-K05 | | | 50% | | |
| FUA-K06 | | | 65% | | |
| FUA-K07 | | | 75% | | |
| FUA-K03 | | | 100% | | |
| FUA-K09 | | | | 25% | |
| FUA-K10 | | | | 35% | |
| FUA-K11 | | | | 60% | |
| FUA-KM01 | 33% | | 56% | | 31% |
| FUA-KM02 | 30% | | 60% | | 62% |

### Expression of wild-type and PSO optimized fungal enzymes in A. niger

The pGBFINFUA- and pGBFINEBA205-expression constructs are introduced by transformation using *A. niger.* In order to introduce the different pGBFINFUA- and pGBFINEBA205- vectors (see Tables 3 to 10) in WT 6, a transformation and subsequent selection of transformants is carried out as described in WO1998/46772 and WO1999/32617. In brief, linear DNA of all the pGBFIN- constructs was isolated and used to transform *A. niger* WT 6. Transformants were selected on acetamide media and colony purified according standard procedures. Colonies were diagnosed for integration at the glaA locus and for copy number using PCR. Three independent transformants for each pGBFINFUA- and pGBFINEBA205-construct with similar estimated copy numbers (putative single copy) were selected and named using the number of the transforming plasmid, as for example FUA-M01-1, FUA-M01-2, FUA-M01-3, EBA205-M01-1, EBA205-M01-2, EBA205-M01-3, etc...., respectively.

### EBA-205 constructs and expression

Transformants of the *A. niger* EBA205- transformants containing the different constructs are picked, resulting about 80% successful overexpression strains, including the wild-type EA205 (wild-type protein). For the subset, the production of CBHI expressed by each of the transformants of the *A. niger* EBA205- transformants containing the different constructs, is measured in the culture supernatant at day 3. In addition, the culture supernatants sampled at day 4 are analyzed by SDS gel electrophoresis and staining.

Protein sequence optimization by reducing M, K or both M and K amino acid has a positive impact on protein secretion and results in increased protein expression levels and increased activity levels for the cellobiohydrolase I enzyme. A clear positive trend may be seen when plotting from left to right the percentage {increase - wild type - percentage decrease} of methionine versus expression level, and in a similar way for activity. The same effect may be seen for lysine.

### FUA constructs and expression

Transformants of the *A. niger* FUA- transformants containing the different constructs are picked, resulting about 80% successful overexpression strains, including the wild-type FUA (wild-type protein). For the subset, the production of alpha-amylase expressed by each of the transformants of the *A. niger* FUA- transformants containing the different constructs, is measured in the culture supernatant at day 3. In addition, the culture supernatants sampled at day 4 are analyzed by SDS gel electrophoresis and staining.

Protein sequence optimization by reducing M, K or both M and K amino acid has a positive impact on protein secretion and results in detectable and thus increased protein expression levels and increased activity levels for the alpha-amylase enzyme. A clear positive trend is observed when plotting from left to right the percentage {increase - wild type - percentage decrease of methionine versus expression level, and in a similar way for activity. The same effect may be seen for lysine.

### Example 3: Construction A. niger expression vectors for wild-type enzymes and enzyme variants according a method of the invention

In this example a number of expression vectors were constructed for variants of the enzymes of the invention. All variants for expression in *Aspergillus* were cloned in a pGBFIN-5 or a pGBTOP- expression vector. The construction, general layout and use of these vectors is described in detail in WO1999/32617.

### A. niger constructs

For expression of EBA205 and FUA (SEQ ID No. 5 and 7) in *A. niger,* the cDNA sequence was codon pair optimized using the method described in WO2008/000632 (SEQ ID No. 54 and 55) and is prepared synthetically (*e.g.* DNA2.0, USA, GeneArt, Germany).

The DNA sequence of the amyB gene encoding the alpha-amylase protein (FUA) was disclosed in J. Biochem. Mol. Biol. 37(4):429-438(2004) (Matsubara T., Ammar Y.B., Anindyawati T., Yamamoto S., Ito K., lizuka M., Minamiura N. "Molecular cloning and determination of the nucleotide sequence of raw starch digesting alpha-amylase from Aspergillus awamori KT-I I.") and also can be retrieved from EMBL Nucleotide Sequence Database (http://www.ebi.ac.uk/embl/index.html) under accession number AB083159. A. niger WT 6 is a strain where both the native 2 copies amyBI and amyBII were removed from the genome [An12g06930 and An05g02100] (*A. niger* genome sequence (EMBL: AM269948 - AM270415; Pel et al., "Genome sequencing and analysis of the versatile cell factory Aspergillus niger CBS 513.88"). Nat Biotechnol. 2007 Feb; 25 (2):221-231).

The *T. emersonii* EBA205, cellobiohydrolase 1 is identical to the one in Genbank [AAL33603]. In the case of FUA, the signal sequence for secretion (SEQ ID NO: 2) is replaced by an optimal signal sequence (SEQ ID NO: 3) (as detailed in WO2010121933).

For both proteins, cDNA sequences including the signal sequences were designed by codon-pair optimization directly from the amino acid sequence (as detailed in WO2008000632). The translational initiation sequence of the glucoamylase glaA promoter was modified into 5'-CACCGTCAAA ATG-3' in all expression constructs generated (as also detailed in WO2006/077258). In addition, an optimal translational termination sequence was used: 5'-TAAA-3' (as detailed in WO2006/077258) in all expression constructs.

The *Talaromyces emersonii* EBA205 was codon pair optimized (as detailed in WO2008000632) and with all appropriate control elements is synthesized completely as *Pac*I *- Asc*I fragment, subcloned and sequence verified. The *Pac*I *- Asc*I restriction sites at the ends of the synthesized fragments are used to allow cloning in the large vector fragment of a *Pac*I - *Asc*I digested pGBFIN-5 expression vector, generating an expression vector pGBFINEBA205 (see Fig. 3). In addition and in a similar way as for the EBA205, an optimized FUA construct was codon pair optimized (as detailed in WO2008000632) and with all appropriate control elements was cloned as *Pac*I - *Asc*I fragment in pGBFIN-5, generating pGBFINFUA (Fig. 4).

Protein sequence optimization (PSO) was applied to the FUA, *A. niger* fungal amylase protein sequence [An12g06930; An05g02100], and the *T. emersonii* EBA205, cellobiohydrolase 1 [Genbank: AAL33603]. All proteins designed were obtained via gene-synthesis providers (*e.g.* DNA2.0, USA, GeneArt, Germany). The obtained fragments were cloned in a pGBFIN vector using the method essentially as described above, generating various pGBFIN- expression constructs. All relevant protein modification details for *A. niger* FUA and EBA205 constructs can be found in Tables 3 - 10. The complete overview with decrease/increase in methionine and lysine content for the mature protein is given in Table 11 and 12.

The experimental design was set-up in such a way that either M-residues, or K-residues, or both M and K residues were replaced in order to study the generic effect of reducing the compositional percentage of these amino-acids. For providing additional evidence on the specific effect of M or K, additional constructs with increased M or K content were used.

### Expression of wild-type and PSO optimized fungal enzymes in A. niger

The pGBFINFUA- and pGBFINEBA205-expression constructs were introduced by transformation using *A. niger.* In order to introduce the different pGBFINFUA- and pGBFINEBA205- vectors (see Tables 3 to 10) in WT 6, a transformation and subsequent selection of transformants was carried out as described in WO1998/46772 and WO1999/32617. In brief, linear DNA of all the pGBFIN- constructs was isolated and used to transform *A. niger* WT 6. Transformants were selected on acetamide media and colony purified according standard procedures. Colonies were diagnosed for integration at the *gla*A locus and for copy number using PCR. Three independent transformants for each pGBFINFUA- and pGBFINEBA205-construct with similar estimated copy numbers (putative single copy) were selected and named using the number of the transforming plasmid, as for example FUA-M01-1, FUA-M01-2, FUA-M01-3, EBA205-M01-1, EBA205-M01-2, EBA205-M01-3, etc...., respectively.

### EBA-205 constructs and expression

Transformants of the *A. niger* EBA205- transformants containing the different constructs were picked, resulting about 80% successful overexpression strains, including the wild-type EA205 (wild-type protein). For the subset, the production of CBHI expressed by each of the transformants of the *A. niger* EBA205- transformants containing the different constructs, was measured in the culture supernatant at day 5. In addition, the culture supernatants sampled at day 5 were analyzed by quantitative SDS PAGE.

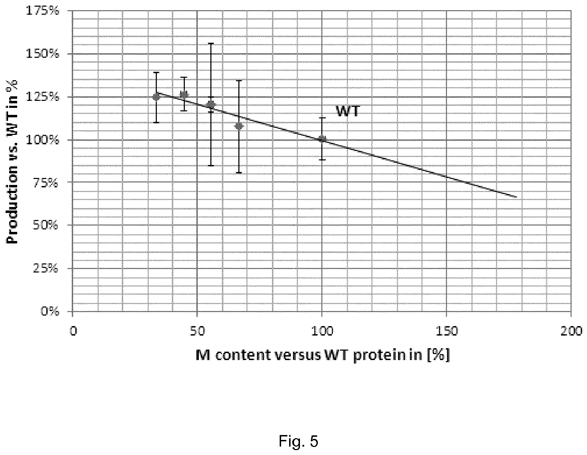

**Table 13. Methionine variants versus WT; expression level in % of WT; measurements italics left out corresponding to Figure 5.**

| **Strain** | **% WT** |
|---|---|
| EBA-205-1 | 92% |
| EBA-205-2 | 0% |
| EBA-205-3 | 108% |
| EBA-205-M01-1 | 130% |
| EBA-205-M01-2 | 78% |
| EBA-205-M01-3 | 114% |
| EBA-205-M02-1 | 117% |
| EBA-205-M02-2 | 123% |
| EBA-205-M02-3 | 0% |
| EBA-205-M03-1 | 145% |
| EBA-205-M03-2 | 136% |
| EBA-205-M03-3 | 79% |
| EBA-205-M04-1 | 129% |
| EBA-205-M04-2 | 115% |
| EBA-205-M04-3 | 134% |
| EBA-205-M05-1 | 108% |
| EBA-205-M05-2 | 128% |
| EBA-205-M05-3 | 136% |

**Table 14. Methionine variants versus WT; expression level in % of WT; measurements italics left out corresponding to Figure 6.**

| **Strain** | **% WT** |
|---|---|
| EBA-205-1 | 92% |
| EBA-205-2 | *0%* |
| EBA-205-3 | 108% |
| EBA-205-K01-1 | 108% |
| EBA-205-K01-2 | 198% |
| EBA-205-K01-3 | 107% |
| EBA-205-K02-1 | 108% |
| EBA-205-K02-2 | 168% |
| EBA-205-K02-3 | 142% |
| EBA-205-K03-1 | 121% |
| EBA-205-K03-2 | *0%* |
| EBA-205-K03-3 | *0%* |
| EBA-205-K04-1 | 664% |
| EBA-205-K04-2 | 132% |
| EBA-205-K04-3 | 120% |
| EBA-205-K05-1 | 135% |
| EBA-205-K05-2 | *59%* |
| EBA-205-K05-3 | *0%* |
| EBA-205-K06-1 | 120% |
| EBA-205-K06-2 | 93% |
| EBA-205-K06-3 | 120% |
| EBA-205-K07-1 | 150% |
| EBA-205-K07-2 | *983%* |
| EBA-205-K07-3 | 152% |
| EBA-205-K08-1 | 57% |
| EBA-205-K08-2 | 88% |
| EBA-205-K08-3 | 63% |
| EBA-205-K09-1 | 98% |
| EBA-205-K09-2 | 86% |
| EBA-205-K09-3 | 47% |
| EBA-205-K10-1 | 64% |
| EBA-205-K10-2 | 86% |
| EBA-205-K10-3 | *0%* |

**Table 15. Combined methionine and lysine variants versus WT; expression level in % of WT;**

| **Strain** | **% WT** |
|---|---|
| FUA-1 | 92% |
| FUA-2 | *0%* |
| FUA-3 | 108% |
| EBA205-KM01-1 | 86% |
| EBA205-KM01-2 | 136% |
| EBA205-KM01-3 | 101% |
| EBA205-KM02-1 | 150% |

From the tables and figures, it is clear that protein sequence optimization (PSO) by reducing M, K or both M and K amino acid has a positive impact on protein secretion and results in increased protein expression levels for the cellobiohydrolase I enzyme. Although not all constructs resulted in triplicate producing strains and expression data, still a clear correlation is observed when plotting from left to right the percentage {decrease - wild type - percentage increase} of methionine versus expression level.

A similar effect is shown for lysine reduction versus expression level.

### FUA constructs and expression

Transformants of the *A. niger* FUA- transformants containing the different constructs were picked, resulting about 80% successful overexpression strains, including the wild-type FUA (wild-type protein). For the subset, the production of alpha-amylase expressed by each of the transformants of the *A. niger* FUA- transformants containing the different constructs, was measured with the fungal alpha-amylase activity assay in the culture supernatant at day 4. The activity is a measure for expression level.

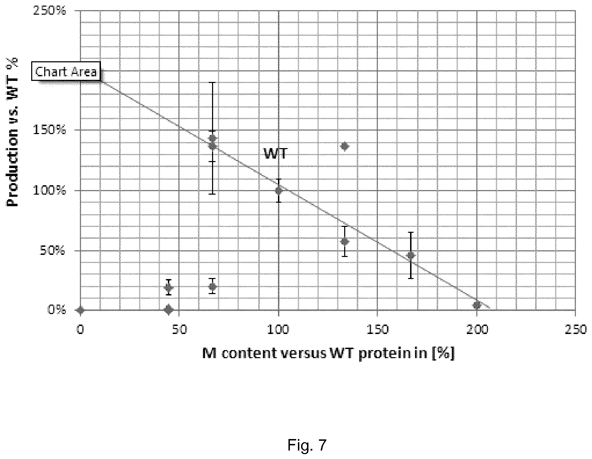

**Table 16. Methionine variants versus WT; activity level in % of WT; measurements italics left out corresponding Figure 7.**

| **Strain** | **% WT** |
|---|---|
| FUA-1 | 105% |
| FUA-2 | *89%* |
| FUA-3 | 107% |
| FUA-M01-1 | 111% |
| FUA-M01-2 | 124% |
| FUA-M01-3 | 196% |
| FUA-M02-1 | 151% |
| FUA-M02-2 | 135% |
| FUA-M02-3 | *126%* |
| FUA-M03-1 | 18% |
| FUA-M03-2 | 15% |
| FUA-M03-3 | 27% |
| FUA-M04-1 | 1% |
| FUA-M04-2 | 1% |
| FUA-M04-3 | 2% |
| FUA-M05-1 | 0% |
| FUA-M05-2 | 0% |
| FUA-M05-3 | 0% |
| FUA-M06-1 | 26% |
| FUA-M06-2 | 18% |
| FUA-M06-3 | 14% |
| FUA-M07-1 | 0% |
| FUA-M07-2 | 1% |
| FUA-M07-3 | 0% |
| FUA-M08-1 | 0% |
| FUA-M08-2 | 0% |
| FUA-M08-3 | 0% |
| FUA-M09-1 | 224% |
| FUA-M09-2 | 137% |
| FUA-M09-3 | *0%* |
| FUA-M10-1 | 57% |
| FUA-M10-2 | 45% |
| FUA-M10-3 | 70% |
| FUA-M11-1 | 35% |
| FUA-M11-2 | 69% |
| FUA-M11-3 | 35% |
| FUA-M12-1 | 4% |
| FUA-M12-2 | 5% |
| FUA-M12-3 | 5% |

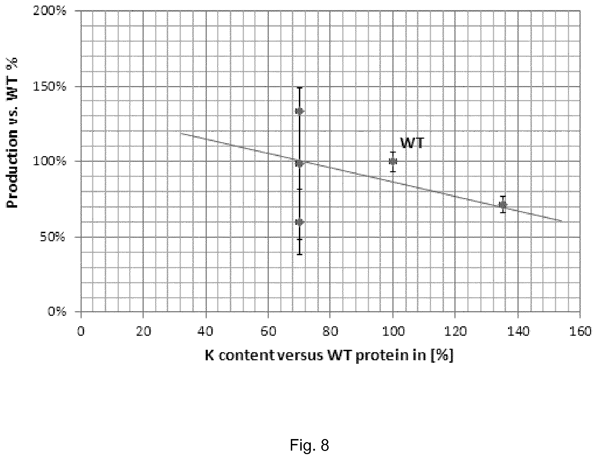

**Table 17. Methionine variants versus WT; activity level in % of WT; measurements italics left out corresponding Figure 8.**

| **Strain** | **% WT** |
|---|---|
| FUA-1 | 106% |
| FUA-2 | 101% |
| FUA-3 | 93% |
| FUA-K01-1 | 45% |
| FUA-K01-2 | 50% |
| FUA-K01-3 | 85% |
| FUA-K02-1 | *0%* |
| FUA-K02-2 | *0%* |
| FUA-K02-3 | 134% |
| FUA-K03-1 | 109% |
| FUA-K03-2 | 44% |
| FUA-K03-3 | 142% |
| FUA-K10-1 | 65% |
| FUA-K10-2 | 76% |
| FUA-K10-3 | 73% |

**Table 18. Combined methionine and lysine variants versus WT; activity level in % of WT;**

| **Strain** | **% WT** |
|---|---|
| FUA-1 | 105% |
| FUA-2 | *89%* |
| FUA-3 | 107% |
| FUA-KM01-1 | 0% |
| FUA-KM01-2 | 17% |
| FUA-KM01-3 | 18% |
| FUA-KM02-1 | 2% |
| FUA-KM02-2 | 11% |
| FUA-KM02-3 | 1% |

Note that some of the PSO engineered variants with reduced M-content show no or reduced activity levels. Typically those proteins likely are too much affected in their folding and no longer fold correctly nor express well. In addition, the culture supernatants sampled at day 4 were analyzed by LC90, which show a similar trend for expression versus no expression of picked strains, although is not quantitative for expression levels. Table 18 shows extremely engineered combined M/K variants, which have not survived the engineering approach and are no longer active.

For a majority of the shown FUA example, protein sequence optimization by reducing M or K K amino acid has a positive impact on protein secretion and results in detectable and thus increased protein expression levels and increased activity levels for the alpha-amylase enzyme. A clear positive trend is observed when plotting from left to right the percentage {decrease - wild type - percentage increase of methionine versus a activity level, and thus in a similar way for expression assuming that the specific activity is not substantially changed for those variants. For lysine also a clear correlation is observed, although less strains and thereby less data-points are available.

## Claims

1. A method for the production of a polypeptide of interest in a host cell, which method comprises:
a. providing a host cell which harbours a nucleic acid encoding a polypeptide of interest, wherein the polypeptide of interest is modified so that it comprises fewer methionine and/or lysine residues than a reference polypeptide, excluding any initial methionine amino acid located at the N-terminal end of the polypeptide sequence, wherein methionine and/or lysine amino acids in the reference polypeptide have been substituted with non-methionine and/or non-lysine amino acids and wherein modification occurs in the mature polypeptide backbone;
b. cultivating the host cell under conditions suitable for production of the polypeptide; and, optionally,
c. recovering the compound of interest,
wherein the number of methionine and/or lysine amino acids in the polypeptide of interest is reduced by at least 25% in comparison with the reference polypeptide, wherein the host cell is a yeast cell or a filamentous fungus cell, wherein the reference polypeptide is a polypeptide the expression of which is desired to increase in the host cell, wherein the substrate specificity or binding specificity of the modified polypeptide is maintained or its specific activity is the same as before improvement.

2. A method according to claim 1, where the reference polypeptide is a corresponding wild-type polypeptide.

3. A method according claim 1 or 2, wherein the reference polypeptide is a polypeptide sequence having at least 70% identity with one or more corresponding wild-type polypeptides.

4. A method according to any one of the preceding claims, wherein the number of methionine and/or lysine amino acids in the reference polypeptide is determined as the average number of methionine and/or lysine amino acids in two or more reference polypeptides.

5. A method according to any one of the preceding claims, wherein the reduction in methionine and/or lysine amino acids in the polypeptide of interest is determined over a length of at least 80 or more contiguous amino acids.

6. A method according to any one of the preceding claims, wherein the reduction of methionine and/or lysine amino acids in the polypeptide of interest is determined without taking into account signal sequences that may be processed in the host cell.

7. A method according to any one of the preceding claims, wherein the polypeptide of interest is a secreted protein.

8. A method for improving the expression level of a polypeptide in a host cell, which method comprises reducing the number of methionine and/or lysine amino acids in the polypeptide as compared with a reference polypeptide, excluding any initial methionine amino acid located at the N-terminal end of the polypeptide sequence, wherein the number of methionine and/or lysine amino acids in the amino acid backbone of the polypeptide is reduced by at least 25%, wherein methionine and/or lysine amino acids in the reference polypeptide have been substituted with non-methionine and/or non-lysine amino acids and wherein modification occurs in the mature polypeptide backbone, wherein the host cell is a yeast cell or a filamentous fungus cell, wherein the reference polypeptide is a polypeptide the expression of which is desired to increase in the host cell, wherein the substrate specificity or binding specificity of the modified polypeptide is maintained or its specific activity is the same as before improvement.

9. The method of claim 8 wherein the reference polypeptide is a corresponding wild-type polypeptide.

10. The method of claim 8 wherein the reference polypeptide is a polypeptide sequence having at least 70% identity with one or more corresponding wild-type polypeptides.

11. The method of claim 8 wherein the number of methionine and/or lysine amino acids in the reference polypeptide is determined as the average number of methionine and/or lysine amino acids in two or more reference polypeptides.

12. The method of claim 8 wherein the reduction in methionine and/or lysine amino acids in polypeptide of interest is determined over a length of at least 80 or more contiguous amino acids.

13. The method of claim 8 wherein the number of methionine and/or lysine amino acids is reduced by at least 30% in comparison with the reference polypeptide.

14. The method of claim 8 wherein the reduction of methionine and/or lysine amino acids in the modified polypeptide is determined without taking into account signal sequences that may be processed in the host cell.

## Patentansprüche

1. Verfahren zur Produktion eines interessierenden Polypeptids in einer Wirtszelle, wobei das Verfahren Folgendes umfasst:
a. Bereitstellen einer Wirtszelle, die eine ein interessierendes Polypeptid codierende Nukleinsäure enthält, wobei das interessierende Polypeptid modifiziert ist, so dass es weniger Methionin- und/oder Lysinreste als ein Referenzpolypeptid umfasst, unter Ausschluss jeglicher am N-terminalen Ende der Polypeptidsequenz liegenden ersten Methionin-Aminosäure, wobei Methionin- und/oder Lysin-Aminosäuren im Referenzpolypeptid durch Nicht-Methionin- und/oder Nicht-Lysin-Aminosäuren substituiert wurden und wobei die Modifikation im Grundgerüst des reifen Polypeptids auftritt;
b. Kultivieren der Wirtszelle unter für die Produktion des Polypeptids geeigneten Bedingungen; und gegebenenfalls
c. Gewinnen der interessierenden Verbindung, wobei die Anzahl an Methionin- und/oder Lysin-Aminosäuren im interessierenden Polypeptid im Vergleich mit dem Referenzpolypeptid um wenigstens 25% verringert ist, wobei es sich bei der Wirtszelle um eine Hefezelle oder eine Fadenpilzzelle handelt, wobei es sich bei dem Referenzpolypeptid um ein Polypeptid handelt, dessen Expression in der Wirtszelle wunschgemäß erhöht wird, wobei die Substratspezifität oder Bindungsspezifität des modifizierten Polypeptids beibehalten wird oder seine spezifische Aktivität die gleiche wie vor der Verbesserung ist.

2. Verfahren nach Anspruch 1, wobei es sich bei dem Referenzpolypeptid um ein entsprechendes Wildtyp-Polypeptid handelt.

3. Verfahren nach Anspruch 1 oder 2, wobei es sich bei dem Referenzpolypeptid um eine Polypeptidsequenz mit wenigstens 70% Identität mit einem oder mehreren entsprechenden Wildtyp-Polypeptiden handelt.

4. Verfahren nach einem der vorhergehenden Ansprüche, wobei die Anzahl an Methionin- und/oder Lysin-Aminosäuren im Referenzpolypeptid als die mittlere Anzahl an Methionin- und/oder Lysin-Aminosäuren in zwei oder mehr Referenzpolypeptiden bestimmt wird.

5. Verfahren nach einem der vorhergehenden Ansprüche, wobei die Verringerung von Methionin- und/oder Lysin-Aminosäuren im interessierenden Polypeptid über eine Länge von wenigstens 80 oder mehr zusammenhängenden Aminosäuren bestimmt wird.

6. Verfahren nach einem der vorhergehenden Ansprüche, wobei die Verringerung der Methionin- und/oder Lysin-Aminosäuren im interessierenden Polypeptid ohne Berücksichtigung von Signalsequenzen, die in der Wirtszelle prozessiert werden können, bestimmt wird.

7. Verfahren nach einem der vorhergehenden Ansprüche, wobei es sich bei dem interessierenden Polypeptid um ein sezerniertes Protein handelt.

8. Verfahren zur Verbesserung des Expressionsniveaus eines Polypeptids in einer Wirtszelle, wobei das Verfahren Verringern der Anzahl an Methionin- und/oder Lysin-Aminosäuren im Polypeptid im Vergleich zu einem Referenzpolypeptid, unter Ausschluss jeglicher am N-terminalen Ende der Polypeptidsequenz liegenden ersten Methionin-Aminosäure, umfasst, wobei die Anzahl an Methionin- und/oder Lysin-Aminosäuren im Aminosäuregrundgerüst des Polypeptids um wenigstens 25% verringert ist, wobei Methionin- und/oder Lysin-Aminosäuren im Referenzpolypeptid durch Nicht-Methionin- und/oder Nicht-Lysin-Aminosäuren substituiert wurden und wobei die Modifikation im Grundgerüst des reifen Polypeptids auftritt, wobei es sich bei der Wirtszelle um eine Hefezelle oder eine Fadenpilzzelle handelt, wobei es sich bei dem Referenzpolypeptid um ein Polypeptid handelt, dessen Expression in der Wirtszelle wunschgemäß erhöht wird, wobei die Substratspezifität oder Bindungsspezifität des modifizierten Polypeptids beibehalten wird oder seine spezifische Aktivität die gleiche wie vor der Verbesserung ist.

9. Verfahren nach Anspruch 8, wobei es sich bei dem Referenzpolypeptid um ein entsprechendes Wildtyp-Polypeptid handelt.

10. Verfahren nach Anspruch 8, wobei es sich bei dem Referenzpolypeptid um eine Polypeptidsequenz mit wenigstens 70% Identität mit einem oder mehreren entsprechenden Wildtyp-Polypeptiden handelt.

11. Verfahren nach Anspruch 8, wobei die Anzahl an Methionin- und/oder Lysin-Aminosäuren im Referenzpolypeptid als die mittlere Anzahl an Methionin- und/oder Lysin-Aminosäuren in zwei oder mehr Referenzpolypeptiden bestimmt wird.

12. Verfahren nach Anspruch 8, wobei die Verringerung von Methionin- und/oder Lysin-Aminosäuren im interessierenden Polypeptid über eine Länge von wenigstens 80 oder mehr zusammenhängenden Aminosäuren bestimmt wird.

13. Verfahren nach Anspruch 8, wobei die Anzahl an Methionin- und/oder Lysin-Aminosäuren im Vergleich mit dem Referenzpolypeptid um wenigstens 30% verringert ist.

14. Verfahren nach Anspruch 8, wobei die Verringerung der Methionin- und/oder Lysin-Aminosäuren im modifizierten Polypeptid ohne Berücksichtigung von Signalsequenzen, die in der Wirtszelle prozessiert werden können, bestimmt wird.

## Revendications

1. Méthode de production d'un polypeptide d'intérêt dans une cellule hôte, laquelle méthode comprend :
a. la fourniture d'une cellule hôte qui héberge un acide nucléique codant pour un polypeptide d'intérêt, où le polypeptide d'intérêt est modifié de façon à comprendre moins de résidus méthionine et/ou lysine par rapport à un polypeptide de référence, à l'exclusion de tout acide aminé méthionine initial situé au niveau de l'extrémité N-terminale de la séquence polypeptidique, où les acides aminés méthionine et/ou lysine dans le polypeptide de référence ont été substitués par des acides aminés différents de la méthionine et/ou différents de la lysine et où la modification a lieu dans le squelette du polypeptide mature ;
b. la culture de la cellule hôte dans des conditions convenables pour la production du polypeptide ; et, éventuellement,
c. la récupération du composé d'intérêt,
dans laquelle le nombre des acides aminés méthionine et/ou lysine dans le polypeptide d'intérêt est réduit d'au moins 25% par rapport au polypeptide de référence, où la cellule hôte est une cellule de levure ou une cellule de champignon filamenteux, où le polypeptide de référence est un polypeptide dont on souhaite l'augmentation de l'expression dans la cellule hôte, où la spécificité de substrat ou la spécificité de fixation du polypeptide modifié est maintenue ou son activité spécifique est la même qu'avant l'amélioration.

2. Méthode selon la revendication 1, dans laquelle le polypeptide de référence est un polypeptide de type sauvage correspondant.

3. Méthode selon la revendication 1 ou 2, dans laquelle le polypeptide de référence est une séquence polypeptidique ayant au moins 70% d'identité avec un ou plusieurs polypeptides de type sauvage correspondants.

4. Méthode selon l'une quelconque des revendications précédentes, dans laquelle le nombre des acides aminés méthionine et/ou lysine dans le polypeptide de référence est déterminé comme le nombre moyen d'acides aminés méthionine et/ou lysine dans deux, ou plus, polypeptides de référence.

5. Méthode selon l'une quelconque des revendications précédentes, dans laquelle la réduction des acides aminés méthionine et/ou lysine dans le polypeptide d'intérêt est déterminée sur une longueur d'au moins 80, ou plus, acides aminés contigus.

6. Méthode selon l'une quelconque des revendications précédentes, dans laquelle la réduction des acides aminés méthionine et/ou lysine dans le polypeptide d'intérêt est déterminée sans prendre en compte les séquences signal pouvant être traitées dans la cellule hôte.

7. Méthode selon l'une quelconque des revendications précédentes, dans laquelle le polypeptide d'intérêt est une protéine sécrétée.

8. Méthode d'amélioration du taux d'expression d'un polypeptide dans une cellule hôte, laquelle méthode comprend la réduction du nombre des acides aminés méthionine et/ou lysine dans le polypeptide par rapport à un polypeptide de référence, à l'exclusion de tout acide aminé méthionine initial situé au niveau de l'extrémité N-terminale de la séquence polypeptidique, où le nombre des acides aminés méthionine et/ou lysine dans le squelette d'acides aminés du polypeptide est réduit d'au moins 25%, où les acides aminés méthionine et/ou lysine dans le polypeptide de référence ont été substitués par des acides aminés différents de la méthionine et/ou différents de la lysine et où la modification a lieu dans le squelette du polypeptide mature, où la cellule hôte est une cellule de levure ou une cellule de champignon filamenteux, où le polypeptide de référence est un polypeptide dont on souhaite l'augmentation de l'expression dans la cellule hôte, où la spécificité de substrat ou la spécificité de fixation du polypeptide modifié est maintenue ou son activité spécifique est la même qu'avant l'amélioration.

9. Méthode selon la revendication 8, dans laquelle le polypeptide de référence est un polypeptide de type sauvage correspondant.

10. Méthode selon la revendication 8, dans laquelle le polypeptide de référence est une séquence polypeptidique ayant au moins 70% d'identité avec un ou plusieurs polypeptides de type sauvage correspondants.

11. Méthode selon la revendication 8, dans laquelle le nombre des acides aminés méthionine et/ou lysine dans le polypeptide de référence est déterminé comme le nombre moyen d'acides aminés méthionine et/ou lysine dans deux, ou plus, polypeptides de référence.

12. Méthode selon la revendication 8, dans laquelle la réduction des acides aminés méthionine et/ou lysine dans le polypeptide d'intérêt est déterminée sur une longueur d'au moins 80, ou plus, acides aminés contigus.

13. Méthode selon la revendication 8, dans laquelle le nombre des acides aminés méthionine et/ou lysine est réduit d'au moins 30% par rapport au polypeptide de référence.

14. Méthode selon la revendication 8, dans laquelle la réduction des acides aminés méthionine et/ou lysine dans le polypeptide modifié est déterminée sans prendre en compte les séquences signal pouvant être traitées dans la cellule hôte.
